# EUROPEAN PATENT SPECIFICATION

(11) **EP 4 263 547 B1**
(45) Date of publication and mention of the grant of the patent: **02.07.2025**
(21) Application number: 21824401.0
(22) Date of filing: 13.12.2021
(51) Int. Cl.: C07D 491/048, A61K 31/506, A61P 11/00, A61P 11/06

(54) **DIHYDROFUROPYRIDINE DERIVATIVES AS RHO- KINASE INHIBITORS**
DIHYDROFUROPYRIDINDERIVATE ALS RHO-KINASE-INHIBITOREN
DÉRIVÉS DE DIHYDROFUROPYRIDINE EN TANT QU'INHIBITEURS DE RHO-KINASE

(30) Priority: 15.12.2020 EP 20214149
(43) Date of publication of application: 25.10.2023
(73) Proprietor: Chiesi Farmaceutici S.p.A., 43122 Parma (IT)
(72) Inventor: RANCATI, Fabio, 43122 Parma (IT); ACCETTA, Alessandro, 43122 Parma (IT); CAPELLI, Anna Maria, 43122 Parma (IT); PALA, Daniele, 43122 Parma (IT); MAZZUCATO, Roberta, 43122 Parma (IT); EDWARDS, Christine, 43122 Parma (IT); PASQUA, Adele Elisa, 43122 Parma (IT)
(74) Representative: Chiesi Farmaceutici S.p.A.
(86) International application number: PCT/EP2021/085380
(87) International publication number: WO 2022/128853

(56) References cited:
- WO-A1-2004/039796
- WO-A1-2019/238628
- OLIVIER DEFERT ET AL: "Rho kinase inhibitors: a patent review (2014 - 2016)", EXPERT OPINION ON THERAPEUTIC PATENTS, vol. 27, no. 4, 16 January 2017 (2017-01-16), GB, pages 507 - 515, XP055400492, ISSN: 1354-3776, DOI: 10.1080/13543776.2017.1272579
- YANGBO FENG ET AL: "Rho Kinase (ROCK) Inhibitors and Their Therapeutic Potential", JOURNAL OF MEDICINAL CHEMISTRY, vol. 59, no. 6, 30 October 2015 (2015-10-30), US, pages 2269 - 2300, XP055535566, ISSN: 0022-2623, DOI: 10.1021/acs.jmedchem.5b00683

## Description

### FIELD OF THE INVENTION

The present invention relates to novel compounds inhibiting Rho Kinase (hereinafter ROCK Inhibitors); methods of preparing such compounds, pharmaceutical compositions containing them and therapeutic use thereof.

### BACKGROUND OF THE INVENTION

The compounds of the invention are inhibitors of the activity or function of the ROCK-I and/or ROCK-II isoforms of the Rho-associated coiled-coil forming protein kinase (ROCK).

Rho-associated coiled-coil forming protein kinase (ROCK) belongs to the AGC (PKA/PKG/PKC) family of serine-threonine kinases. Two human isoforms of ROCK have been described, ROCK-I (also referred to as p160 ROCK or ROKβ or ROCK1) and ROCK-II (ROKα or ROCK2) are approximately 160 kDa proteins containing an N-terminal Ser/Thr kinase domain, followed by a coiled-coil structure, a pleckstrin homology domain, and a cysteine-rich region at the C-terminus (Riento, K.; Ridley, A. J. Rocks: multifunctional kinases in cell behaviour. Nat. Rev. Mol. Cell Biol. 2003, 4, 446-456).

Both ROCK-II and ROCK-I are expressed in many human and rodent tissues including the heart, pancreas, lung, liver, skeletal muscle, kidney and brain (above Riento and Ridley, 2003). In patients with pulmonary hypertension, ROCK activity is significantly higher in both lung tissues and circulating neutrophils as compared with controls
(Duong-Quy S, Bei Y, Liu Z, Dinh-Xuan AT. Role of Rho-kinase and its inhibitors in pulmonary hypertension. Pharmacol Ther. 2013;137(3):352-64). A significant correlation was established between neutrophil ROCK activity and the severity and duration of pulmonary hypertension (Duong-Quy et al., 2013).

There is now substantial evidence that ROCK is involved in many of the pathways that contribute to the pathologies associated with several acute and chronic pulmonary diseases, including asthma, COPD, bronchiectasis and ARDS/ALI. Given the biological effect of ROCK, selective inhibitors have the potential to treat a number of pathological mechanisms in respiratory diseases, such as smooth muscle hyper-reactivity, bronchoconstriction, airway inflammation and airway remodeling, neuromodulation and exacerbations due to respiratory tract viral infection (Fernandes LB, Henry PJ, Goldie RG. Rho kinase as a therapeutic target in the treatment of asthma and chronic obstructive pulmonary disease. Ther Adv Respir Dis. 2007 Oct;1(1):25-33). Indeed the Rho kinase inhibitor Y-27632 causes bronchodilatation and reduces pulmonary eosinophilia trafficking and airways hyperresponsiveness (Gosens, R.; Schaafsma, D.; Nelemans, S. A.; Halayko, A. J. Rhokinase as a drug target for the treatment of airway hyperresponsiveness in asthma. Mini-Rev. Med. Chem. 2006, 6, 339-348). Pulmonary ROCK activation has been demonstrated in humans with idiopathic pulmonary fibrosis (IPF) and in animal models of this disease. ROCK inhibitors can prevent fibrosis in these models and, more importantly, induce the regression of already established fibrosis, thus indicating ROCK inhibitors as potential powerful pharmacological agents to halt progression of pulmonary fibrosis (Jiang, C.; Huang, H.; Liu, J.; Wang, Y.; Lu, Z.; Xu, Z. Fasudil, a rho-kinase inhibitor, attenuates bleomycin-induced pulmonary fibrosis in mice. Int. J. Mol. Sci. 2012, 13, 8293-8307).

Various compounds have been described in the literature as Rho Kinase Inhibitors. See e.g. WO2004/039796 disclosing phenylaminopyrimidine compounds derivatives; WO2006/009889 disclosing indazole compound derivatives; WO2010/032875 disclosing nicotinamide compounds derivatives; WO2009/079008 disclosing pyrazole derivatives; WO2014/118133 disclosing pyrimidine derivatives and, of the same Applicant of the present invention, WO2018/115383 disclosing bicyclic dihydropyrimidine and WO 2018/138293, WO 2019/048479, WO 2019/121223, WO 2019/121233, WO 2019/121406, WO 2019/238628, WO 2020/016129 disclosing tyrosine-amide compounds derivatives and analogues.

The compounds disclosed exhibit substantial structural differences from the compounds of the present invention.

There remains a potential for developing novel and pharmacologically improved ROCK inhibitors in many therapeutic areas.

In view of the number of pathological responses which are mediated by ROCK enzymes, there is a continuing need for inhibitors of such enzymes which can be useful in the treatment of many disorders. The present invention relates to novel compounds differing from the structures disclosed in the art at least for a common new core scaffold. In fact the invention relates to compounds that are characterized by 2,3-dihydrofuro[3,2-c]pyridine moiety, particularly 2,3-dihydrofuro[3,2-c]pyridin-4-amine, particularly preferably N-(3-(((2,3-dihydrofuro[3,2-c]pyridin-4-yl)amino)methyl)phenyl)formamide and 3-(((2,3-dihydrofuro[3,2-c]pyridin-4-yl)amino)methyl)benzamide derivatives, which are inhibitors of ROCK-I and ROCK-II, which are inhibitors of ROCK-I and ROCK-II isoforms of the Rho-associated coiled-coil forming protein kinase (ROCK) that have therapeutically desirable characteristics, particularly promising for some pulmonary diseases including asthma, chronic obstructive pulmonary disease (COPD), idiopathic pulmonary fibrosis (IPF) and pulmonary hypertension (PH) and specifically pulmonary arterial hypertension (PAH The compounds of the invention may be prepared for administration by any route consistent with their pharmacokinetic properties. The compound of the invention are active as inhibitors of ROCK-I and ROCK-II isoforms, they are potent and have advantageously other improved properties such as selectivity and other in-vitro properties indicative for a preferred route of administartion.

### SUMMARY OF THE INVENTION

The present invention is directed to a class of compounds, acting as inhibitors of the Rho Kinase (ROCK), of formula (I)

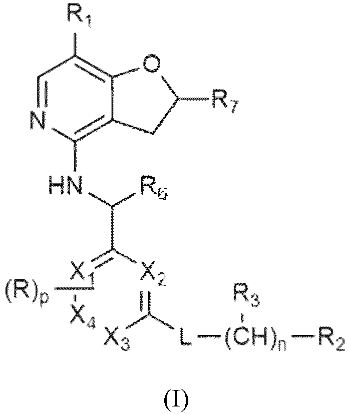

Wherein the variables X₁, X₂, X₃ and X₄, p, R, R₁, L, n, R₂ and R₃, R₄ and R₅, R₆ and R₇ are as defined in the detailed description of the invention; single enantiomers, diastereoisomers and mixtures thereof in any proportion or pharmaceutically acceptable salts and solvates thereof.

In one aspect, the present invention refers to a compound of formula (I) for use as a medicament. In one aspect the present invention provides the use of a compound of the invention for the manufacture of a medicament.

In a further aspect, the present invention provides the use of a compound of the invention for the preparation of a medicament for the treatment of any disease associated with ROCK enzyme mechanisms, that is to say characterized by ROCK enzyme aberrant activity and/or wherein an inhibition of activity is desirable and in particular through the selective inhibition of the ROCK enzyme isoforms over other Kinases.

In another aspect, the present invention provides a compound of formula (I) for use in a method for prevention and/or treatment of any disease associated with ROCK enzyme mechanisms as above defined, said method comprises administering to a patient in need of such treatment a therapeutically effective amount of a compound of the invention.

In a Particular aspect the compounds of the invention are used alone or combined with other active ingredients and may be administered for the prevention and/or treatment of a pulmonary disease including asthma, chronic obstructive pulmonary disease (COPD), idiopathic pulmonary fibrosis (IPF) and pulmonary hypertension (PH) and specifically pulmonary arterial hypertension (PAH).

### DETAILED DESCRIPTION OF THE INVENTION

### Definitions

The term "Pharmaceutically acceptable salts" refers to derivatives of compounds of formula (I) wherein the parent compound is suitably modified by converting any of the free acid or basic group, if present, into the corresponding addition salt with any base or acid conventionally intended as being pharmaceutically acceptable.

Suitable examples of said salts may thus include mineral or organic acid addition salts of basic residues such as amino groups, as well as mineral or organic basic addition salts of acid residues such as carboxylic groups.

Cations of inorganic bases which can be suitably used to prepare salts of the invention comprise ions of alkali or alkaline earth metals such as potassium, sodium, calcium or magnesium. Those obtained by reacting the main compound, functioning as a base, with an inorganic or organic acid to form a salt comprise, for example, salts of hydrochloric, hydrobromic, sulfuric, phosphoric, methane sulfonic, camphor sulfonic, acetic, oxalic, maleic, fumaric, succinic and citric acids.

Many organic compounds can form complexes with solvents in which they are reacted or from which they are precipitated or crystallized. These complexes are known as "solvates" which are a further object of the invention. Polymorphs and crystalline forms of compounds of formula (I), or of pharmaceutically acceptable salts, or solvates thereof are a further object of the invention.

The term "Halogen" or "halogen atoms" includes fluorine, chlorine, bromine, and iodine atom; meaning Fluoro, Chloro, Bromo, Iodo as substituent.

The term "(C₁-C₆)Alkyl" refers to straight-chained or branched alkyl groups wherein the number of carbon atoms is in the range 1 to 6. Particular alkyl groups are for example methyl, ethyl, n-propyl, isopropyl, t-butyl, and the like.

The expressions "(C₁-C₆)Haloalkyl" refer to the above defined "(C₁-C₆)alkyl" groups wherein one or more hydrogen atoms are replaced by one or more halogen atoms, which can be the same or different from each other. Examples include halogenated, poly-halogenated and fully halogenated alkyl groups wherein all of the hydrogen atoms are replaced by halogen atoms, e.g. trifluoromethyl or difluoro methyl groups.

By way of analogy, the terms "(C₁-C₆)Hydroxyalkyl" and "(C₁-C₆)aminoalkyl" refer to the above defined "(C₁-C₆)alkyl" groups wherein one or more hydrogen atoms are replaced by one or more hydroxy (OH) or amino group respectively, examples being hydroxymethyl and aminomethyl and the like.

The definition of aminoalkyl encompasses alkyl groups (i.e. "(C₁-C₆)alkyl" groups) substituted by one or more amino group (-NR₈R₉). An example of aminoalkyl is a mono-aminoalkyl group such as R₈R₉N-(C₁-C₆)alkyl. The substituent R₈ and R₉ they are defined as R₄ and R₅ in the detailed description of the invention herebelow.

The term "(C₃-C₁₀)cycloalkyl" likewise "(C₃-C₈)cycloalkyl" or "(C₃-C₆)cycloalkyl" refers to saturated cyclic hydrocarbon groups containing the indicated number of ring carbon atoms. Examples include cyclopropyl, cyclobutyl, cyclopentyl, cyclohexyl and cycloheptyl, and polycyclic ring systems such as adamantan-yl.

The expression "Aryl" refers to mono, bi- or tri-cyclic carbon ring systems which have 6 to 20, preferably from 6 to 15 ring atoms, wherein at least one ring is aromatic. The expression "heteroaryl" refers to mono-, bi- or tri-cyclic ring systems with 5 to 20, preferably from 5 to 15 ring atoms, in which at least one ring is aromatic and in which at least one ring atom is a heteroatom (e.g. N, S or O).

Examples of aryl or heteroaryl monocyclic ring systems include, for instance, phenyl, thienyl, pyrrolyl, pyrazolyl, imidazolyl, isoxazolyl, oxazolyl, isothiazolyl, thiazolyl, pyridinyl, pyrimidinyl, pyrazinyl, pyridazinyl, triazinyl, furanyl radicals and the like.

Examples of aryl or heteroaryl bicyclic ring systems include naphthalenyl, biphenylenyl, purinyl, pteridinyl, pyrazolopyrimidinyl, benzotriazolyl, benzoimidazole-yl, quinolinyl, isoquinolinyl, indolyl, isoindolyl, indazolyl, benzothiopheneyl, benzodioxinyl, dihydrobenzodioxinyl, indenyl, dihydro-indenyl, dihydrobenzo[1,4]dioxinyl, benzothiazole-2-yl, dihydrobenzodioxepinyl, benzooxazinyl, 1,2,3,4-tetrahydroisoquinoline-6-yl, 4,5,6,7-tetrahydrothiazolo[4,5-c]pyridine, 4,5,6,7-tetrahydrobenzo[d]thiazol-2-yl, 5,6,7,8-tetrahydro-1,7-naphthyridine, radicals and the like.

Examples of aryl or heteroaryl tricyclic ring systems include fluorenyl radicals as well as benzocondensed derivatives of the aforementioned heteroaryl bicyclic ring systems.

The derived expression "(C₃-C₁₀)heterocycloalkyl" likewise "(C₃-C₈)heterocycloalkyl" or "(C₃-C₆)heterocycloalkyl" refers to saturated or partially unsaturated monocyclic cycloalkyl groups of the indicated number of carbons, in which at least one ring carbon atom is replaced by at least one heteroatom (e.g. N, NH, S or O) or may bear an -oxo (=O) substituent group. Said heterocycloalkyl (i.e. heterocyclic radical or group) is further optionally substituted on the available points in the ring, namely on a carbon atom, or on an heteroatom available for substitution. Examples of heterocycloalkyl are represented by: oxetanyl, tetrahydro-furanyl, pyrrolidinyl, imidazolidinyl, thiazolidinyl, piperazinyl, piperidinyl, morpholinyl, thiomorpholinyl, dihydro- or tetrahydro-pyridinyl, tetrahydropyranyl, pyranyl, 2H- or 4H-pyranyl, dihydro- or tetrahydrofuranyl, dihydroisoxazolyl, pyrrolidin-2-one-yl, dihydropyrrolyl, 5-oxopyrrolidin-3-yl, (1R,5S,6r)-3-oxabicyclo[3.1.0]hexan-6-yl, octahydrocyclopenta[c]pyrrol-5-yl, 4,5,6,7-tetrahydropyrazolo[1,5-a]pyrazin-2-yl; 4,5,6,7-tetrahydrothiazolo[5,4-c]pyridin-2-yl radicals and the like.

The term "Aryl(C₁-C₆)alkyl" refers to an aryl ring linked to a straight-chained or branched alkyl groups wherein the number of constituent carbon atoms is in the range from 1 to 6, e.g. phenylmethyl (i.e. benzyl), phenylethyl or phenylpropyl.

Likewise the term "Heteroaryl(C₁-C₆)alkyl" refers to an heteroaryl ring linked to a straight-chained or branched alkyl groups wherein the number of constituent carbon atoms is in the range from 1 to 6, e.g. furanylmethyl.

The term "alkanoyl", refers to HC(O)- or to alkylcarbonyl groups (e.g. (C₁-C₆)alkylC(O)-) wherein the group "alkyl" has the meaning above defined. Examples include formyl, acetyl, propanoyl, butanoyl.

The term "(C₁-C₁₀) alkoxy" or "(C₁-C₁₀) alkoxyl", likewise "(C₁-C₆) alkoxy" or "(C₁-C₆) alkoxyl" etc., refers to a straight or branched hydrocarbon of the indicated number of carbons, linked to the rest of the molecule through an oxygen bridge. "(C₁-C₆)Alkylthio" refers to the above hydrocarbon linked through a sulfur bridge.

The derived expression "(C₁-C₆)haloalkoxy" or "(C₁-C₆)haloalkoxyl" refers to the above defined haloalkyl, linked through an oxygen bridge. An example of (C₁-C₆)haloalkoxy is trifluoromethoxy.

Likewise derived expression "(C₃-C₆)heterocycloalkyl-(C₁-C₆)alkyl" and "(C₃-C₆)cycloalkyl-(C₁-C₆) alkyl" refer to the above defined heterocycloalkyl and cycloalkyl groups linked to the rest of the molecule via an alkyl group of the indicated number of carbons, corresponding e.g. to linear formula (C₃-C₆)heterocycloalkyl-(CH₂)ₘ- or (C₃-C₆)cycloalkyl-(CH₂)ₘ₋ for example piperidin-4-yl-methyl, cyclohexylethyl.

The derived expression "(C₁-C₆)alkoxy-(C₁-C₆)alkyl" refers to the above defined alkoxy group linked to the rest of the molecule via an alkyl group of the indicated number of carbons, for example methoxymethyl.

Likewise "(C₁-C₆)haloalkoxy (C₁-C₆)alkyl" refers to the above defined (C₁-C₆)haloalkoxy" group linked to the rest of the molecule via an alkyl group of the indicated number of carbons, for example difluoromethoxypropyl.

An oxo moiety is represented by (O) as an alternative to the other common representation, e.g. (=O). Thus, in terms of general formula, the carbonyl group is herein preferably represented as -C(O)- as an alternative to the other common representations such as -CO-, -(CO)- or -C(=O)-. In general the bracketed group is a lateral group, not included into the chain, and brackets are used, when deemed useful, to help disambiguating linear chemical formulas; e.g. the sulfonyl group -SO₂- might be also represented as -S(O)₂- to disambiguate e.g. with respect to the sulfinic group -S(O)O-.

Likewise, the group -(CHR₃)ₙ-R₂ herein is a linear representation of the terminal part of the charachterizing group 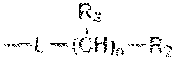 found in formula (I), and (Ia).

When a numerical index the statement (value) "p is zero" or "p is 0" means that the substituent or group bearing the index p (e.g. (R)p) is absent, that is to say no substituent, other than H when needed, is present. Likewise when the index is attached to a bridging divalent group (e.g. (CH₂)n) the statement " n in each occurrence is zero..." or "n is 0" means that the bridging group is absent, that is to say it is a bond.

Whenever basic amino or quaternary ammonium groups are present in the compounds of formula (I), physiological acceptable anions, selected among chloride, bromide, iodide, trifluoroacetate, formate, sulfate, phosphate, methanesulfonate, nitrate, maleate, acetate, citrate, fumarate, tartrate, oxalate, succinate, benzoate, p-toluenesulfonate, pamoate and naphthalene disulfonate may be present. Likewise, in the presence of acidic groups such as COOH groups, corresponding physiological cation salts may be present as well, for instance including alkaline or alkaline earth metal ions.

Compounds of formula (I) when they contain one or more stereogenic center, may exist as optical stereoisomers.

Where the compounds of the invention have at least one stereogenic center, they may accordingly exist as enantiomers. Where the compounds of the invention possess two or more stereogenic centers, they may additionally exist as diastereoisomers. It is to be understood that all such single enantiomers, diastereoisomers and mixtures thereof in any proportion are encompassed within the scope of the present invention. The absolute configuration (R) or (S) for carbon bearing a stereogenic center is assigned on the basis of Cahn-Ingold-Prelog nomenclature rules based on groups' priorities.

"Single stereoisomer", "single diastereoisomer" or "single enantiomer", when reported near the chemical name of a compound indicate that the isomer was isolated as a single diastereoisomer or enantiomer (e.g via chiral chromatography) but the absolute configuration at the relevant stereogenic center was not determined/assigned.

Atropisomers result from hindered rotation about single bonds where the steric strain barrier to rotation is high enough to allow for the isolation of the conformers (Bringmann G et al, Angew. Chemie Int. Ed. 44 (34), 5384-5427, 2005. doi:10.1002/anie.200462661).

Oki defined atropisomers as conformers that interconvert with a half-life of more than 1000 seconds at a given temperature (Oki M, Topics in Stereochemistry 14, 1-82, 1983).

Atropisomers differ from other chiral compounds in that in many cases they can be equilibrated thermally whereas in the other forms of chirality isomerization is usually only possible chemically.

Separation of atropisomers is possible by chiral resolution methods such as selective crystallization. In an atropo-enantioselective or atroposelective synthesis one atropisomer is formed at the expense of the other. Atroposelective synthesis may be carried out by use of chiral auxiliaries like a Corey Bakshi Shibata (CBS) catalyst, an asymmetric catalyst derived from proline, or by approaches based on thermodynamic equilibration when an isomerization reaction favors one atropisomer over the other.

Racemic forms of compounds of formula (I) as well as the individual atropisomers (substantially free of its corresponding enantiomer) and stereoisomer-enriched atropisomers mixtures are included in the scope of the present invention.

The invention further concerns the corresponding deuterated derivatives of compounds of formula (I). In the context of the present invention, deuterated derivative means that at least one position occupied by a hydrogen atom is occupied by deuterium in an amount above its natural abundance. Preferably, the percent of deuterium at that position is at least 90%, more preferably at least 95%, even more preferably 99%.

All preferred groups or embodiments described above and herebelow for compounds of formula (I) may be combined among each other and apply as well *mutatis mutandis.*

As above mentioned, the present invention refers to compounds of general formula (I) as reported below, acting as ROCK inhibitors, to processes for the preparation thereof, pharmaceutical compositions comprising them either alone or in combination with one or more active ingredient, in admixture with one or more pharmaceutically acceptable carrier.

A first aspect of the present invention is directed to a class of compounds of formula (I) 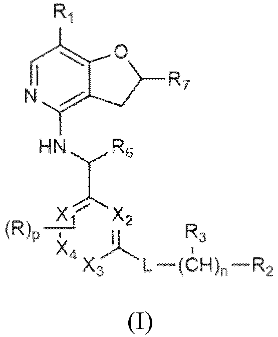 wherein
**X₁, X₂, X₃** and **X₄** are all CH or one of X₁, X₂, X₃ and X₄ is N and the others are CH;
p is zero or an integer from 1 to 4;
each R, when present, is halogen in each occurrence independently selected from (C₁-C₆)alkyl and halogen selected from F, Cl, Br and I; wherein preferably R is F, Cl or methyl;
R₁ is pyrazolyl, preferably pyrazol-4-yl;
L is -C(O)NH- or -NHC(O)- ;
n is in each occurrence independently 0 (i.e. R₃ is absent) or an integer selected from 1, 2 or 3;
R₂ and R₃ are in each occurrence independently selected from the group consisting of
-H,
halogen,
-OH,
-(CH₂)ₘNR₄R₅,
(C₁-C₆)alkyl,
(C₁-C₆)hydroxyalkyl,
(C₁-C₆) alkoxy,
(C₁-C₆) alkoxy(C₁-C₆)alkyl,
(C₁-C₆)haloalkyl,
(C₁-C₆)haloalkoxy,
(C₁-C₆)haloalkoxy(C₁-C₆)alkyl,
(C₃-C₁₀)cycloalkyl,
aryl, heteroaryl and (C₃-C₆)heterocycloalkyl,
each of which cycloalkyl, aryl, heteroaryl and heterocycloalkyl
is in its turn optionally and independently substituted with one or more groups selected from
halogen,
-OH,
(C₁-C₆)alkyl,
(C₁-C₆)hydroxyalkyl,
(C₁-C₆)alkoxy,
(C₁-C₆)alkoxy(C₁-C₆)alkyl,
(C₁-C₆)haloalkyl,
(C₁-C₆)haloalkoxy,
-(CH₂)ₘNR₄R₅,
-O-(CH₂)ₘNR₄R₅,
alkanoyl,
aryl, heteroaryl, cycloalkyl,
aryl-(C₁-C₆)alkyl,
(C₃-C₆)heterocycloalkyl,
(C₃-C₈)heterocycloalkyl-(C₁-C₆)alkyl,
each of said aryl, heteroaryl, cycloalkyl, heterocycloalkyl is still further optionally substituted by one or more group selected independently from halogen, -OH, (C₁-C₈)alkyl, (C₁-C₆)haloalkyl, (C₁-C₆)hydroxyalkyl;
m is in each occurrence independently 0 or an integer selected from 1, 2 or 3;
R₄ and R₅, the same or different, are selected from the group consisting of
-H,
(C₁-C₆)alkyl,
(C₁-C₆)haloalkyl,
(C₁-C₆)hydroxyalkyl,
(C₃-C₆)heterocycloalkyl;
R₆ and R7 are independently selected from the group consisting of -H, (C₁-C₆)alkyl;
single enantiomers, diastereoisomers and mixtures thereof in any proportion and/or pharmaceutically acceptable salts and solvates thereof.

In a preferred embodiment the invention is directed to a compound of formula (I) wherein X₃ and X₄ are all CH groups and X₁ or X₂ are in the alternative independently a CH group or a nitrogen atom ;
R₁ is pyrazol-4-yl;
all the other variables being as defined above.

Said preferred group of compounds is represented by the formula (Ia)

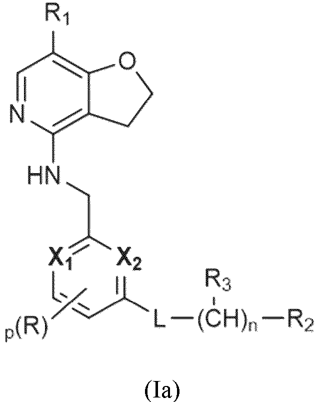

Particularly preferred are compound of formula (I) as above defined, wherein X₁ , X₂, X₃ , X₄ are all CH group;
each R, when present, is halogen in each occurrence independently selected from F, Cl, Br and I, wherein preferably R is F;
R₁ is pyrazolyl, preferably pyrazol-4-yl;
L is -C(O)NH-- or -NHC(O)- ;
n is 0 (i.e. R₃ is absent);
R₂ is in each occurrence independently selected from the group consisting of
(C₁-C₆)alkyl,
(C₁-C₆)hydroxyalkyl,
(C₁-C₆)alkoxy(C₁-C₆)alkyl,
(C₁-C₆)haloalkyl,
(C₁-C₆)haloalkoxy (C₁-C₆)alkyl;
all the other variables being as defined above,
single enantiomers, diastereoisomers and mixtures thereof in any proportion and/or pharmaceutically acceptable salts and solvates thereof.

Said preferred group of compounds is represented by the formula (Ib)

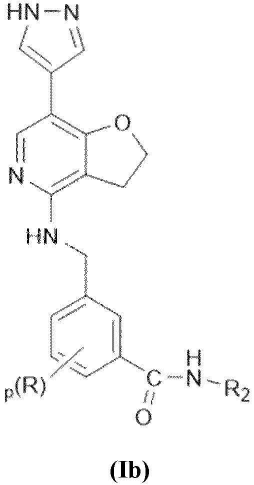

In another preferred embodiment the invention is directed to a compound of formula (I) wherein **X₁, X₂, X₃** and **X₄** are all CH;
p is zero or an integer from 1 to 4;
each R, when present, is halogen in each occurrence independently selected from F, Cl, Br and I, wherein preferably R is F;
R₁ is pyrazolyl, preferably pyrazol-4-yl;
L is -C(O)NH or-NHC(O)- ;
n is in each occurrence independently 0 or an integer selected from 1, 2 or 3;
R₃ when present is H, and
R₂ is selected from the group consisting of
aryl, heteroaryl and (C₃-C₆)heterocycloalkyl,
each of which aryl, heteroaryl and heterocycloalkyl
is in its turn optionally and independently substituted with one or more groups selected from
(C₁-C₆)alkyl,
(C₁-C₆)hydroxyalkyl,
(C₁-C₆) alkoxy,
(C₁-C₆) alkoxy (C₁-C₆)alkyl,
-(CH₂)ₘNR₄R₅,
-O-(CH₂)ₘNR₄R₅,
aryl, heteroaryl, cycloalkyl,
aryl-(C₁-C₆)alkyl,
(C₃-C₆)heterocycloalkyl,
(C₃-C₈)heterocycloalkyl-(C₁-C₆)alkyl,
each of said aryl, heteroaryl, cycloalkyl, heterocycloalkyl is still further optionally substituted by one or more group selected independently from halogen, -OH, (C₁-C₈)alkyl, (C₁-C₆)haloalkyl, (C₁-C₆)hydroxyalkyl;
m is in each occurrence independently 0 or an integer selected from 1, 2 or 3;
R₄ and R₅, the same or different, are selected from the group consisting of
-H,
(C₁-C₆)alkyl,
(C₁-C₆)haloalkyl,
(C₁-C₆)hydroxyalkyl,
(C₃-C₆)heterocycloalkyl;
single enantiomers, diastereoisomers and mixtures thereof in any proportion and/or pharmaceutically acceptable salts and solvates thereof.

Particularly preferred in this last embodiment is a compound wherein R₂ is selected from (pyridinyl)methyl, (pyridinyl)ethyl, methoxypyridinyl, ((dimethylamino)ethoxy)pyridinyl, or from 1-(2-(dimethylamino)ethyl)-1H-indazole-5-yl, 1-(2-morpholinoethyl)-1H-indazole-5-yl, 1-(1-methylpiperidin-4-yl)-1H-indazole-5-yl;
all the other variables and substitution being as defined above,
single enantiomers, diastereoisomers and mixtures thereof in any proportion and/or pharmaceutically acceptable salts and solvates thereof.

Thus, a group of particularly preferred compounds are

| **Exampl e** | **Chemical Name** |
|---|---|
| 43 | N-(3-(((7-(1H-pyrazol-4-yl)-2,3-dihydrofuro[3,2-c]pyridin-4-yl)amino)methyl)phenyl)-1-(2-(dimethylamino)ethyl)-1H-indazole-5-carboxamide |
| 44 | N-(3-(((7-(1H-pyrazol-4-yl)-2,3-dihydrofuro[3,2-c]pyridin-4-yl)amino)methyl)phenyl)-1-(1-methylpiperidin-4-yl)-1H-indazole-5-carboxamide |
| 46 | N-(3-(((7-(1H-pyrazol-4-yl)-2,3-dihydrofuro[3,2-c]pyridin-4-yl)amino)methyl)phenyl)-1-(2-morpholinoethyl)-1H-indazole-5-carboxamide |
| 17 | 3-(((7-(1H-pyrazol-4-yl)-2,3-dihydrofuro[3,2-c]pyridin-4-yl)amino)methyl)-N-(2-(pyridin-4-yl)ethyl)benzamide |
| 22 | 3-(((7-(1H-pyrazol-4-yl)-2,3-dihydrofuro[3,2-c]pyridin-4-yl)amino)methyl)-N-(2-(pyridin-3-yl)ethyl)benzamide |
| 31 | 3-(((7-(1H-pyrazol-4-yl)-2,3-dihydrofuro[3,2-c]pyridin-4-yl)amino)methyl)-N-(5-methoxypyridin-2-yl)benzamide |
| 53 | 3-(((7-(1H-pyrazol-4-yl)-2,3-dihydrofuro[3,2-c]pyridin-4-yl)amino)methyl)-N-(pyridin-4-ylmethyl)benzamide |
| 57 | 3-(((7-(1H-pyrazol-4-yl)-2,3-dihydrofuro[3,2-c]pyridin-4-yl)amino)methyl)-N-(5-(2-(dimethylamino)ethoxy)pyridin-2-yl)benzamide |

A further preferred group of compounds according to the invention are those of formula (I) wherein
**X₁, X₂, X₃** and **X₄** are all CH;
p is zero or 1;
each R, when present, is F;
R₁ is pyrazol 4-yl;
L is -C(O)NH- or -NHC(O)- ;
n is in each occurrence independently 0 or an integer selected from 1, 2;
R₂ and R₃ are in each occurrence independently selected from the group consisting of
-H,
(C₁-C₆)alkyl which is methyl,
(C₁-C₆) alkoxy (C₁-C₆)alkyl which is 2-methoxyethyl,
(C₁-C₆)haloalkyl which is 3-fluoropropyl,
(C₃-C₁₀)cycloalkyl which is cyclopropyl, cyclobutyl,
aryl which is phenyl;
heteroaryl which is pyridinyl, pyrazolyl, thiophenyl, imidazolyl, oxazolyl, isoxazolyl, thiazolyl, pyrimidinyl, 1,2,5-oxadiazol-3-yl, 4,5,6,7-tetrahydropyrazolo[1,5-a]pyrazin-2-yl, 4,5,6,7-tetrahydrothiazolo[5,4-c]pyridin-2-yl, 4,5,6,7-tetrahydrobenzo[d]thiazol-2-yl, 1,2,3,4-tetrahydroisoquinoline-6yl, 5,6,7,8-tetrahydro-1,7-naphthyridinyl, 1H-indole-5yl, isoindolin-yl, 1H-indazole-5yl;
and
(C₃-C₆)heterocycloalkyl which is piperidinyl, morpholinyl, tetrahydrofuranyl, tetrahydro-2H-pyran-yl;
each of which cycloalkyl, aryl, heteroaryl and heterocycloalkyl
is in its turn optionally and independently substituted with one or more groups selected from
halogen which is Fluoro;
(C₁-C₆)alkyl which is methyl;
(C₁-C₆)hydroxyalkyl, which is hydroxyethyl;
(C₁-C₆) alkoxy, which is methoxy;
-(CH₂)ₘNR₄R₅ which is (dimethylamino)methyl, 2-(dimethylamino)ethyl, dimethylamino wherein R₄ and R₅ are methyl and m is 0, 1 or 2, oxetan-3-ylamino wherein R₄ is H, R₅ is oxetanyl and m is 0;
-O-(CH₂)ₘNR₄R₅ which is ((dimethylamino)ethoxy)pyridinyl;
cycloalkyl which is cyclopropyl,
aryl-(C₁-C₆)alkyl, which is benzyl, phenethyl,
(C₃-C₆)heterocycloalkyl, which is oxetan-3-yl, piperidin-4-yl,
(C₃-C₈)heterocycloalkyl-(C₁-C₆)alkyl, which is morpholinoethyl, pyrrolidin-1-ylmethyl,
each of said heterocycloalkyl is still further optionally substituted by (C₁-C₈)alkyl which is methyl;
R₆ is -H, or methyl; and R₇ is -H
single enantiomers, diastereoisomers and mixtures thereof in any proportion and/or pharmaceutically acceptable salts and solvates thereof.

The invention also provides a pharmaceutical composition comprising a compound of formula (I), or a pharmaceutically acceptable salt thereof in admixture with one or more pharmaceutically acceptable carrier or excipient, either alone or in combination with one or more further active ingredient as detailed below.

According to specific embodiments, the invention provides the compounds listed in the table below single enantiomers, diastereoisomers and mixtures thereof in any proportion and/or pharmaceutically acceptable salts and solvates thereof.

| **Ex No.** | **Chemical Name** |
|---|---|
| 1 | 3-(((7-(1H-pyrazol-4-yl)-2,3-dihydrofuro[3,2-c]pyridin-4-yl)amino)methyl)-N-(1-cyclopropylpiperidin-4-yl)benzamide |
| 2 | 3-(((7-(1H-pyrazol-4-yl)-2,3-dihydrofuro[3,2-c]pyridin-4-yl)amino)methyl)-N-((1-methyl-1H-pyrazol-3-yl)methyl)benzamide |
| 3 | 3-(((7-(1H-pyrazol-4-yl)-2,3-dihydrofuro[3,2-c]pyridin-4-yl)amino)methyl)-N-(3-fluoropropyl)benzamide |
| 4 | 3-(((7-(1H-pyrazol-4-yl)-2,3-dihydrofuro[3,2-c]pyridin-4-yl)amino)methyl)-N-(pyridin-2-ylmethyl)benzamide |
| 5 | 3-(((7-(1H-pyrazol-4-yl)-2,3-dihydrofuro[3,2-c]pyridin-4-yl)amino)methyl)-N-(cyclopropylmethyl)benzamide |
| 6 | 3-(((7-(1H-pyrazol-4-yl)-2,3-dihydrofuro[3,2-c]pyridin-4-yl)amino)methyl)-N-(5,5-dimethyltetrahydrofuran-3-yl)benzamide |
| 7 | 3-(((7-(1H-pyrazol-4-yl)-2,3-dihydrofuro[3,2-c]pyridin-4-yl)amino)methyl)-N-(2-methoxyethyl)benzamide |
| 8 | 3-(((7-(1H-pyrazol-4-yl)-2,3-dihydrofuro[3,2-c]pyridin-4-yl)amino)methyl)-N-((1-methyl-1H-imidazol-4-yl)methyl)benzamide |
| 9 | 3-(((7-(1H-pyrazol-4-yl)-2,3-dihydrofuro[3,2-c]pyridin-4-yl)amino)methyl)-N-(pyrimidin-5-ylmethyl)benzamide |
| 10 | N-((1,2,5-oxadiazol-3-yl)methyl)-3-(((7-(1H-pyrazol-4-yl)-2,3-dihydrofuro[3,2 c]pyridin-4-yl)amino)methyl)benzamide |
| 11 | 3-(((7-(1H-pyrazol-4-yl)-2,3-dihydrofuro[3,2-c]pyridin-4-yl)amino)methyl)-N-((tetrahydro-2H-pyran-2-yl)methyl)benzamide |
| 12 | 3-(((7-(1H-pyrazol-4-yl)-2,3-dihydrofuro[3,2-c]pyridin-4-yl)amino)methyl)-N-(3,3-difluorocyclobutyl)benzamide |
| 13 | 3-(((7-(1H-pyrazol-4-yl)-2,3-dihydrofuro[3,2-c]pyridin-4-yl)amino)methyl)-N-(isoxazol-3-ylmethyl)benzamide |
| 14 | 3-(((7-(1H-pyrazol-4-yl)-2,3-dihydrofuro[3,2-c]pyridin-4-yl)amino)methyl)-N-(thiazol-4-ylmethyl)benzamide |
| 15 | 3-(((7-(1H-pyrazol-4-yl)-2,3-dihydrofuro[3,2-c]pyridin-4-yl)amino)methyl)-N-(5-methyl-4,5,6,7-tetrahydropyrazolo[1,5-a]pyrazin-2-yl)benzamide |
| 16 | 3-(((7-(1H-pyrazol-4-yl)-2,3-dihydrofuro[3,2-c]pyridin-4-yl)amino)methyl)-N-(2-(1-methylpiperidin-4-yl)ethyl)benzamide |
| 17 | 3-(((7-(1H-pyrazol-4-yl)-2,3-dihydrofuro[3,2-c]pyridin-4-yl)amino)methyl)-N-(2-(pyridin-4-yl)ethyl)benzamide |
| 18 | 3-(((7-(1H-pyrazol-4-yl)-2,3-dihydrofuro[3,2-c]pyridin-4-yl)amino)methyl)-N-(3-((dimethylamino)methyl)phenyl)benzamide |
| 19 | 3-(((7-(1H-pyrazol-4-yl)-2,3-dihydrofuro[3,2-c]pyridin-4-yl)amino)methyl)-N-(3-((dimethylamino)methyl)benzyl)benzamide |
| 20 | 3-(((7-(1H-pyrazol-4-yl)-2,3-dihydrofuro[3,2-c]pyridin-4-yl)amino)methyl)-N-(2-morpholinoethyl)benzamide |
| 21 | 3-(((7-(1H-pyrazol-4-yl)-2,3-dihydrofuro[3,2-c]pyridin-4-yl)amino)methyl)-N-(5-methyl-4,5,6,7-tetrahydrothiazolo[5,4-c]pyridin-2-yl)benzamide |
| 22 | 3-(((7-(1H-pyrazol-4-yl)-2,3-dihydrofuro[3,2-c]pyridin-4-yl)amino)methyl)-N-(2-(pyridin-3-yl)ethyl)benzamide |
| 23 | (S)-3-(((7-(1H-pyrazol-4-yl)-2,3-dihydrofuro[3,2-c]pyridin-4-yl)amino)methyl)-N-(2-hydroxy-1-phenylethyl)benzamide |
| 24 | 3-(((7-(1H-pyrazol-4-yl)-2,3-dihydrofuro[3,2-c]pyridin-4-yl)amino)methyl)-N-(4-((dimethylamino)methyl)benzyl)benzamide |
| 25 | 3-(((7-(1H-pyrazol-4-yl)-2,3-dihydrofuro[3,2-c]pyridin-4-yl)amino)methyl)-N-((5-methylthiophen-2-yl)methyl)benzamide |
| 26 | 3-(((7-(1H-pyrazol-4-yl)-2,3-dihydrofuro[3,2-c]pyridin-4-yl)amino)methyl)-N-(1-phenethylpiperidin-4-yl)benzamide |
| 27 | 3-(((7-(1H-pyrazol-4-yl)-2,3-dihydrofuro[3,2-c]pyridin-4-yl)amino)methyl)-N-(1-(2-(dimethylamino)ethyl)-1H-pyrazol-4-yl)benzamide |
| 28 | 3-(((7-(1H-pyrazol-4-yl)-2,3-dihydrofuro[3,2-c]pyridin-4-yl)amino)methyl)-N-(2-(1-(oxetan-3-yl)piperidin-4-yl)ethyl)benzamide |
| 29 | 3-(((7-(1H-pyrazol-4-yl)-2,3-dihydrofuro[3,2-c]pyridin-4-yl)amino)methyl)-N-((5-methyl-4,5,6,7-tetrahydrothiazolo[5,4-c]pyridin-2-yl)methyl)benzamide |
| 30 | 3-(((7-(1H-pyrazol-4-yl)-2,3-dihydrofuro[3,2-c]pyridin-4-yl)amino)methyl)-N-((2-methylisoindolin-5-yl)methyl)benzamide |
| 31 | 3-(((7-(1H-pyrazol-4-yl)-2,3-dihydrofuro[3,2-c]pyridin-4-yl)amino)methyl)-N-(5-methoxypyridin-2-yl)benzamide |
| 32 | 3-(((7-(1H-pyrazol-4-yl)-2,3-dihydrofuro[3,2-c]pyridin-4-yl)amino)methyl)-N-(pyrimidin-4-yl)benzamide |
| 33 | 3-(((7-(1H-pyrazol-4-yl)-2,3-dihydrofuro[3,2-c]pyridin-4-yl)amino)methyl)-N-(6-(dimethylamino)-4,5,6,7-tetrahydrobenzo[d]thiazol-2-yl)benzamide |
| 34 | 3-(((7-(1H-pyrazol-4-yl)-2,3-dihydrofuro[3,2-c]pyridin-4-yl)amino)methyl)-N-(6-(oxetan-3-ylamino)-4,5,6,7-tetrahydrobenzo[d]thiazol-2-yl)benzamide |
| 35 | N-(3-(((7-(1H-pyrazol-4-yl)-2,3-dihydrofuro[3,2-c]pyridin-4-yl)amino)methyl)phenyl)-4-((dimethylamino)methyl)benzamide |
| 36 | N-(3-(((7-(1H-pyrazol-4-yl)-2,3-dihydrofuro[3,2-c]pyridin-4-yl)amino)methyl)phenyl)-5-methyl-4,5,6,7-tetrahydrothiazolo[4,5-c]pyridine-2-carboxamide |
| 37 | N-(3-(((7-(1H-pyrazol-4-yl)-2,3-dihydrofuro[3,2-c]pyridin-4-yl)amino)methyl)phenyl)-5-methyl-4,5,6,7-tetrahydrothiazolo[5,4-c]pyridine-2-carboxamide |
| 38 | N-(3-(((7-(1H-pyrazol-4-yl)-2,3-dihydrofuro[3,2-c]pyridin-4-yl)amino)methyl)phenyl)-5-(2-hydroxyethyl)-4,5,6,7-tetrahydrothiazolo[5,4-c]pyridine-2-carboxamide |
| 39 | N-(3-(((7-(1H-pyrazol-4-yl)-2,3-dihydrofuro[3,2-c]pyridin-4-yl)amino)methyl)phenyl)-1-methyl-1H-indazole-5-carboxamide |
| 40 | N-(3 -(((7-(IH-pyrazol-4-yl)-2,3 -dihydrofuro[3,2-c]pyridin-4-yl)amino)methyl)phenyl)-1,2,3,4-tetrahydroisoquinoline-6-carboxamide |
| 41 | N-(3-(((7-(1H-pyrazol-4-yl)-2,3-dihydrofuro[3,2-c]pyridin-4-yl)amino)methyl)phenyl)-1-methyl-1H-indazole-3-carboxamide |
| 42 | N-(3-(((7-(1H-pyrazol-4-yl)-2,3-dihydrofuro[3,2-c]pyridin-4-yl)amino)methyl)phenyl)-1-methyl-1H-indazole-4-carboxamide |
| 43 | N-(3-(((7-(1H-pyrazol-4-yl)-2,3-dihydrofuro[3,2-c]pyridin-4-yl)amino)methyl)phenyl)-1-(2-(dimethylamino)ethyl)-1H-indazole-5-carboxamide |
| 44 | N-(3-(((7-(1H-pyrazol-4-yl)-2,3-dihydrofuro[3,2-c]pyridin-4-yl)amino)methyl)phenyl)-1-(1-methylpiperidin-4-yl)-1H-indazole-5-carboxamide |
| 45 | N-(3-(((7-(1H-pyrazol-4-yl)-2,3-dihydrofuro[3,2-c]pyridin-4-yl)amino)methyl)phenyl)-2-methyl-1,2,3,4-tetrahydroisoquinoline-6-carboxamide |
| 46 | N-(3-(((7-(1H-pyrazol-4-yl)-2,3-dihydrofuro[3,2-c]pyridin-4-yl)amino)methyl)phenyl)-1-(2-morpholinoethyl)-1H-indazole-5-carboxamide |
| 47 | N-(5-(((7-(1H-pyrazol-4-yl)-2,3-dihydrofuro[3,2-c]pyridin-4-yl)amino)methyl)-2-fluorophenyl)-1,2,3,4-tetrahydroisoquinoline-6-carboxamide |
| 48 | N-(3-(((7-(1H-pyrazol-4-yl)-2,3-dihydrofuro[3,2-c]pyridin-4-yl)amino)methyl)phenyl)-5-(pyrrolidin-1-ylmethyl)thiazole-2-carboxamide |
| 49 | N-(3-(((7-(1H-pyrazol-4-yl)-2,3-dihydrofuro[3,2-c]pyridin-4-yl)amino)methyl)phenyl)-3-((dimethylamino)methyl)benzamide |
| 50 | N-(3-(((7-(1H-pyrazol-4-yl)-2,3-dihydrofuro[3,2-c]pyridin-4-yl)amino)methyl)phenyl)-2-phenylacetamide |
| 51 | N-(3-(((7-(1H-pyrazol-4-yl)-2,3-dihydrofuro[3,2-c]pyridin-4-yl)amino)methyl)phenyl)-7-methyl-5,6,7,8-tetrahydro-1,7-naphthyridine-3-carboxamide |
| 52 | N-(3-(1-((7-(1H-pyrazol-4-yl)-2,3-dihydrofuro[3,2-c]pyridin-4-yl)amino)ethyl)phenyl)acetamide |
| 53 | 3-(((7-(1H-pyrazol-4-yl)-2,3-dihydrofuro[3,2-c]pyridin-4-yl)amino)methyl)-N-(pyridin-4-ylmethyl)benzamide |
| 54 | 3-(((7-(1H-pyrazol-4-yl)-2,3-dihydrofuro[3,2-c]pyridin-4-yl)amino)methyl)-N-(1-benzylpiperidin-4-yl)benzamide |
| 55 | 3-(((7-(1H-pyrazol-4-yl)-2,3-dihydrofuro[3,2-c]pyridin-4-yl)amino)methyl)-N-((tetrahydro-2H-pyran-2-yl)methyl)benzamide (Enantiomer 1) |
| 56 | 3-(((7-(1H-pyrazol-4-yl)-2,3-dihydrofuro[3,2-c]pyridin-4-yl)amino)methyl)-N-((tetrahydro-2H-pyran-2-yl)methyl)benzamide (Enantiomer 2) |
| 57 | 3-(((7-(1H-pyrazol-4-yl)-2,3-dihydrofuro[3,2-c]pyridin-4-yl)amino)methyl)-N-(5-(2-(dimethylamino)ethoxy)pyridin-2-yl)benzamide |

The compounds of the invention, including all the compounds hereabove listed, can be prepared from readily available starting materials using the following general methods and procedures or by using slightly modified processes readily available to those of ordinary skill in the art. Although a particular embodiment of the present invention may be shown or described herein, those skilled in the art will recognize that all embodiments or aspects of the present invention can be prepared using the methods described herein or by using other known methods, reagents and starting materials. When typical or preferred process conditions (i.e. reaction temperatures, times, mole ratios of reactants, solvents, pressures, etc.) are given, other process conditions can also be used unless otherwise stated. While the optimum reaction conditions may vary depending on the particular reactants or solvent used, such conditions can be readily determined by those skilled in the art by routine optimization procedures.

Thus, processes of preparation described below and reported in the following schemes should not be viewed as limiting the scope of the synthetic methods available for the preparation of the compounds of the invention.

In some cases a step is needed in order to mask or protect sensitive or reactive moieties, generally known protective groups (PG) could be employed, in accordance with general principles of chemistry (Protective group in organic syntheses, 3rd ed. T. W. Greene, P. G. M. Wuts). A suitable protective group for intermediates requiring protection of a carboxylic acid (herein reported as PG₁) can be C₁-C₄ esters (PG₁: methyl, isopropyl, tert-butyl or ethyl), preferably methyl. A suitable protective group for intermediates requiring the amino group protection (herein reported as PG₂) can be carbamates such as tert-butylcarbamate (PG₂: tert-butoxycarbonyl or Boc), benzylcarbamate (PG₂: Benzyloxycarbonyl or Cbz), ethylcarbamate (PG₂: ethoxycarbonyl) or methylcarbamate (PG₂: methoxycarbonyl), preferably PG₂ is Boc. A suitable protective group PG₃ for intermediates requiring ring NH protection of five membered heterocycles can be a THP (2-tetrahydrofuranyl) or Boc.

The compounds of formula (I), here reported again for clarity, including all the compounds here above listed, can be usually prepared according to the procedures shown in the schemes below. Where a specific detail or step differs from the general schemes it has been detailed in the specific examples, and/or in additional schemes.

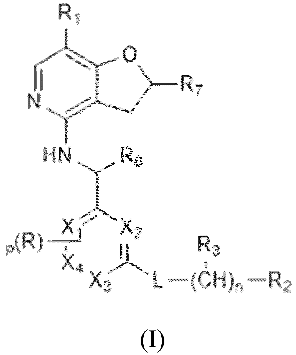

Compounds of formula (I) can contain one or more stereogenic centre. Enantiomerically pure compounds can be prepared according to generally known reactions, e.g. according to the reactions described below, by means of enantiomerically pure starting materials and intermediates. These intermediates may be commercially available or readily produced from commercial sources by those of ordinary skill in the art.

In another approach, enantiomerically pure compounds can be prepared from the corresponding racemates by means of chiral chromatography purification. Stereochemically pure compounds may be obtained by chiral separation from a diastereoisomeric mixture, or (whenever, there are two or more stereogenic centres -i.e. chiral center- in compounds of formula (I)) stepwise by chromatographic separation of diastereoisomers followed by further chiral separation into single stereoisomers.

Examples 1 to 32, 35 to 48, 51 to 52 can be prepared according to *scheme 1* providing at least one non-limiting synthetic route for examples of the invention.

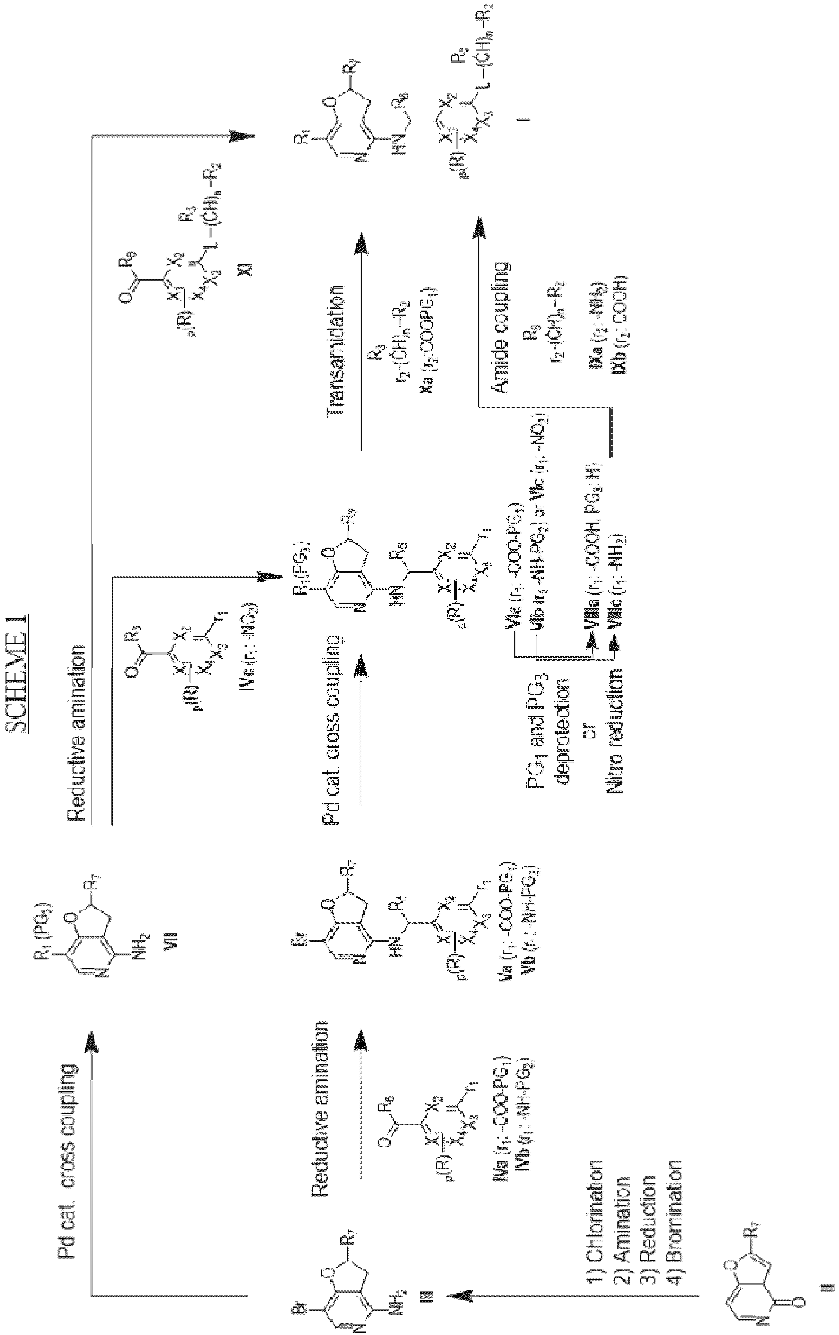

Intermediate **II** can be converted into intermediate **III** by means of four consecutive steps including 1) chlorination, 2) amination, 3) reduction and 4) bromination.

For example, the chlorination step may be carried out by refluxing intermediate **II** with an appropriate chlorinating agent (neat or in solution with an organic solvent such as DCM or dioxane) such as POCl₃ or SOCl₂.

The amination step can be carried out by introducing a masked ammonia such as benzophenone imine through a Buchwald type palladium catalyzed reaction using, for example, tris(dibenzylideneacetone)dipalladium(0)/BINAP catalytic system followed by reaction of the linked benzophenone imine with hydroxylamine to give the corresponding furo[3,2-c]pyridin-4-amine. Reduction of furo[3,2-c]pyridin-4-amine to give 2,3-dihydrofuro[3,2-c]pyridin-4-amine (step 3) can be carried out for example by hydrogenating a solution of the furo[3,2-c]pyridin-4-amine in MeOH / acetic acid in the presence of a Pd/C catalyst under high H₂ pressure (e.g. 10 bar) and at a temperature of 50°C or higher. Finally, intermediate **III** can be obtained by means of bromination of 2,3-dihydrofuro[3,2-c]pyridin-4-amine (step 4) by reaction with a brominating agent such as N-bromosuccinimide in a polar aprotic solvent such as acetonitrile for a few hours at low temperature (e.g. -10 - 0 °C).

Intermediate **III** and carbonyl intermediate **IVa** (or **IVb**) can be combined to give intermediate **Va** (or **Vb**) through a reductive amination reaction that can be performed in an appropriate solvent such as DCM or THF, in the presence of a Lewis acid such as chloro(triisopropoxy)titanium(IV) or titanium tetraisopropoxide(IV) followed by addition of a reducing agent such as sodium triacetoxyborohydride or sodium cyanoborohydride in the presence of an organic acid such as acetic acid or trifluoroacetic acid.

Intermediate **Va** can be converted into intermediate **VIa** by a direct introduction of group R₁ through a metal catalyzed cross coupling reaction such as Suzuki coupling, Stille coupling or similar (Strategic application of named reactions in organic synthesis, L. Kurti, B. Czako, Ed. 2005). For example, a Suzuki coupling can be performed by reacting intermediate **Va** with the corresponding boronic acid or boron pinacolate ester of group R₁ (in some cases R₁ contains a ring NH moiety that needs to be masked to reduce reactivity or for synthetic convenience, it can be opportunely protected with a PG₃ group such as THP or Boc), in the presence of a Pd catalyst such as tris(dibenzylideneacetone)dipalladium(0), PdCl₂(dppf)₂.DCM adduct or tetrakistriphenylphosphinepalladium(0), in an organic solvent such as dioxane, THF or DMF with or without water, with an inorganic base such as an alkaline carbonate (for example Cs₂CO₃) or an inorganic phosphate (for example K₃PO₄), under heating (90-150°C). Boronic acid and boronic pinacolate esters are generally commercially available or may be readily prepared by those skilled in the art starting from commercially available reagents.

Intermediate **VIc** can be prepared from intermediate **VII** and **IVc** by reductive amination using a similar method to that described for the transformation of intermediate **III** into **Va.** Intermediate **VII** can be obtained from intermediate **III** using a similar process to that described above for transformation of intermediate **Va** into intermediate **VIa.**

Removal of PG₁ (when PG₁ is methyl) from intermediate **VIa** to give intermediate **VIIIa** (PG₃ is H) may be carried out by acidic or basic hydrolysis. For example, acidic hydrolysis of PG₁ (when PG₁ is methyl) can be carried out by heating (up to 100°C) **VIa** with a concentrated aqueous acid such as hydrochloric acid or sulfuric acid (if PG₃ is present, it is removed concurrently in the same reaction conditions).

Intermediate **VIc** can be converted into intermediate **VIIIc** by reduction of the nitro group by using a reducing system such as catalytic hydrogenation, redox with Fe/Sn in acidic condition or using hydrides. When PG₃ is THP, reduction of **VIc** to **VIIIc** is performed by catalytic hydrogenation with Pd/C in MeOH in order to retain the NH protecting group.

In another embodiment of the present invention intermediate **VIIIc** can be prepared reacting intermediate **Vb** as done for Va leading to intermediate **VIb** that can be easily converted into **VIIIc** by mean of a simple removal of the protecting group PG2 on the amine.

Reaction between acid intermediate **VIIIa** (PG₃ is H) and amino intermediate **IXa** (or alternatively acid **IXb** with amine **VIIIc**) to give a compound of formula **I** may be carried out under suitable amide coupling reaction conditions. For example, acid intermediate **VIIIa** may be reacted in the presence of an activating agent such as TBTU, HATU or COMU, with an organic base such as DIPEA or TEA, in a suitable organic solvent such as DCM or DMF, and at temperature generally around RT for a time ranging from a few hours to overnight. An alternative amide coupling condition that may be considered involves the reaction of intermediate **VIIIa** and **IXa** in the presence of 1-(methylsulfonyl)-1H-benzotriazole as a coupling agent, with an organic base such as TEA, at temperature up to 150°C for a few hours (for example 4 h). Wherein a compound of formula **I** contains in R₂ or R₃ a primary or secondary amine, this amino moiety needs to be masked during the amide coupling step by using suitably protected (generally Boc) intermediates **IXa** or **IXb.**

In another approach, a compound of formula **I** (wherein L is -NHC(O)-) can be synthesized from amino intermediate **VIIIc** and methyl ester intermediate **Xa** by means of a transamidation reaction. For example, this reaction can be carried out by reacting a methyl ester intermediate and an amino intermediate in a suitable organic solvent such as THF or DCM, in the presence of a suitable Lewis acid such as bis(trimethylaluminum)-1,4-diazabicyclo[2.2.2]octane adduct or InCl₃ at temperatures up to 120°C.

In a different approach, a compound of formula **I** can be prepared from intermediate **VII** and intermediate **XI** by means of reductive amination using similar conditions to those described for the transformation of intermediate **III** into intermediate **Va.**

Wherein for convenience the PG₃ group (i.e. THP) on R₁ was kept throughout the synthetic sequence, it can be easily removed by heating (up to 100°C) the protected precursor with concentrated aqueous acid such as hydrochloric acid or sulfuric acid for a time up to 1 h or less.

In another approach, a compound of formula (**I)** can be prepared according to *scheme 2* providing at least one synthetic route for examples 49-50, 53-54 and 57.

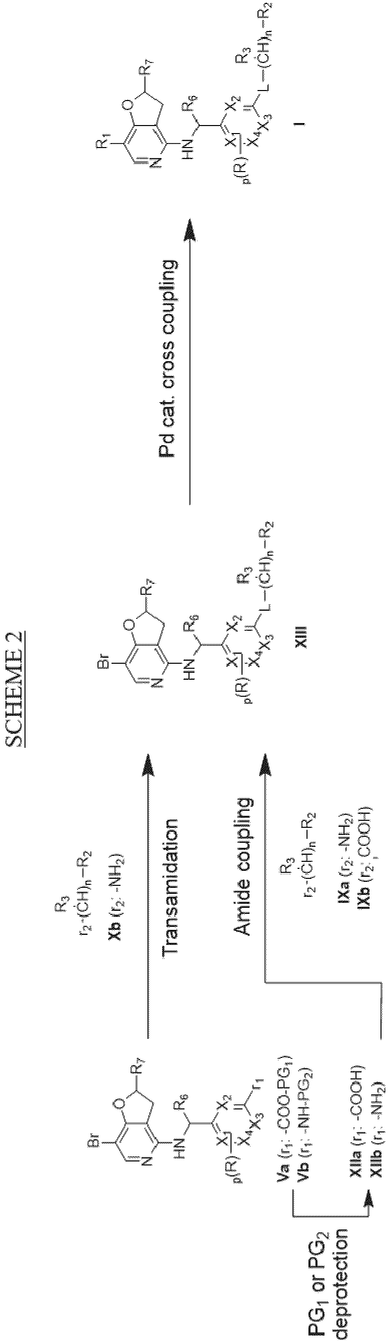

Compounds of formula **I** can be obtained from intermediate **XIII** by a direct introduction of group R₁ through a metal catalyzed cross coupling reaction such as Suzuki coupling, Stille coupling or similar (Strategic application of named reactions in organic synthesis, L. Kurti, B. Czako, Ed. 2005) in the same way (*scheme 1*) as that described for transformation of intermediate **Va** into **VIa.** Wherein for synthetic convenience R₁ boronic acid or pinacolate needs to be protected with a PG₃ group (i.e. THP or Boc), PG₃ can be easily removed by heating (up to 100°C) the protected precursor with concentrated aqueous acid such as hydrochloric acid or sulfuric acid for a time up to 1 h or less.

Intermediate **XIII** can be obtained by an amide coupling between acid intermediate **XIIa** and amino intermediate **IXa** (or acid **IXb** and amine **XIIb**) using similar conditions to those described above (scheme 1) for the reaction of **VIIIa** and intermediate **IXa.** Intermediate **XIIa** and **XIIb** can be obtained from **Va** and **Vb** (described in *scheme 1*) by deprotection of PG₁ or PG₂, respectively.

Deprotection of PG₁ can be performed by basic or acid hydrolysis. Basic hydrolysis of **Va** (when PG₁ is Me or iPr) to give **XIIa** can be performed by means of using an inorganic base such as LiOH or NaOH in a mixture of an organic solvent such as THF and/or methanol with water, generally at RT and for a time ranging from 1 h to overnight.

Removal of PG₂ (when PG₂ is a Boc group) from intermediate **Vb** (for conveninency of description the preparation of **Vb** is reported into Scheme 1) to give the intermediate **XIIb** can be carried out by acidic deprotection. For example, an acidic Boc cleavage can be carried out by treatment with concentrated hydrochloric acid or trifluoroacetic acid.

Intermediate **XIII** (wherein L is -C(O)NH-) can be alternatively obtained from ester intermediate **Va** and amine intermediate **Xb** by a transamidation reaction in a similar manner (scheme 1) to that described for the reaction of intermediate **VIIIc** and amino intermediate **Xa.**

Where for convenience the PG₃ group (i.e. THP) on R₁ was kept throughout the synthetic sequence, it can be easily removed by heating (up to 100°C) the protected precursor with concentrated aqueous acid such as hydrochloric acid or sulfuric acid for a time up to 1 h or less.

Wherein a compound of formula **I** contains in R₂/R₃ a secondary or tertiary amine, or an amide, such compounds can be obtained by further elaboration of a compound of formula **I** (wherein R₂/R₃ contain a primary or secondary amine) by a reductive amination reaction or an amidation using generally accepted methods. Examples 33 and 34 were prepared by elaboration of a precursor of formula **I** containing a primary or secondary amine in R₂/R₃.

As herein described in details, the compounds of the invention are inhibitors of kinase activity, in particular Rho-kinase activity.

In one aspect the invention provides a compound of formula (I) for use as a medicament, preferably for the prevention and /or treatment of pulmonary disease.

In a further aspect the invention provides the use of a compound (I), or a pharmaceutically acceptable salt thereof, in the manufacture of a medicament for the treatment of disorders associated with ROCK enzymes mechanisms, particularly for the treatment of disorders such as pulmonary diseases.

In particular the invention provides compounds of formula (I) for use in the prevention and /or treatment of pulmonary disease selected from the group consisting of asthma, chronic obstructive pulmonary disease (COPD), idiopathic pulmonary fibrosis (IPF), pulmonary hypertension (PH) and specifically Pulmonary Arterial Hypertension (PAH).

Moreover the invention provides a compound of formula (I) for use in a method for the prevention and/or treatment of disorders associated with ROCK enzymes mechanisms, said method comprising administering to a patient in need of such treatment a therapeutically effective amount of a compound of the invention.

In particular the invention provides a compound of formula (I) for use in methods for the prevention and/or treatment wherein the disorder is a respiratory disease selected from asthma, chronic obstructive pulmonary disease (COPD), idiopathic pulmonary fibrosis (IPF), Pulmonary hypertension (PH) and specifically Pulmonary Arterial Hypertension (PAH).

Preferred is the use of the compounds of the invention for the prevention of the aforesaid disorders.

Equally preferred is the use of the compounds of the invention for the treatment of the aforesaid disorders.

Generally speaking, compounds which are ROCK inhibitors may be useful in the treatment of many disorders associated with ROCK enzymes mechanisms.

In one embodiment, the disorders that can be treated by the compounds of the present invention include glaucoma, inflammatory bowel disease (IBD) and pulmonary diseases selected from asthma, chronic obstructive pulmonary disease (COPD), interstitial lung disease such as idiopathic pulmonary fibrosis (IPF) and pulmonary arterial hypertension (PAH).

In another embodiment, the disorder that can be treated by the compound of the present invention is selected from the group consisting of asthma, chronic obstructive pulmonary disease (COPD) and interstitial lung disease such as idiopathic pulmonary fibrosis (IPF) and pulmonary arterial hypertension (PAH).

In a further embodiment, the disorder is selected from idiopathic pulmonary fibrosis (IPF) and pulmonary arterial hypertension (PAH).

The methods of treatment referred to in the description comprise administering a safe and effective amount of a compound of formula (I) or a pharmaceutically acceptable salt thereof to a patient in need thereof. As used herein, "safe and effective amount" in reference to a compound of formula (I) or a pharmaceutically acceptable salt thereof or other pharmaceutically-active agent means an amount of the compound sufficient to treat the patient's condition but low enough to avoid serious side effects and it can nevertheless be routinely determined by the skilled artisan. The compounds of formula (I) or pharmaceutically acceptable salts thereof may be administered once or according to a dosing regimen wherein a number of doses are administered at varying intervals of time for a given period of time. Typical daily dosages may vary depending upon the particular route of administration chosen.

The invention also provides pharmaceutical compositions of compounds of formula (I) in admixture with one or more pharmaceutically acceptable carrier or excipient, for example those described in Remington's Pharmaceutical Sciences Handbook, XVII Ed., Mack Pub., N.Y., U.S.A.

The present invention is also directed to use of the compounds of the invention and their pharmaceutical compositions for various route of administration.

Administration of the compounds of the invention and their pharmaceutical compositions may be accomplished according to patient needs, for example, orally, nasally, parenterally (subcutaneously, intravenously, intramuscularly, intrasternally and by infusion), by inhalation, rectally, vaginally, topically, locally, transdermally, and by ocular administration.

Various solid oral dosage forms can be used for administering compounds of the invention including such solid forms as tablets, gelcaps, capsules, caplets, granules, lozenges and bulk powders. The compounds of the present invention can be administered alone or combined with various pharmaceutically acceptable carriers, diluents (such as sucrose, mannitol, lactose, starches) and known excipients, including suspending agents, solubilizers, buffering agents, binders, disintegrants, preservatives, colorants, flavorants, lubricants and the like. Time release capsules, tablets and gels are also advantageous.

Various liquid oral dosage forms can also be used for administering compounds of the invention, including aqueous and non-aqueous solutions, emulsions, suspensions, syrups, and elixirs. Such dosage forms can also contain suitable known inert diluents such as water and suitable known excipients such as preservatives, wetting agents, sweeteners, flavorants, as well as agents for emulsifying and/or suspending the compounds of the invention. The compounds of the present invention may be injected, for example, intravenously, in the form of an isotonic sterile solution. Other preparations are also possible.

Suppositories for rectal administration of the compounds of the invention can be prepared by mixing the compound with a suitable excipient such as cocoa butter, salicylates and polyethylene glycols.

Formulations for vaginal administration can be in the form of cream, gel, paste, foam, or spray formula containing, in addition to the active ingredient, such as suitable carriers, are also known.

For topical administration the pharmaceutical composition can be in the form of creams, ointments, liniments, lotions, emulsions, suspensions, gels, solutions, pastes, powders, sprays, and drops suitable for administration to the skin, eye, ear or nose. Topical administration may also involve transdermal administration via means such as transdermal patches.

Some preferred compounds of the invention exhibit profile suitable for inhalatory route administration.

Optimisation of drugs for inhaled delivery need certain characteristics that allow administered compound to the lung to maintain a sufficient local concentration (lung retention) to exert a pharmacological effect of the desired duration, and non-relevant levels in unwanted compartments (i.e. plasma). To attenuate lung absorpion, one or more features of the compounds need to be optimized such as, and not limited to, minimizing membrane permeability, reducing dissolution rate or introducing a degree of basicity into the compound to enhance binding to the phospholipid-rich lung tissue or through lysosomial trapping. In some embodiments, compounds of invention show one or more of the features above that are desirable for an inhaled compound.

Other preferred compounds of the invention exhibit profile suitable for oral route administration.

Optimization of drugs for oral delivery need certain characteristics that allow orally administered compound to be absorbed by GI (gastrointestinal) tract and to be poorly cleared in order to give a good bioavailability (F%), thus to maintain a sufficient concentration in plasma and target tissues for a time adequate to sustain pharmacological effect. To enhance oral bioavalability, one or more features of the compounds need to be optimized such as, and not limited to, maximizing membrane permeability and reducing metabolic hot spots (optimizing in-vitro clearance). In some embodiments, compounds of invention show one or more of the features above for an oral compound.

For the treatment of the diseases of the respiratory tract, the compounds according to the invention may be administered by inhalation.

Inhalable preparations include inhalable powders, propellant-containing metering aerosols or propellant-free inhalable formulations.

For administration as a dry powder, single- or multi-dose inhalers known from the prior art may be utilized. In that case the powder may be filled in gelatine, plastic or other capsules, cartridges or blister packs or in a reservoir.

A diluent or carrier, usually non-toxic and chemically inert to the compounds of the invention, e.g. lactose or any other additive suitable for improving the respirable fraction may be added to the powdered compounds of the invention.

Inhalation aerosols containing propellant gas such as hydrofluoroalkanes may contain the compounds of the invention either in solution or in dispersed form. The propellant-driven formulations may also contain other ingredients such as co-solvents, stabilizers and optionally other excipients.

The propellant-free inhalable formulations comprising the compounds of the invention may be in the form of solutions or suspensions in an aqueous, alcoholic or hydroalcoholic medium and they may be delivered by jet or ultrasonic nebulizers known from the prior art or by soft-mist nebulizers such as Respimat^{®}.

Further preferably the invention provides compounds of formula (I) and/or pharmaceutical compositions thereof, for use via inhalatory route of administration particularly in the prevention and /or treatment of asthma, chronic obstructive pulmonary disease COPD, idiopathic pulmonary fibrosis (IPF), pulmonary hypertension (PH) and specifically Pulmonary Arterial Hypertension (PAH), preferably in the prevention and /or treatment of asthma, chronic obstructive pulmonary disease COPD, idiopathic pulmonary fibrosis (IPF).

Further preferably the invention provides compounds of formula (I) and/or pharmaceutical compositions thereof, for use via oral route of administration particularly in the prevention and /or treatment of asthma, chronic obstructive pulmonary disease COPD, idiopathic pulmonary fibrosis (IPF), pulmonary hypertension (PH) and specifically Pulmonary Arterial Hypertension (PAH), preferably in the prevention and /or treatment of pulmonary hypertension (PH) and specifically Pulmonary Arterial Hypertension (PAH).

The compounds of the invention , regardless of the route of administration and desease to be treated, can be administered as the sole active agent or in combination (i.e. as co-therapeutic agents administered in fixed dose combination or in combined therapy of separately formulated active ingredients) with other pharmaceutical active ingredients selected from organic nitrates and NO donors; inhaled NO; stimulator of soluble guanylate cyclase (sGC); prostaciclin analogue PGI2 and agonist of prostacyclin receptors; compounds that inhibit the degradation of cyclic guanosine monophosphate (cGMP) and/or cyclic adenosine monophosphate (cAMP), such as inhibitors of phosphodiesterases (PDE) 1, 2, 3, 4 and/or 5, especially PDE 5 inhibitors; human neutrophilic elastase inhibitors; compounds inhibiting the signal transduction cascade, such as tyrosine kinase and/or serine/threonine kinase inhibitors; antithrombotic agents, for example platelet aggregation inhibitors, anticoagulants or profibrinolytic substances; active substances for lowering blood pressure, for example calcium antagonists, angiotensin II antagonists, ACE inhibitors, endothelin antagonists, renin inhibitors, aldosterone synthase inhibitors, alpha receptor blockers, beta receptor blockers, mineralocorticoid receptor antagonists; neutral endopeptidase inhibitor; osmotic agents; ENaC blockers; anti-inflammatories including corticosteroids and antagonists of chemokine receptors; antihistamine drugs; anti-tussive drugs; antibiotics such as macrolide and DNase drug substance and selective cleavage agents such as recombinant human deoxyribonuclease I (rhDNase); agents that inhibit ALK5 and/or ALK4 phosphorylation of Smad2 and Smad3; tryptophan hydroylase 1 (TPH1) inhibitors and multi-kinase inhibitors, beta2-agonists, corticosteroids, anticholinergic or antimuscarinic agents, mitogen-activated protein kinases (P38 MAP kinase) inhibitors, nuclear factor kappa-B kinase subunit beta (IKK2) inhibitors, leukotriene modulators, non-steroidal anti-inflammatory agents (NSAIDs), mucus regulators, mucolytics, expectorant/mucokinetic modulators, peptide mucolytics, inhibitors of JAK, SYK inhibitors, inhibitors of PI3Kdelta or PI3Kgamma and combinations thereof.

In a preferred embodiment, the compounds of the invention are dosed in combination with phosphodiesterase V such as sildenafil, vardenafil and tadalafil; organic nitrates and NO donors (for example sodium nitroprusside, nitroglycerin, isosorbide mononitrate, isosorbide dinitrate, molsidomine or SIN-1 , and inhaled NO); synthetic prostacyclin analogue PGI2 such as iloprost, treprostinil, epoprostenol and beraprost; agonist of prostacyclin receptors such as selexipag and compounds of WO 2012/007539; stimulator of soluble guanylate cyclase (sGC) like riociguat and tyrosine kinase like imatinib, sorafenib and nilotinib and endothelin antagonist (for example macitentan, bosentan, sitaxentan and ambrisentan).

In a further embodiment the compounds of the invention are dosed in combination with beta2-agonists such as salbutamol, salmeterol, and vilanterol, corticosteroids such as fluticasone propionate or furoate, flunisolide, mometasone furoate, rofleponide and ciclesonide, dexametasone, anticholinergic or antimuscarinic agents such as ipratropium bromide, oxytropium bromide, tiotropium bromide, oxybutynin, and combinations thereof.

In a further embodiment the compounds of the invention are dosed in combination with mitogen-activated protein kinases (P38 MAP kinase) inhibitors, nuclear factor kappa-B kinase subunit beta (IKK2) inhibitors, leukotriene modulators, non-steroidal anti-inflammatory agents (NSAIDs), mucus regulators, mucolytics, expectorant/mucokinetic modulators, peptide mucolyticsinhibitors of JAK, SYK inhibitors, inhibitors of PI3Kdelta or PI3Kgamma.

The invention is also directed to a kit comprising the pharmaceutical compositions of compounds of the invention alone or in combination with or in admixture with one or more pharmaceutically acceptable carriers and/or excipients and a device which may be a single- or multi-dose dry powder inhaler, a metered dose inhaler or a nebulizer.

The dosages of the compounds of the invention depend upon a variety of factors including the particular disease to be treated, the severity of the symptoms, the route of administration, the frequency of the dosage interval, the particular compound utilized, the efficacy, toxicology profile, and pharmacokinetic profile of the compound.

Advantageously, the compounds of formula (I) can be administered for example, at a dosage comprised between 0.001 and 10000 mg/day, preferably between 0.1 and 500 mg/day.

When the compounds of formula (I) are administered by inhalation route, they are preferably given at a dosage comprised between 0.001 and 500 mg/day, preferably between 0.1 and 100 mg/day.

A pharmaceutical composition comprising a compound of the invention suitable to be administered by inhalation is in various respirable forms, such as inhalable powders (DPI), propellant-containing metering aerosols (PMDI) or propellant-free inhalable formulations (e.g. UDV).

The invention is also directed to a device comprising the pharmaceutical composition comprising a compound according to the invention, which may be a single- or multi-dose dry powder inhaler, a metered dose inhaler and a nebulizer particularly soft mist nebulizer.

Although for the treatment of the diseases of the respiratory tract, the compounds according to the invention can be administered by inhalation; they may be in some case preferably be administered by the oral route.

When the compounds of formula (I) are administered by oral route, they are preferably given at a dosage comprised from 0.001 mg to 100 mg per kg body weight of a human, often 0.01 mg to about 50 mg per kg, for example 0.1 to 10 mg per kg, in single or multiple doses per day.

A pharmaceutical composition comprising a compound of the invention suitable to be administered by the oral route can be in various solid or liquid forms, such as tablets, gelcaps, capsules, caplets, granules, lozenges and bulk powders or aqueous and non-aqueous solutions, emulsions, suspensions, syrups, and elixirs formulations.

The following examples illustrate the invention in more detail.

### PREPARATION OF INTERMEDIATES AND EXAMPLES

### General Experimental details

Chemical Names of the compounds were generated with Structure To Name Enterprise 10.0 Cambridge Software or latest.

Purification by 'chromatography' or 'flash chromatography' refers to purification using the Biotage SPl purification system or equivalent MPLC system using a pre-packed polypropylene column containing unbounded activated silica with irregular particles with average size of 50 µm and nominal 60Å porosity. When 'NH-silica' and 'C18-silica' are specified, they refer respectively to aminopropyl chain bonded silica and octadecyl carbon chain (C18)-bonded silica. Fractions containing the required product (identified by TLC and/or LCMS analysis) were pooled and concentrated *in vacuo* or freeze-dried.

Where an Isolute^{®} SCX-2 cartridge was used, 'Isolute^{®} SCX-2 cartridge' refers to a pre-packed polypropylene column containing a non-end-capped propylsulphonic acid functionalised silica strong cation exchange sorbent.

### LCMS Methods

### Method 1

| | | | |
|---|---|---|---|
| Instrumentation | Acquity H-Class (quaternary pump/PDA detector) + QDa Mass Spectrometer | | |
| Column | CSH C18 1.7µm, 50 × 2.1mm at 40°C | | |
| Mobile Phase A | 0.1% Formic acid in water (v/v) | | |
| Mobile Phase B | 0.1% Formic acid in acetonitrile (v/v) | | |
| Flow | 1.0 mL/min | | |
| Gradient Program | Time (min) | % A | %B |
| | 0.0 | 97 | 03 |
| | 1.5 | 01 | 99 |
| | 1.9 | 01 | 99 |
| | 2.0 | 97 | 03 |
| | 2.5 | 97 | 03 |
| Detectors | UV, diode array 190-400nm | | |
| | | | MS ionisation method - Electrospray (positive/negative ion) |

### Method 2

| | | | |
|---|---|---|---|
| Instrumentation | Waters Acquity Classic + 996 PDA detector + Waters ZMD Mass Spectrometer | | |
| Column | CSH C18 1.7µm, 50 × 2.1mm at 40°C | | |
| Mobile Phase A | 0.1% Formic acid in water (v/v) | | |
| Mobile Phase B | 0.1% Formic acid in acetonitrile (v/v) | | |
| Flow | 1.0mL/min | | |
| Gradient Program | Time (min) | % A | %B |
| | 0.0 | 97 | 3 |
| | 0.15 | 97 | 3 |
| | 2.3 | 1 | 99 |
| | 2.4 | 1 | 99 |
| | 2.5 | 97 | 3 |
| Detectors | UV, diode array 190-400nm | | |
| | MS ionisation method - Electrospray (positive/negative ion) | | |

### Method 3

| | | | |
|---|---|---|---|
| Instrumentation | UPLC + Waters DAD + Waters SQD2, single quadrupole UPLC-MS | | |
| Column | HSS C18 1.8µm 100 x 2.1mm. (Plus guard cartridge), maintained at 40°C | | |
| Mobile Phase A | 0.1% Formic acid in water (v/v) | | |
| Mobile Phase B | 0.1% Formic acid in acetonitrile (v/v) | | |
| Flow | 0.4mL/min | | |
| Gradient Program | Time (min) | % A | %B |
| | 0.0 | 95 | 05 |
| | 0.4 | 95 | 05 |
| | 6.0 | 05 | 95 |
| | 6.8 | 05 | 95 |
| | 7.0 | 95 | 05 |
| | 8.0 | 95 | 05 |
| Detectors | UV, diode array 210nm-400nm | | |
| | MS ionisation method - Electrospray (positive/negative ion) | | |

### Method 4

| | | | |
|---|---|---|---|
| Instrumentation | UPLC + Waters DAD + Waters SQD2, single quadrupole UPLC-MS | | |
| Column | BEH Shield RP18 1.7µm 100 x 2.1mm. (Plus guard cartridge), maintained at 40°C | | |
| Mobile Phase A | Aqueous 10mM ammonium hydrogen carbonate | | |
| Mobile Phase B | Acetonitrile | | |
| Flow | 0.4mL/min | | |
| Gradient Program | Time (min) | % A | %B |
| | 0.0 | 95 | 05 |
| | 0.4 | 95 | 05 |
| | 6.0 | 05 | 95 |
| | 6.8 | 05 | 95 |
| | 7.0 | 95 | 05 |
| | 8.0 | 95 | 05 |
| Detectors | UV, diode array 210nm-400nm | | |
| | MS ionisation method - Electrospray (positive/negative ion) | | |

### Method 5

| | | | |
|---|---|---|---|
| Instrumentation | Acquity i-Class (quarternary pump/PDA detector) + Quattro Micro Mass Spectrometer | | |
| Column | BEH C18 1.7µm, 100 × 2.1mm, maintained at 40°C | | |
| Mobile Phase A | 0.1% Formic acid in water (v/v) | | |
| Mobile Phase B | 0.1% Formic acid in acetonitrile (v/v) | | |
| Flow | 0.4mL/min | | |
| Gradient Program | Time (min) | % A | %B |
| | 0.0 | 95 | 05 |
| | 0.4 | 95 | 05 |
| | 6.0 | 05 | 95 |
| | 6.8 | 05 | 95 |
| | 7.0 | 95 | 05 |
| | 8.0 | 95 | 05 |
| Detectors | UV, diode array 200-500nm | | |
| | MS ionisation method - Electrospray (positive/negative ion) | | |

### Method 6

| | | | |
|---|---|---|---|
| Instrumentation | Acquity UPLC (binary pump/PDA detector) + QDa Mass Spectrometer | | |
| Column | CSH C18 1.7µm, 50 × 2.1mm, at 40°C | | |
| Mobile Phase A | 0.05% Formic acid (v/v) in 95/5 water/acetonitrile | | |
| Mobile Phase B | 0.05% Formic acid (v/v) in 5/95 water/acetonitrile | | |
| Flow | 1.0 mL/min | | |
| Gradient Program | Time (min) | % A | %B |
| | 0.0 | 95 | 05 |
| | 1.50 | 05 | 95 |
| | 1.90 | 05 | 95 |
| | 2.0 | 05 | 95 |
| | 2.3 | 05 | 95 |
| Detectors | UV, diode array 200-500nm | | |
| | MS ionisation method - Electrospray (positive/negative ion) | | |

### Method 7 and Method 8

| | | | |
|---|---|---|---|
| Instrumentation | Shimadzu LCMS-2020 Single Quadrupole Liquid Chromatograph Mass Spectromete | | |
| Column | Aquity HSS C18 1.8µm, 50 × 2.1mm, at 25°C | | |
| Mobile Phase A | 0.1% Formic acid (v/v) in water | | |
| Mobile Phase B | 0.1% Formic acid (v/v) in acetonitrile | | |
| Flow | 0.5 mL/min | | |
| Gradient Program | Time (min) | % A | %B |
| | 0.00 | 95 | 05 |
| | 4.00 | 05 | 95 |
| | 5.00 | 05 | 95 |
| | 5.20 | 95 | 05 |
| | 6.00 | 95 | 05 |
| Detectors | UV, 254 nm and 214 nm (method 13) | | |
| | UV, 254 nm and 220 nm (method 14) | | |
| | MS ionisation method - Electrospray (positive/negative ion) | | |

### Method 9

| | | | |
|---|---|---|---|
| Instrumentation | Shimadzu LCMS-2020 Single Quadrupole Liquid Chromatograph Mass Spectrometer | | |
| Column | Aquity HSS C18 1.8µm, 50 × 2.1mm, at 25°C | | |
| Mobile Phase A | 0.1% Formic acid (v/v) in water | | |
| Mobile Phase B | 0.1% Formic acid (v/v) in acetonitrile | | |
| Flow | 0.5 mL/min | | |
| Gradient Program | Time (min) | % A | %B |
| | 0.00 | 95 | 05 |
| | 10.00 | 05 | 95 |
| | 10.50 | 05 | 95 |
| | 11.00 | 95 | 05 |
| | 12.00 | 95 | 05 |
| Detectors | UV, 254 nm and 214 nm | | |
| | MS ionisation method - Electrospray (positive/negative ion) | | |

### Method 10

| | | | |
|---|---|---|---|
| Instrumentation | Agilent Technologies 1260 Infinity II with DAD detector / Agilent Technologies InfinityLab LC/MSD | | |
| Column | BEH C18 1.7µm, 50 × 2.1mm, at 25°C | | |
| Mobile Phase A | 0.05% Aqueous ammonium hydroxide (v/v) | | |
| Mobile Phase B | acetonitrile | | |
| Flow | 0.5 mL/min | | |
| Gradient Program | Time (min) | % A | %B |
| | 0.00 | 80 | 20 |
| | 5.00 | 70 | 30 |
| | 5.60 | 70 | 30 |
| | 5.90 | 05 | 95 |
| | 7.10 | 05 | 95 |
| | 7.50 | 80 | 20 |
| | 9.00 | 80 | 20 |
| Detectors | UV, Diode array 190 - 400 nm | | |
| | MS ionisation method - Electrospray (positive/negative ion) | | |

### Method 11

| | | | |
|---|---|---|---|
| Instrumentation | Acquity UPLC (binary pump/PDA detector) + QDa Mass Spectrometer | | |
| Column | CSH C18 1.7µm, 50 × 2.1mm, at 40°C | | |
| Mobile Phase A | 0.05% Formic acid (v/v) in 95/5 water/acetonitrile | | |
| Mobile Phase B | 0.05% Formic acid (v/v) in 5/95 water/acetonitrile | | |
| Flow | 1.0 mL/min | | |
| Gradient Program | Time (min) | % A | %B |
| | 0.0 | 95 | 05 |
| | 3.50 | 05 | 95 |
| | 3.90 | 05 | 95 |
| | 4.00 | 05 | 95 |
| | 4.3 | 05 | 95 |
| Detectors | UV, diode array 200-500nm | | |
| | MS ionisation method - Electrospray (positive/negative ion) | | |

### Method 12

| | | | |
|---|---|---|---|
| Instrumentation | Acquity UPLC (binary pump/PDA detector) + QDa Mass Spectrometer | | |
| Column | BEH C18 1.7µm, 50 × 2.1mm, at 40°C | | |
| Mobile Phase A | 0.05% Formic acid (v/v) in 95/5 water/acetonitrile | | |
| Mobile Phase B | 0.05% Formic acid (v/v) in 5/95 water/acetonitrile | | |
| Flow | 1.0 mL/min | | |
| Gradient Program | Time (min) | % A | %B |
| | 0.0 | 95 | 05 |
| | 1.50 | 05 | 95 |
| | 1.90 | 05 | 95 |
| | 2.0 | 05 | 95 |
| | 2.3 | 05 | 95 |
| Detectors | UV, diode array 200-500nm | | |
| | MS ionisation method - Electrospray (positive/negative ion) | | |

### Method 13

| | | | |
|---|---|---|---|
| Instrumentation | Acquity UPLC (binary pump/PDA detector) + QDa Mass Spectrometer | | |
| Column | CSH C18 1.7µm, 50 × 2.1mm, at 50°C | | |
| Mobile Phase A | Aqueous ammonmium formate (25mM) pH 3 | | |
| Mobile Phase B | 0.1% Formic acid in acetonitrile | | |
| Flow | 0.35 mL/min | | |
| Gradient Program | Time (min) | % A | %B |
| | 0.00 | 99 | 01 |
| | 0.50 | 99 | 01 |
| | 3.00 | 70 | 30 |
| | 6.50 | 50 | 50 |
| | 7.50 | 20 | 80 |
| | 8.10 | 99 | 01 |
| | 10.00 | 99 | 01 |
| Detectors | UV, diode array 200-500nm | | |
| | MS ionisation method - Electrospray (positive/negative ion) | | |

### Method 14

| | | | |
|---|---|---|---|
| Instrumentation | Acquity UPLC (binary pump/PDA detector) + QDa Mass Spectrometer | | |
| Column | Kinetex C8 1.7µm, 50 × 2.1mm, at 40°C | | |
| Mobile Phase A | 0.05% Formic acid (v/v) in 95/5 water/acetonitrile | | |
| Mobile Phase B | 0.05% Formic acid in (v/v) 5/95 water/acetonitrile | | |
| Flow | 1.0 mL/min | | |
| Gradient Program | Time (min) | % A | %B |
| | 0.0 | 95 | 05 |
| | 1.50 | 05 | 95 |
| | 1.90 | 05 | 95 |
| | 2.0 | 05 | 95 |
| | 2.3 | 05 | 95 |
| Detectors | UV, diode array 200-500nm | | |
| | MS ionisation method - Electrospray (positive/negative ion) | | |

### Method 15

| | | | |
|---|---|---|---|
| Instrumentation | Acquity UPLC (binary pump/PDA detector) + QDa Mass Spectrometer | | |
| Column | Kinetex C8 1.7µm, 50 × 2.1mm, at 40°C | | |
| Mobile Phase A | 0.05% Formic acid (v/v) in 95/5 water/acetonitrile | | |
| Mobile Phase B | 0.05% Formic acid (v/v) in 5/95 water/acetonitrile | | |
| Flow | 1.0 mL/min | | |
| Gradient Program | Time (min) | % A | %B |
| | 0.0 | 95 | 05 |
| | 3.50 | 05 | 95 |
| | 3.90 | 05 | 95 |
| | 4.00 | 05 | 95 |
| | 4.3 | 05 | 95 |
| Detectors | UV, diode array 200-500nm | | |
| | MS ionisation method - Electrospray (positive/negative ion) | | |

### Method 16

| | | | |
|---|---|---|---|
| Instrumentation | Acquity H-Class (quaternary pump/PDA detector) + QDa Mass Spectrometer | | |
| Column | Acquity BEH C18 1.7µm, 50 × 2.1mm at 40°C | | |
| Mobile Phase A | 0.03% Aqueous ammonia (v/v) (7.66mM) | | |
| Mobile Phase B | 0.03% ammonia in Acetonitrile (v/v) (7.66mM) | | |
| Flow | 0.8 mL/min | | |
| Gradient Program | Time (min) | % A | %B |
| | 0.0 | 97 | 3 |
| | 1.5 | 3 | 97 |
| | 2.3 | 3 | 97 |
| | 2.4 | 97 | 3 |
| | 2.5 | 97 | 3 |
| Detectors | UV, diode array 190-400nm | | |
| | MS ionisation method - Electrospray (positive/negative ion) | | |

### NMR Methods

NMR spectra were obtained on a Bruker Avance 400 MHz, 5mm QNP probe H, C, F, P, single Z gradient, two channel instrument running TopSpin 2.1, or on a Bruker Avance III 400 MHz, 5mm BBFO Plus probe, single Z gradient, two channel instrument running TopSpin 3.0, or on a Varian Unity Inova 400 spectrometer with a 5 mm inverse detection triple resonance probe operating at 400 MHz. Chemical shift are reported as δ values in ppm relative to tetramethylsilane. Coupling constants (J values) are given in hertz (Hz) and multiplicities are reported using the following abbreviation: s=singlet, d=doublet, t=triplet, q=quartet, m=multiplet, br=broad, nd=not determined.

### SFC Methods

Where compounds were purified using Supercritical Fluid Chromatography (SFC) either a Waters Thar Prep100 preparative SFC system (P200 CO₂ pump, 2545 modifier pump, 2998 UV/VIS detector, 2767 liquid handler with Stacked Injection Module) or a Waters Thar Investigator semi preparative system (Waters Fluid Delivery Module, 2998 UV/VIS detector, Waters Fraction Collection Module) was used. The compounds were purified using the column and conditions specified and fractions that contained the desired product were concentrated by vacuum centrifugation.

The modifier used under basic conditions was diethyl amine (0.1% V/V). Alternate modifiers such as formic acid (0.1% V/V), acetic acid (0.1% V/V), were used as an acidic modifier.

### MDAP Methods

Compounds were purified by reverse phase HPLC using a Waters Fractionlynx preparative HPLC system (2525 pump, 2996/2998 UV/VIS detector, 2767 liquid handler) or Gilson preparative HPLC system (322 pump, 155 UV/VIS detector, GX-281 liquid handler) or equivalent system. Collection was triggered by a threshold absorbance value at 260 nm and the presence of target molecular ion as observed under ESI conditions. The fractions that contained the desired product were lyophilized. The specific details of the conditions used, including the column, solvents, gradient and modifier (acidic or basic), are provided for some examples and merely provided for assistance. When specific conditions are not provided, they can be readily optimized by those skilled in the art.

In the procedures that follow, some of the starting materials are identified through an "Intermediate" or "Example" number with indications on step name. When reference is made to the use of a "similar" or "analogous" procedure, as will be appreciated by those skilled in the art, such a procedure may involve minor variations, for example reaction temperature, reagent/solvent amount, reaction time, work-up conditions or chromatographic purification conditions.

The stereochemistry of the compounds in the Examples, where indicated, has been assigned on the assumption that absolute configuration at resolved stereogenic centers of starting materials is maintained throughout any subsequent reaction conditions. All solvents and commercial reagents were used as received. Where the preparation of starting materials is not described, these are commercially available, known in the literature, or readily obtainable by those skilled in the art using standard procedures.

### Abbreviations

ACN (acetonitrile), BINAP (2,2'-Bis(diphenylphosphino)-1,1'-binaphthalene), COMU ((1-Cyano-2-ethoxy-2-oxoethylidenaminooxy)dimethylamino-morpholino-carbenium hexafluorophosphate), DCM (dichloromethane), DIPEA or DIEA (N-Ethyldiisopropylamine), DMF (N,N-Dimethylformamide), DMSO (Dimethylsulfoxide), dppf (1,1'-Ferrocenediyl-bis(diphenylphosphine)), EtOH (ethanol), EtOAc (ethyl acetate), FA (Formic acid), HATU (1-[Bis(dimethylamino)methylene]-1H-1,2,3-triazolo[4,5-b]pyridinium 3-oxid hexafluorophosphate, N-[(Dimethylamino)-1H-1,2,3-triazolo-[4,5-b]pyridin-1-ylmethylene]-N-methylmethanaminium hexafluorophosphate N-oxide), HPLC (High performance liquid chromatography), LCMS ( Liquid chromatography - mass spectrometry), MDAP (Mass-directed auto-purification), MeOH (methanol), Me-THF (2-Methyltetrahydrofuran), MTBE (methyl tert-butyl ether), NMP (N-methylpyrrolidone), NMR (Nuclear magnetic resonance), Rt (Retention time), RT (Room temperature), SCX (Strong cation exchange), TBTU (2-(1H-Benzotriazole-1-yl)-1,1,3,3-tetramethylaminium tetrafluoroborate), TFA (Trifluoroacetic acid), THF (Tetrahydrofuran), XPhos Pd G2 Chloro(2-dicyclohexylphosphino-2',4',6'-triisopropyl-1,1'-biphenyl)[2-(2'-amino-1,1'-biphenyl)]palladium(II).

### PREPARATION OF INTERMEDIATES

### Intermediate A1

### Step A

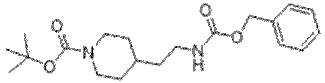

### tert-Butyl 4-(2-(((benzyloxy)carbonyl)amino)ethyl)piperidine-1-carboxylate (Intermediate A1-a)

tert-Butyl 4-(2-aminoethyl)piperidine-1-carboxylate (0.3 g, 1.3 mmol) was dissolved in DCM (4.0 mL), then benzyl (2,5-dioxopyrrolidin-1-yl) carbonate (0.33 g, 1.3 mmol) was added under stirring followed by triethylamine (0.18 mL, 1.3 mmol). The solution was stirred at RT for 3 h. The reaction mixture was diluted with DCM (30 mL) and the organic layer washed with saturated aqueous NH₄Cl (50 mL), then saturated aqueous NaHCO₃ (50 mL), dried over Na₂SO₄ and concentrated to dryness to give the title compound (0.48g) that was used in the next step without further purification.

LCMS (Method 6): Rt = 1.22 min, m/z 385.1 [M+Na]⁺

### Step B

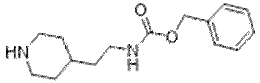

### Benzyl (2-(piperidin-4-yl)ethyl)carbamate (Intermediate A1-b)

Intermediate A1-a (0.48 g, 1.313 mmol) was dissolved in a mixture of DCM (15 mL) / TFA (5 mL) at 0°C and the solution stirred at RT for 3 h. The reaction mixture was dried under reduced pressure and the crude purified on an Isolute^{®} SCX-2 cartridge to give the title compound (0.36 g).

LCMS (Method 6): Rt = 0.41 min, m/z 263.0 [M+H]⁺

### Step C

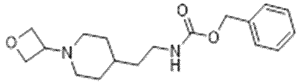

### Benzyl (2-(1-(oxetan-3-yl)piperidin-4-yl)ethyl)carbamate (Intermediate A1-c)

Intermediate A1-b (0.20 g, 0.76 mmol) and oxetan-3-one (0.055 g, 0.76 mmol) in DCM (10 mL) was stirred at RT for 15 min, then sodium triacetoxyborohydride (0.24 g, 1.14 mmol) was added in one portion. The solution was stirred at RT for 4 h, then diluted with DCM, and the organic phase washed with saturated aqueous NaHCO₃, dried over Na₂SO₄, concentrated to dryness and purified through an Isolute^{®} SCX-2 cartridge to give the title compound (0.24 g).

LCMS (Method 6): Rt = 0.43 min, m/z 319.3 [M+H]⁺

### Step D

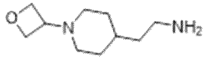

### 2-(1-(Oxetan-3-yl)piperidin-4-yl)ethan-1-amine (Intermediate A1)

Intermediate A1-c (0.10 g, 0.31 mmol), acetic acid (0.019 g, 0.31 mmol) and Pd/C (0.100 g, 0.942 mmol) in EtOH (5 mL) was degassed under vacuum/H₂ cycles (3 times), then hydrogenated at RT for 4 h. The reaction mixture was filtered and evaporated to dryness to give the title compound (0.058 g) that was used in the next step without further purification.

LCMS (Method 6): Rt = 0.12 min, m/z 185.3 [M+H]⁺

### Intermediate A2

### Step A

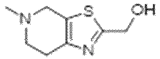

### (5-Methyl-4,5,6,7-tetrahydrothiazolo[5,4-c]pyridin-2-yl)methanol

### (Intermediate A2-a)

To a 0°C cooled suspension of 5-methyl-4,5,6,7-tetrahydrothiazolo[5,4-c]pyridine-2-carboxylic acid hydrochloride (0.2 g, 0.85 mmol) in THF (5 mL), lithium aluminium hydride solution in THF (1.0 mL, 1.00 mmol) was added dropwise and stirring was continued at RT for 1 h. The reaction was quenched by the addition of saturated aqueous Na₂SO₄, the mixture filtered, dried over Na₂SO₄ and concentrated to dryness to give the title compound (0.125 g).

LCMS (Method 6): Rt = 0.13 min, m/z 185.0 [M+H]⁺

### Step B

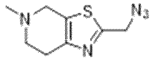

### 2-(Azidomethyl)-5-methyl-4,5,6,7-tetrahydrothiazolo[5,4-c]pyridine

### (Intermediate A2-b)

Intermediate A2-a (0.125 g, 0.68 mmol), diphenyl phosphorazidate (0.146 mL, 0.68 mmol) and 1,8-diazabicyclo[5.4.0]undec-7-ene (0.101 mL, 0.68 mmol) were dissolved in anhydrous THF (5 mL) and the resulting solution heated at 90°C under microwave irradiation for 1 h. The reaction mixture was evaporated to dryness and purified on NH-silica by elution with 0-10% MeOH in DCM to give the title compound (80 mg).

LCMS (Method 6): Rt = 0.14 min, m/z 210.0 [M+H]⁺

### Step C

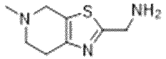

### (5-Methyl-4,5,6,7-tetrahydrothiazolo[5,4-c]pyridin-2-yl)methanamine

### (Intermediate A2)

Intermediate A2-b (0.08 g, 0.38 mmol) was dissolved in a mixture of THF (5 mL) and water (0.5 mL), then triphenylphosphine (0.301 g, 1.15 mmol) was added and the solution was refluxed for 2 h. The mixture was dried under reduced pressure and purified on an Isolute^{®} SCX-2 cartridge to give the title compound (15 mg).

LCMS (Method 6): Rt = 0.10 min, m/z 184.0 [M+H]⁺

### Intermediate A3

### Step A

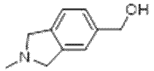

### (2-Methylisoindolin-5-yl)methanol (Intermediate A3-a)

Intermediate A3-a was prepared using a procedure similar to that used for the synthesis of intermediate A2-a by replacing 5-methyl-4,5,6,7-tetrahydrothiazolo[5,4-c]pyridine-2-carboxylic acid hydrochloride of *step a* with methyl 2-methylisoindoline-5-carboxylate.

### LCMS (Method 6): Rt = 0.13 min, m/z 164.0 [M+H]⁺

### Step B

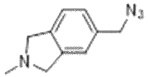

### 5-(Azidomethyl)-2-methylisoindoline (Intermediate A3-b)

Intermediate A3-b was prepared using a procedure similar to that used for the synthesis of intermediate A2-b by replacing intermediate A2-a of *step b* with intermediate A3-a.

LCMS (Method 6): Rt = 0.20 min, m/z 189.1 [M+H]⁺

### Step C

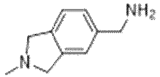

### (2-Methylisoindolin-5-yl)methanamine (Intermediate A3)

Intermediate A3 was prepared using a procedure similar to that used for the synthesis of intermediate A2 by replacing intermediate A2-b of *step* c with intermediate A3-b.

LCMS (Method 6): Rt = 0.10 min, m/z 163.0 [M+H]⁺

### Intermediate A4

### Step A

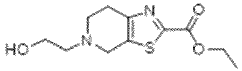

### Ethyl 5-(2-hydroxyethyl)-4,5,6,7-tetrahydrothiazolo[5,4-c]pyridine-2-carboxylate (Intermediate A4-a)

Ethyl 4,5,6,7-tetrahydrothiazolo[5,4-c]pyridine-2-carboxylate (150 mg, 0.707 mmol) and cesium carbonate (691 mg, 2.120 mmol) were dissolved in acetonitrile (6 mL) and reacted for 10 min before addition of potassium iodide (58.7 mg, 0.353 mmol) and 2-bromoethanol (0.201 mL, 1.413 mmol). The reaction mixture was stirred for 7 h at 75°C, then filtered and evaporated to dryness. The resulting crude material was purified by flash chromatography on NH-silica by eluting with 0-10% MeOH in DCM. Appropriate fractions were evaporated to give the desired product (167 mg).

### LCMS (Method 6): Rt = 0.16 min, m/z 257.0 [M+H]⁺

### Step B

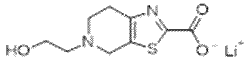

### Lithium 5-(2-hydroxyethyl)-4,5,6,7-tetrahydrothiazolo[5,4-c]pyridine-2-carboxylate (Intermediate A4)

Intermediate A4-b was dissolved in THF (2.5 mL) and water (2.5 mL) then lithium hydroxide (78 mg) was added and the mixture stirred for 1 h at RT. The solvent was evaporated under vacuum to give the title compound (265 mg).

LCMS (Method 6): Rt = 0.14 min, m/z 228.8 [M+H]⁺

### Intermediate A5

### Step A

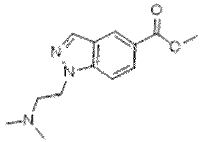

### Methyl 1-(2-(dimethylamino)ethyl)-1H-indazole-5-carboxylate (Intermediate A5-a)

To a solution of methyl 1H-indazole-5-carboxylate (250 mg, 1.419 mmol) and N,N-dimethylethanolamine (0.289 mL, 2.84 mmol) in toluene (6 mL) was added 2-(trimethylphosphoranylidene)acetonitrile solution 0.5 M in THF (5.68 mL, 2.84 mmol). The reaction mixture was stirred at 110°C for 18 h. The solvent was evaporated under vacuum and then the crude was taken in EtOAc and washed with saturated aqueous NaHCO₃ solution. The organic phase was evaporated under vacuum and purified by flash chromatography on NH-silica by eluting with 1/1 heptane/EtOAc + 1% triethylamine. Appropriate fractions were pooled and dried to give the title compound (249 mg).

LCMS (Method 6): Rt = 0.36 min, m/z 248.2 [M+H]⁺

### Step B

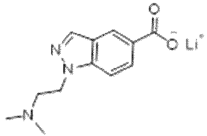

### Lithium 1-(2-(dimethylamino)ethyl)-1H-indazole-5-carboxylate (Intermediate A5)

Intermediate A5-a (249 mg, 1.01 mmol) was dissolved in MeOH (2.5 mL) and water (2.5 mL) then lithium hydroxide (36.2 mg, 1.510 mmol) was added in one portion and the mixture stirred for 1 h at RT. Solvent was evaporated under vacuum to give the desired product (200 mg) that was used in the next step without further elaboration.

LCMS (Method 6): Rt = 0.16 min, m/z 234.2 [M+H]⁺

### Intermediate A6

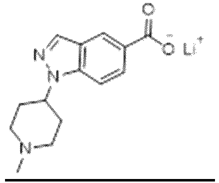

### Lithium 1-(1-methylpiperidin-4-yl)-1H-indazole-5-carboxylate (Intermediate A6)

Intermediate A6 was prepared using a procedure similar to that used for the synthesis of intermediate A-5 by replacing N,N-dimethylethanolamine with 1-methylpiperidin-4-ol.

LCMS (Method 6): Rt = 0.39 min, m/z 260.0 [M+H]⁺

### Intermediate A7

### Step A

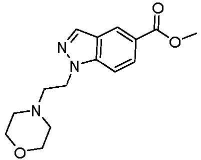

### Methyl 1-(2-morpholinoethyl)-1H-indazole-5-carboxylate (Intermediate A7-a)

Methyl 1H-indazole-5-carboxylate (500 mg, 2.84 mmol) and potassium carbonate (1.18 g, 8.51 mmol) were mixed in DMF (10 mL) and stirred at RT for 20 min, then 4-(2 chloroethyl)morpholine hydrochloride (1.205 mL, 5.68 mmol) was added. The reaction mixture was stirred at 70°C for 24 h. The mixture was diluted with EtOAc (10 mL) and washed with saturated aqueous NaHCO₃ solution (10 mL). The aqueous phase was extracted with EtOAc (2 x 10 mL). The combined organic phases were evaporated under vacuum. The resulting crude material was purified by flash chromatography on NH-silica by eluting with 0-50% EtOAc in hexane. Appropriate fractions were combined and dried to give the desired product (325 mg).

LCMS (Method 6): Rt = 0.38 min, m/z 290.0 [M+H]⁺

### Step B

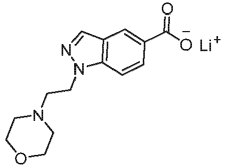

### Lithium 1-(2-morpholinoethyl)-1H-indazole-5-carboxylate (Intermediate A7)

Intermediate A7 was prepared from intermediate A7-a using a procedure similar to that used in *step b* for the synthesis of intermediate A5 from intermediate A5-a.

LCMS (Method 6): Rt = 0.21 min, m/z 275.9 [M+H]⁺

### Intermediate A8

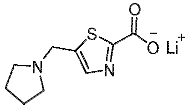

### Lithium 5-(pyrrolidin-1-ylmethyl)thiazole-2-carboxylate (Intermediate A8)

Intermediate A8 was prepared using a procedure similar to that used for the synthesis of intermediate A7 by replacing in *step a* respectively the 4-(2-chloroethyl) morpholine hydrochloride with ethyl 5-(bromomethyl)thiazole-2-carboxylate and methyl 1H-indazole-5-carboxylate with pyrrolidine.

LCMS (Method 6): Rt = 0.14 min, m/z 213.0 [M+H]⁺

### Intermediate B

### Step A

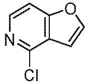

### 4-Chlorofuro[3,2-c]pyridine (Intermediate B-a)

A mixture of furo[3,2-c]pyridin-4-ol (70.4 g, 0.52 mol) in phosphoryl trichloride (430 mL) was heated at reflux for 1 h. Phosphoryl trichloride was distilled off, the residue poured into ice/water and neutralized to pH~6 with aqueous saturated NaHCO₃. The aqueous phase was extracted twice with DCM, then the organic layer was washed with saturated aqueous NaCl and evaporated to dryness. The crude material was purified by column chromatography on silica gel eluting with EtOAc-hexane to give the title compound (72.8 g).

LCMS (Method 7): Rt = 2.71 min, m/z 153.9 [M+H]⁺

### Step B

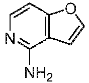

### Furol[3,2-c]pyridin-4-amine (Intermediate B-b)

A solution of intermediate B-a (72.8 g, 0.47 mol) in dry toluene (730 mL) was purged with argon over 20 min, then racemic BINAP (17.72 g, 0.028 mol), tris(dibenzylideneacetone)dipalladium(0) (8.69 g, 0.0095 mol) and potassium tert-butoxide (74.50 g, 0.66 mol) were added. After addition of benzophenone imine (95.5 mL, 0.57 mol), the mixture was heated at 90°C for 1.5 h. The reaction mixture was cooled to RT, diluted with THF and filtered through a pad of diatomaceous earth followed by washing with THF and diethyl ether. The combined filtrate was evaporated and the residue taken into MeOH (260 mL) and added dropwise to a solution of hydroxylamine hydrochloride (98.87 g, 1.42 mol) in MeOH (1200 mL) which had previously been neutralized in an ice bath with NaOH (56.91 g, 1.42 mol). The reaction mixture was stirred at RT for 1 h and evaporated to dryness. The crude material was purified by chromatography on silica by eluting with 10-100% EtOAc in hexane to give a solid that was further purified by trituration and filtration in a mixture of MTBE and DCM. A second purification by chromatography on silica by eluting with 0-10% MeOH in DCM afforded the pure title compound (45.1 g).

LCMS (Method 8): Rt = 0.83 min, m/z 135.0 [M+H]⁺

### Step C

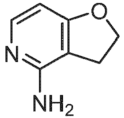

### 2,3-dihydrofuro[3,2-c]pyridin-4-amine (Intermediate B-c)

Intermediate B-b (44.1 g, 0.33 mol) was dissolved in MeOH (530 mL) and acetic acid (56.4 mL), then 10% Pd/C (50% wet, 17.74 g) was added and the reaction mixture purged with argon before being hydrogenated at a pressure of 10 bar of H₂ at 50°C under vigorous stirring. After 20 h a further half equivalent of 10% Pd/C (50% wet) and further 3 h of hydrogenation were needed in order to achieve full conversion. The reaction mixture was filtered and washed with MeOH. The combined filtrate was evaporated and the residue partitioned between EtOAc (500 mL) and water (500 mL). The aqueous layer was washed with further EtOAc (300 mL), neutralized with solid NaHCO₃ and saturated with NaCl. This aqueous mixture was extracted with DCM (8 x 300 mL) and the combined organic layers washed with saturated aqueous NaCl (800 mL), dried over Na₂SO₄ and evaporated to afford the title compound (24.57 g).

LCMS (Method 9): Rt = 0.81 min, m/z 137.1 [M+H]⁺

### Step D

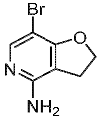

### 7-bromo-2,3-dihydrofuro[3,2-c]pyridin-4-amine (Intermediate B)

Intermediate B-c (24.57 g, 0.180 mol) was dissolved in ACN (1230 mL) and then a solution of N-bromosuccinimide (35.33 g, 0.198 mol) in ACN (490 mL) was added dropwise over 3 h at -10°C in darkness. The reaction was quenched with aqueous saturated NaHCO₃ (500 mL), water (500 mL), EtOAc (1000 mL) and aqueous 5% NaCl (500 mL). The resulting organic and aqueous phases were separated, and the aqueous layer further washed with EtOAc (1000 mL). The combined organic layers were washed with aqueous 5% NaCl (7 x 2000 mL) and concentrated to dryness. The residual solid was treated with a mixture of EtOAc (500 mL) and water (200 mL), placed in a sonic bath for some minutes and acidified with aqueous 10% KHSO₄ (300 mL). The solid that appeared was collected by filtration. The biphasic filtrate was partitioned and the organic layer washed twice with aqueous 10% KHSO₄ (200 mL each). The combined aqueous layer was washed with EtOAc (3 x 500 mL) and mixed with the previous collected solid. The resulting aqueous mixture was neutralized to pH7 with NaHCO₃ and extracted with EtOAc (3 x 1000 mL). The combined organic phase was washed with saturated aqueous NaCl (500 mL), dried over anhydrous MgSO₄, and concentrated to give the title compound as a solid (27.1 g).

LCMS (Method 10): Rt = 1.69 min, m/z 215.0/217.0 [M+H]⁺

### Intermediate C1

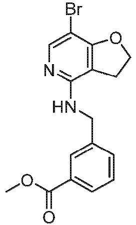

### Methyl 3-(((7-bromo-2,3-dihydrofuro[3,2-c]pyridin-4-yl)amino)methyl)benzoate (Intermediate C1)

Intermediate B (15.6 g, 0.074 mol) and methyl 3-formylbenzoate (18.1g, 0.11 mol) were dissolved in anhydrous DCM (470 mL) with molecular sieves and kept under an inert atmosphere. After 10 min, chloro(triisopropoxy)titanium(IV) (35.4 mL, 0.148 mol) was added dropwise and the resulting mixture stirred at RT over 2.5 h. Sodium triacetoxyborohydride (31.4g, 0.148 mol) followed by acetic acid (8.5 mL, 0.148 mol) were added and the mixture stirred at RT overnight. The reaction mixture was quenched with methanol and the solvents were evaporated. The residue was dissolved in EtOAc and aqueous saturated NaHCO₃ solution. After being stirred for 15 min, the mixture was filtered through a thin pad of diatomaceous earth and washed with EtOAc. The combined filtrate was collected and organic-aqueous phases were separated. The organic layer was dried over Na₂SO₄ and evaporated. The crude material was purified by chromatography on silica by eluting with 20% - 40% EtOAc in hexane to give the title compound (19.3 g).

LCMS (Method 6): Rt = 0.85 min, m/z 362.9/364.9 [M+H]⁺

### Intermediate C2

The following intermediate was prepared using a procedure similar to that used for the synthesis of intermediate C1 by replacing methyl 3-formylbenzoate with the starting material given in the table.

| | **Structure** | **Starting material** | **LC-MS** |
|---|---|---|---|
| C2 | 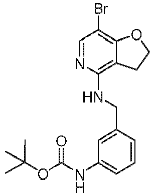 | tert-butyl (3-formylphenyl)carbamate | Rt = 0.87 min, m/z 420.2 / 422.4 [M+H]⁺ (Method 6) |

### Intermediate D1

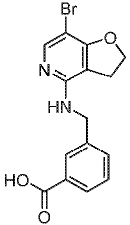

### 3-(((7-Bromo-2,3-dihydrofuro[3,2-c]pyridin-4-yl)amino)methyl)benzoic acid

### (Intermediate D1)

A solution of intermediate C1 (100 mg, 0.27 mmol), lithium hydroxide monohydrate (0.035 g, 0.83 mmol) in THF (1 mL), MeOH (1 mL) and water (2 mL) was stirred at RT for 1.5 h. The resulting mixture was diluted with water and extracted with EtOAc. The pH of the aqueous phase was adjusted to pH ~2-3 with aqueous 1M HCl. The organic layer was dried over Na₂SO₄ and evaporated *in vacuo* to give the product as a solid (89 mg).

LCMS (Method 1): Rt = 0.81 min, m/z 348.9/350.9 [M+H]⁺

### Intermediate D2

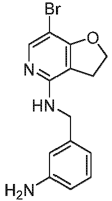

### N-(3-Aminobenzyl)-7-bromo-2,3-dihydrofuro[3,2-c]pyridin-4-amine

### (Intermediate D2)

Intermediate C2 (800 mg) was dissolved in acetonitrile (6 mL) and then added with aqueous HCl 12 M. The solution was stirred at RT and after 1 h a precipitate appeared. The solid was collected by filtration to give the title compound (550 mg).

LCMS (Method 6): Rt = 0.38 min, m/z 320.0/322.0 [M+H]⁺

### Intermediate E1

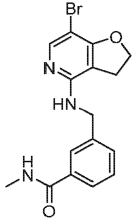

### 3-(((7-Bromo-2,3-dihydrofuro[3,2-c]pyridin-4-yl)amino)methyl)-N-methylbenzamide (Intermediate E1)

A mixture of Intermediate D1 (40 mg, 0.12 mmol), methylamine hydrochloride (23 mg, 0.35 mmol), TBTU (150 mg, 0.46 mmol) and N,N-diisopropylethylamine (0.12 mL, 0.69 mmol) in DCM (4 mL) was stirred at RT for 18 h. The resulting mixture was diluted with water and extracted with DCM. The organic layer was dried over sodium sulphate and evaporated *in vacuo.* The residue, diluted with MeOH, was passed down an Isolute^{®} SCX-2 cartridge eluting with MeOH and then 2M methanolic ammonia. The solution was concentrated in *vacuo* to give the desired product (29 mg).

LCMS (Method 1): Rt = 0.73 min, m/z 362.0/364.0 [M+H]⁺

### Intermediate E2

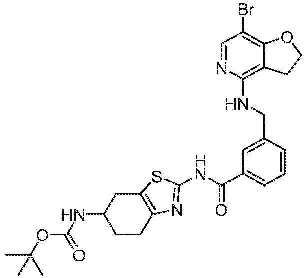

### tert-Butyl (2-(3-(((7-bromo-2,3-dihydrofuro[3,2-c]pyridin-4-yl)amino)-methyl)benzamido)-4,5,6,7-tetrahydrobenzo[d]thiazol-6-yl)carbamate

### (Intermediate E2)

Diisopropylethylamine (1.0 mL, 6.0 mmol) was added to a stirred solution of 3-(((7-bromo-2,3-dihydrofuro[3,2-c]pyridin-4-yl)amino)methyl)benzoic acid (700 mg, 2.0 mmol), tert-butyl (2-amino-4,5,6,7-tetrahydrobenzo[d]thiazol-6-yl)carbamate (540 mg, 2.0 mmol) and TBTU (837 mg, 2.61 mmol) in DMF (12 mL). The mixture was stirred at RT for 6 h then allowed to stand for 16 h. The mixture was diluted with EtOAc (25 mL) and the solution was washed with water (25 mL). The aqueous phase was extracted with EtOAc (25 mL). The combined organic phase was washed with aqueous saturated NaCl (2 x 25 mL), dried (Na₂SO₄) and concentrated under reduced pressure. The residue was taken into DCM and loaded onto an Isolute^{®} SCX-2 column, washed with DCM then 2:1 DCM:MeOH, after eluted with 2:1 DCM:2M methanolic ammonia and concentrated under reduced pressure. The residue was taken into DCM and purified by flash chromatography on a Si column eluting with 0-50% EtOAc in DCM then with 100% EtOAc to afford the desired product as a fawn foam (775 mg).

LCMS (Method 2): Rt =1.46 min, m/z 600.0/601.9 [M+H]⁺

### Intermediate E3

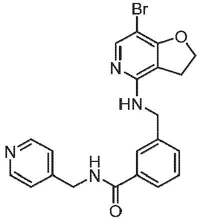

### 3-(((7-Bromo-2,3-dihydrofuro[3,2-c]pyridin-4-yl)amino)methyl)-N-(pyridin-4-ylmethyl)benzamide (Intermediate E3)

Intermediate C1 (250 mg, 0.688 mmol), pyridin-4-ylmethanamine (0.210 mL, 2.065 mmol) and bis(trimethylaluminum) 1,4 diazabicyclo[2.2.2]octane adduct (529 mg, 2.065 mmol) were dissolved in THF (6 mL) and the mixture purged with nitrogen. The resulting mixture was heated under microwave irradiation at 120°C for 30 min. The reaction mixture was quenched with water, extracted with DCM and the organic phase evaporated under vacuum. The resulting crude was purified by flash chromatography on C18-silica by eluting with 0-20% B in A (A: water/acetonitrile 95/5 + 0.1% HCOOH, B: acetonitrile/water 95/5 + 0.1% HCOOH). The appropriate fractions were pooled and evaporated to afford the title compound (164 mg).

LCMS (Method 6): Rt =0.39 min, m/z 439.1/441.1 [M+H]⁺

### Intermediate E4

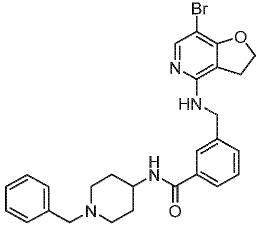

### N-(1-Benzylpiperidin-4-yl)-3-(((7-bromo-2,3-dihydrofuro[3,2-c]pyridin-4-yl)amino)methyl)benzamide (Intermediate E4)

Intermediate E4 was prepared using a similar procedure to that previously described for intermediate E3 by replacing pyridin-4-ylmethanamine with 1-benzylpiperidin-4-amine.

LCMS (Method 6): Rt =0.51 min, m/z 521.2/523.1 [M+H]⁺

### Intermediate E5

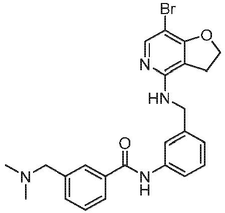

### N-(3-(((7-Bromo-2,3-dihydrofuro[3,2-c]pyridin-4-yl)amino)methyl)phenyl)-3-((dimethylamino)methyl)benzamide (Intermediate E5)

3-((Dimethylamino)methyl)benzoic acid hydrochloride (98 mg, 0.454 mmol) and COMU (0.133 mL, 0.273 mmol) were dissolved in anhydrous DCM (5 mL) and stirred for 10 min, then intermediate D2 (81mg, 0.23 mmol) and DIEA (0.158 mL, 0.908 mmol) were added and the mixture stirred for 2 h at RT. The reaction mixture was diluted with DCM and the organic phase washed twice with saturated aqueous NaHCO₃ and evaporated under vacuum. The crude product was purified by flash chromatography on silica by eluting with 0-30% of 80/20 DCM/MeOH (+ 1% triethylamine) in DCM. Appropriate fractions were combined and evaporated under vacuum to give the desired product (115 mg).

LCMS (Method 6): Rt =0.42 min, m/z 481.0/483.0 [M+H]⁺

### Intermediate E6

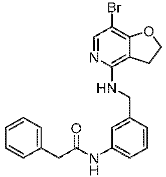

### N-(3-(((7-Bromo-2,3-dihydrofuro[3,2-c]pyridin-4-yl)amino)methyl)phenyl)-2-phenylacetamide (Intermediate E6)

2-Phenylacetic acid (112 mg, 0.82 mmol) and COMU (0.240 mg, 0.49 mmol) were dissolved in DCM (4 mL) and reacted for 10 min at RT, then intermediate D2 (150 mg, 0.41mmol) and DIEA (0.143 mL, 0.819 mmol) were added. The reaction was stirred for 2 h at RT. The reaction mixture diluted with DCM, washed twice with saturated aqueous NaHCO₃ and evaporated. The resulting crude was purified by flash chromatography on C18-silica by eluting with 0-10% B in A (A: water/acetonitrile 95/5 + 0.1% HCOOH, B: acetonitrile/water 95/5 + 0.1% HCOOH) to give the desired product (126 mg).

LCMS (Method 6): Rt =0.81 min, m/z 438.0/440.0 [M+H]⁺

### Intermediate F

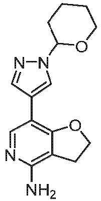

### 7-(1-(Tetrahydro-2H-pyran-2-yl)-1H-pyrazol-4-yl)-2,3-dihydrofuro[3,2-clpyridin-4-amine (Intermediate F)

### Intermediate B (1.80 g, 8.4 mmol), 1-(tetrahydro-2H-pyran-2-yl)-1H-pyrazole-4-boronic acid (3.03 g, 11 mmol), and cesium carbonate (8.18 g, 25 mmol) were mixed in DMF (72 mL) and water (36 mL). The reaction was purged with argon for 10 min, then tetrakis(triphenylphosphine)palladium(0) (0.97 g, 0.8 mmol) was added and the reaction mixture heated at 70°C overnight. The reaction was allowed to cool to RT, diluted with water (72 mL) and extracted with EtOAc (5 x 250 mL). The combined organic layers were washed with saturated aqueous NaCl (505 mL) and concentrated to dryness. The crude was purified on silica by eluting with 0-8% MeOH in DCM to afford the title compound (1.13 g).

LCMS (Method 6): Rt =0.41 min, m/z 287.2 [M+H]⁺

### Intermediate G

### Step A

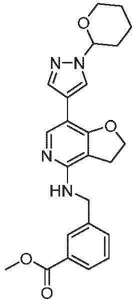

### Methyl 3-(((7-(1-(tetrahydro-2H-pyran-2-yl)-1H-pyrazol-4-yl)-2,3-dihydrofuro[3,2-c]pyridin-4-yl)amino)methyl)benzoate (Intermediate G-a)

Intermediate C1 (0.9 g, 2,478 mmol), 1-(tetrahydro-2H-pyran-2-yl)-4-(4,4,5,5-tetramethyl-1,3,2-dioxaborolan-2-yl)-1H-pyrazole (1.378 g, 4.96 mmol) and potassium phosphate tribasic (1.578 g, 7.43 mmol) were dissolved in a mixture of THF (10 mL) and water (10 mL). The mixture was purged with argon for 10 min, then chloro(2-dicyclohexylphosphino-2',4',6'-triisopropyl-1,1'-biphenyl)[2-(2'-amino-1,1'-iphenyl)]palladium(II) (0.292 g, 0.372 mmol) was added and the reaction stirred at RT for 6 h. The mixture was diluted with water (30 mL) and extracted with THF (3 x 25 mL). The organic phase was dried over Na₂SO₄ and concentrated to dryness. The crude was purified by flash chromatography on silica by eluting with 0-10% MeOH in DCM to afford the desired product (1.08 g).

LCMS (Method 6): Rt =0.69 min, m/z 435.3 [M+H]⁺

### Step B

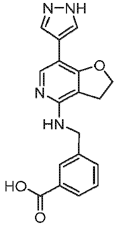

### 3-(((7-(1H-Pyrazol-4-yl)-2,3-dihydrofuro[3,2-c]pyridin-4-yl)amino)methyl)benzoic acid (Intermediate G)

Intermediate G-a (1.26 g, 2.90 mmol) was suspended in aqueous 6M HCl (35 mL, 0.435 mmol). The mixture was heated at 90 °C for 30 min and then evaporated to dryness to afford the title compound (1.26 g) that was used in the next steps without further purifications.

LCMS (Method 6): Rt =0.46 min, m/z 337.2 [M+H]⁺

### Intermediate H1

### Step A

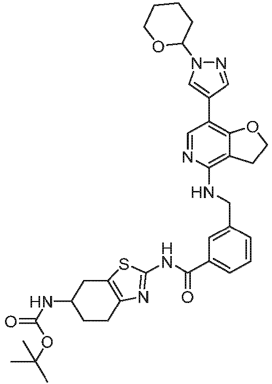

### tert-Butyl (2-(3-(((7-(1-(tetrahydro-2H-pyran-2-yl)-1H-pyrazol-4-yl)-2,3-di-hydrofuro[3,2-c]pyridin-4-yl)amino)methyl)benzamido)-4,5,6,7-tetrahydrobenzo[d]-thiazol-6-yl)carbamate (Intermediate H1-a)

Chloro(2-dicyclohexylphosphino-2',4',6'-triisopropyl-1,1'-biphenyl)[2-(2'-amino-1,1'-diphenyl)]palladium(II) (59 mg, 0.075 mmol) was added to a mixture of intermediate E2 (300 mg 0.5 mmol), potassium phosphate tribasic (318 mg, 1.5 mmol), 1-(tetrahydropyran-2-yl)-1H-pyrazole-4-boronic acid pinacol ester (278 mg, 1.0 mmol) in THF (2.0 mL) and water (2 mL) which had been degassed with argon. The mixture was heated at 80°C for 1 h in a microwave reactor. The cold mixture was diluted with EtOAc (15 mL) and washed with water (10 mL). The aqueous phase was extracted with EtOAc (2 x 10 mL). The combined organic phase was dried (Na₂SO₄) and concentrated *in vacuo.* The residue was purified by flash chromatography on silica by eluting sequentially with DCM, 20% EtOAc in DCM, 50% EtOAc in DCM and EtOAc to afford the desired product (307 mg).

LCMS (Method 2): Rt =1.26 min, m/z 672.3 [M+H]⁺

### Step B

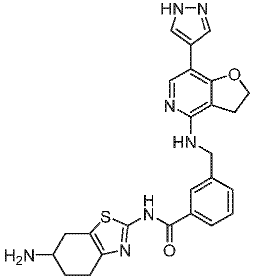

### 3-(((7-(1H-Pyrazol-4-yl)-2,3-dihydrofuro[3,2-c]pyridin-4-yl)amino)methyl)-N-(6-amino-4,5,6,7-tetrahydrobenzo[d]thiazol-2-yl)benzamide (Intermediate H1)

A solution of intermediate H1-a (305 mg, 0.45 mmol), in 1.25M hydrogen chloride solution in MeOH (10 mL), was stirred for 1.25 h, then treated with 12M aqueous hydrochloric acid (2.0 mL). The resulting mixture was stirred for 2.5 h then a further aliquot of 12M aqueous hydrochloric acid (1.0 mL) was added. The mixture was stirred for a further 3 h then concentrated *in vacuo.* The residue was taken into MeOH (30 mL) and the solution was filtered through an Isolute^{®} SCX-2 cartridge. The cartridge was washed with MeOH then eluted with 20% 2M methanolic ammonia in DCM. Concentration of the basic fractions afforded the desired product (150 mg).

LCMS (Method 2): Rt = 0.63 min, m/z 488.0 [M+H]⁺

### Intermediate I1 to I3

The following intermediates were prepared using a procedure similar to that used for the synthesis of intermediate C1 by replacing respectively intermediate B with intermediate F and methyl 3-formylbenzoate with starting material given into the table.

| | **Structure** | **Starting material** | **LC-MS** |
|---|---|---|---|
| I1 | 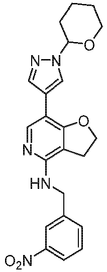 | 3-nitrobenzaldehyde | Rt = 0.44 min, m/z 422.2 [M+H]⁺ (Method 6) |
| I2 | 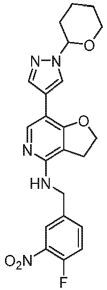 | 4-fluoro-3-nitrobenzaldehyde | Rt = 0.63 min, m/z 439.7 [M+H]⁺ (Method 6) |
| I3 | 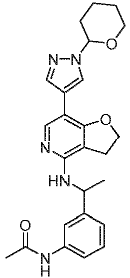 | N-(3-acetylphenyl)acetamide | Rt = 0.56 min, m/z 448.3 [M+H]⁺ (Method 6) |

### Intermediate J1

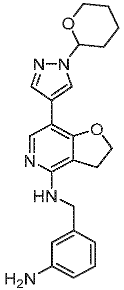

### N-(3-Aminobenzyl)-7-(1-(tetrahydro-2H-pyran-2-yl)-1H-pyrazol-4-yl)-2,3-dihydrofuro[3,2-c]pyridin-4-amine (Intermediate J1)

Intermediate I1 (330 mg, 0.783 mmol) was dissolved in ethanol (7.5 mL) then Pd/C 5% wet (667 mg, 0.313 mmol) was added and the reaction stirred under a blanket of hydrogen for 1 h. The reaction mixture was filtered and the solvent evaporated under vacuum to give the title compound (178 mg) which was used in next steps without further purifications.

LCMS (Method 6): Rt = 0.44 min, m/z 392.0 [M+H]⁺

### Intermediate J2

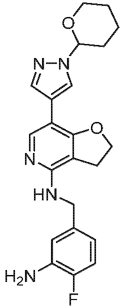

### N-(3-Amino-4-fluorobenzyl)-7-(1-(tetrahydro-2H-pyran-2-yl)-1H-pyrazol-4-yl)-2,3-dihydrofuro[3,2-c]pyridin-4-amine (Intermediate J2)

Intermediate J2 was prepared from intermediate I2 using a procedure similar to that used for the synthesis of intermediate J1 from intermediate I1.

LCMS (Method 6): Rt = 0.55 min, m/z 410.0 [M+H]⁺

### Intermediate K1

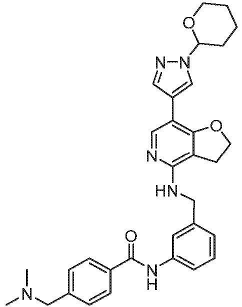

### 4-((Dimethylamino)methyl)-N-(3-(((7-(1-(tetrahydro-2H-pyran-2-yl)-1H-pyrazol-4-yl)-2,3-dihydrofuro[3,2-c]pyridin-4-yl)amino)methyl)phenyl)benzamide

### (Intermediate K1)

4-((Dimethylamino)methyl)benzoic acid hydrochloride (55.1 mg, 0.26 mmol), DIEA (0.140 mL, 0.51 mmol) and HATU (116 mg, 0.31 mmol) were dissolved in anhydrous DCM (4 mL) and reacted for 10 min, then intermediate J1 (50 mg, 0.128 mmol) was added and the reaction mixture stirred for 1 h at RT. The reaction mixture was diluted with DCM and washed with water (2 x 2.5 mL). The organic layer was dried under vacuum and the residue purified by flash chromatography on NH-silica by eluting with 0-10% MeOH in DCM. Appropriate fractions were combined and evaporated under vacuum to give the desired compound (16 mg).

LCMS (Method 6): Rt = 0.41 min, m/z 553.0 [M+H]⁺

### Intermediate K2 to K14

The following intermediates were prepared using a procedure similar to that used for the synthesis of intermediate K1 by replacing intermediate J1 and 4-((dimethylamino)methyl)benzoic acid hydrochloride with the starting materials given the table below.

| | **Structure** | **Starting material** | **LC-MS** |
|---|---|---|---|
| K2 | 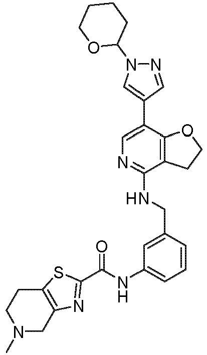 | Intermediate J1 and 5-methyl-4,5,6,7-tetrahydrothiazolo [4,5-c]pyridine-2-carboxylic acid | Rt = 0.76 min, m/z 572.3 [M+H]⁺ (Method 15) |
| K3 | 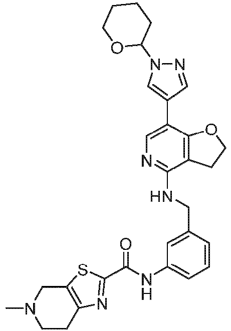 | Intermediate J1 and 5-methyl-4,5,6,7-tetrahydrothiazolo [5,4-c]pyridine-2-carboxylic acid | Rt = 0.72 min, m/z 572.3 [M+H]⁺ (Method 15) |
| K4 | 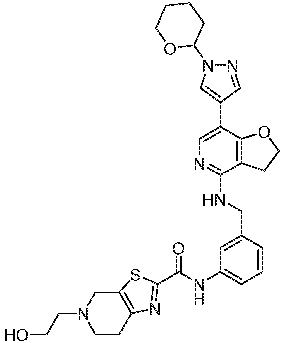 | Intermediate J1 and Intermediate A4 | Rt = 0.67 min, m/z 602.1 [M+H]⁺ (Method 15) |
| K5 | 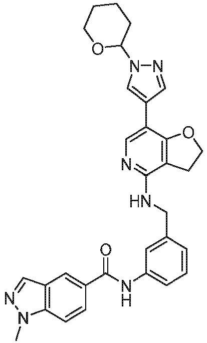 | Intermediate J1 and 1-methyl-1H-indazole-5-carboxylic acid | Rt = 1.09 min, m/z 550.1 [M+H]⁺ (Method 15) |
| K6 | 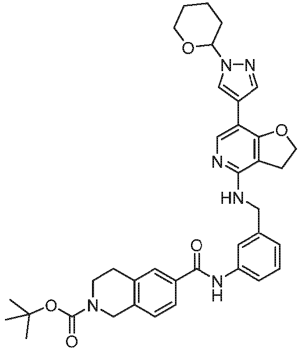 | Intermediate J1 and 2-(tert-butoxycarbonyl)-1,2,3,4-tetrahydroisoquin oline-6-carboxylic acid | Rt = 0.87 min, m/z 651.8 [M+H]⁺ (Method 6) |
| K7 | 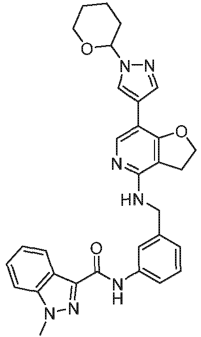 | Intermediate J1 and 1-methyl-1H-indazole-3-carboxylic acid | Rt = 0.74 min, m/z 550.1 [M+H]⁺ (Method 6) |
| K8 | 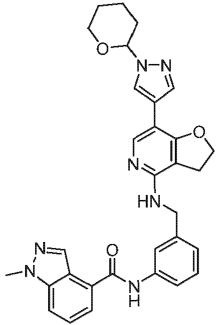 | Intermediate J1 and 1-methyl-1H-indazole-4-carboxylic acid | Rt = 1.09 min, m/z 550.1 [M+H]⁺ (Method 6) |
| K9 | 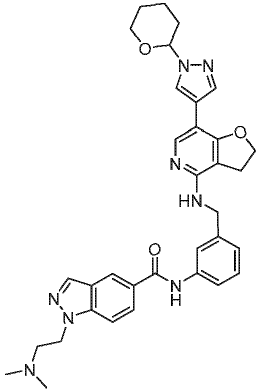 | Intermediate J1 and Intermediate A5 | Rt = 0.43 min, m/z 606.8 [M+H]⁺ (Method 6) |
| K10 | 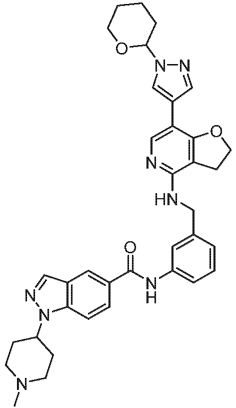 | Intermediate J1 and Intermediate A6 | Rt = 0.44 min, m/z 633.2 [M+H]⁺ (Method 6) |
| K11 | 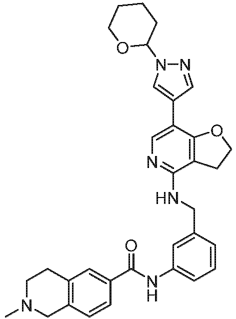 | Intermediate J1 and 2-methyl-1,2,3,4-tetrahydroisoquin oline-6-carboxylic acid | Rt = 0.45 min, m/z 564.9 [M+H]⁺ (Method 6) |
| K12 | 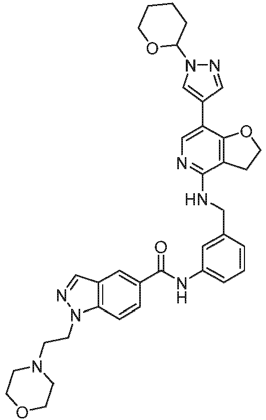 | Intermediate J1 and Intermediate A7 | Rt = 0.52 min, m/z 648.9 [M+H]⁺ (Method 14) |
| K13 | 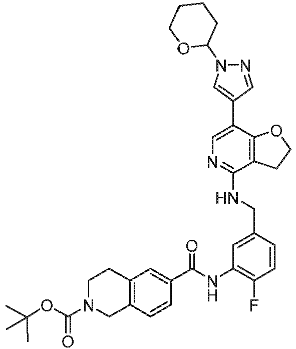 | Intermediate J2 and 2-(tert-butoxycarbonyl)-1,2,3,4-tetrahydroisoquin oline-6-carboxylic acid | Rt = 0.52 min, m/z 668.9[M+H]⁺ (Method 6) |
| K14 | 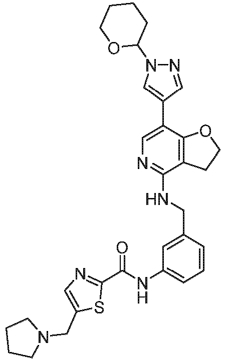 | Intermediate J1 and Intermediate A8 | Rt = 0.46 min, m/z 585.9 [M+H]⁺ (Method 6) |

### Intermediate K15

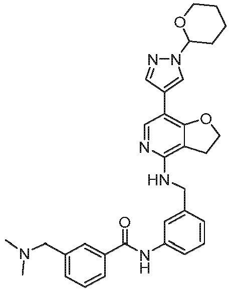

### 3-((Dimethylamino)methyl)-N-(3-(((7-(1-(tetrahydro-2H-pyran-2-yl)-1H-pyrazol-4-yl)-2,3-dihydrofuro[3,2-c]pyridin-4-yl)amino)methyl)phenyl)benzamide

### (Intermediate K15)

1-(Tetrahydro-2H-pyran-2-yl)-4-(4,4,5,5-tetramethyl-1,3,2-dioxaborolan-2-yl)-1H-pyrazole (48.8 mg, 0.176 mmol), intermediate E6 (65 mg, 0.135 mmol) and cesium carbonate (132 mg, 0.405 mmol) were mixed into DMF (2 mL) and water (1 mL) and purged with argon for 10 min. Tetrakis(triphenylphosphine)palladium(0) (0.018 mL, 0.014 mmol) was added and the reaction heated to 70°C for 2 h. The reaction mixture was concentrated and the crude purified by flash chromatography on C18-silica by eluting with 0-30% B in A (A: water/acetonitrile 95:5 + 0.1% HCOOH, B: acetonitrile/water 95:5 + 0.1% HCOOH). Appropriate fractions were pooled and evaporated to dryness to give the title compound (37.5 mg).

LCMS (Method 6): Rt = 0.41 min, m/z 553.3 [M+H]⁺

### Intermediate K16

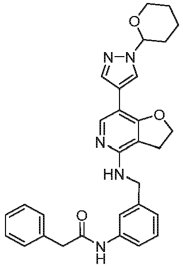

### 2-Phenyl-N-(3-(((7-(1-(tetrahydro-2H-pyran-2-yl)-1H-pyrazol-4-yl)-2,3-dihydrofuro[3,2-c]pyridin-4-yl)amino)methyl)phenyl)acetamide (Intermediate K16)

Intermediate K16 was prepared from intermediate E7 using a procedure similar to that used for the synthesis of intermediate K15 from intermediate E6.

LCMS (Method 11): Rt = 1.2 min, m/z 510.1 [M+H]⁺

### Intermediate K17

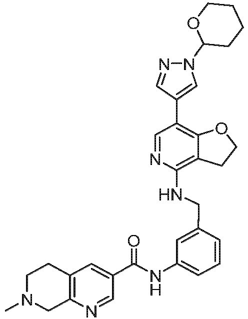

### 7-Methyl-N-(3-(((7-(1-(tetrahydro-2H-pyran-2-yl)-1H-pyrazol-4-yl)-2,3-dihydrofuro[3,2-c]pyridin-4-yl)amino)methyl)phenyl)-5,6,7,8-tetrahydro-1,7-naphthyridine-3-carboxamide (Intermediate K17)

Intermediate J1 (102 mg, 0.261 mmol), bis(trimethylaluminum)-1,4-diazabicyclo[2.2.2]octane adduct (134 mg, 0.523 mmol), ethyl 7-methyl-5,6,7,8-tetrahydro-1,7-naphthyridine-3-carboxylate (115 mg, 0.523 mmol) and THF (3 mL) were charged in a closed vessel and heated at 110°C for 1 h under microwave irradiation. The reaction was quenched by the addition of water and then diluted with DCM. The organic phase was separated and evaporated under vacuum. The crude material was purified by flash chromatography on C18-silica by eluting with 0-60% B in A (A: water/acetonitrile 95:5 + 0.1% HCOOH, B: acetonitrile/water 95:5 + 0.1% HCOOH) to afford the title compound (11 mg).

LCMS (Method 6): Rt = 0.39 min, m/z 565.9 [M+H]⁺

### PREPARATION OF EXAMPLES

### Example 1

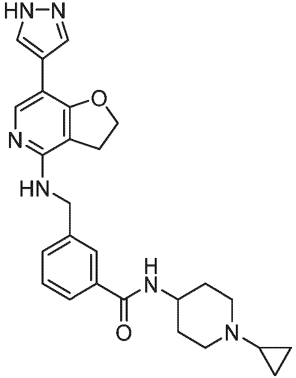

### 3-(((7-(1H-Pyrazol-4-yl)-2,3-dihydrofuro[3,2-c]pyridin-4-yl)amino)methyl)-N-(1-cyclopropylpiperidin-4-yl)benzamide (Example 1)

To a mixture of intermediate G (50 mg, 0.14 mmol), 1-cyclopropylpiperin-4-amine (21 mg, 0.149 mmol), DIPEA (0.078 mL, 0.446 mmol) and TBTU (62 mg, 0.193 mmol) in DMF (1 mL) was stirred at RT for 3 h. The reaction mixture was diluted with MeOH and applied to a Isolute^{®} SCX-2 cartridge eluting with MeOH and then 2M methanolic ammonia. The relevant fractions were concentrated in *vacuo* and the crude material was purified by chromatography on silica by eluting with 0-10% 2M methanolic ammonia in DCM to give the title compound (16 mg).

LCMS (Method 5) Rt = 1.75 min, m/z 459.4 [M+H]⁺

¹H NMR (400 MHz, DMSO-d6) δ 12.8 (s, 1H), 8.18 (d, J = 7.7Hz, 1H), 8.07 (s, 1H), 7.86 (s, 2H), 7.79 (s, 1H), 7.65 (d, 7.8Hz, 1H), 7.46 (d, J = 7.8Hz, 1H), 7.35 (t, J = 7.8Hz, 1H), 6.71 (t, J = 6.7Hz, 1H), 4.68 (t, J = 8.8Hz, 2H), 4.60 (d, J = 6.2Hz, 2H), 3.81-3.68 (m, 1H), 3.04 (t, J = 8.8Hz, 2H), 2.96-2.88 (m, 2H), 2.26-2.16 (m, 2H), 1.78-1.69 (m, 2H), 1.62-1.55 (m, 1H), 1.53-1.41 (m, 2H), 0.43-0.37 (m, 2H), 0.30-0.25 (m, 2H).

### Examples 2 to 31

The following examples were prepared from Intermediate G and the amine given in a manner 'analogous' or 'similar' to that used for example 1. Such procedures may involve minor variations, for example reaction temperature, reagent/solvent amount, reaction time, work-up conditions or chromatographic purification conditions (eg. HPLC-MDAP or flash chromatography). In some cases, where modification involved reaction solvent (eg. DCM-DMF mix instead of DMF) or coupling agents (e.g. HATU instead of TBTU), such changes were reported as a note.

| **Ex** | **Structure** | **Amine** | **¹H NMR** | **LC-MS** |
|---|---|---|---|---|
| 2 | 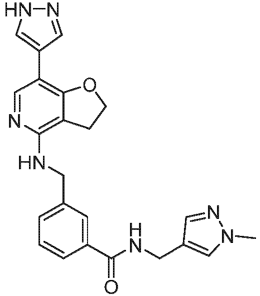 3-(((7-(1H-pyrazol-4-yl)-2,3-dihydrofuro[3,2-c]pyridin-4-yl)amino)methyl)-N-((1-methyl-1H-pyrazol-3-yl)methyl)benzamide | (1-Methyl-1H-pyrazol-4-yl)methanami ne | (400 MHz, DMSO-d6) δ 12.58 (s, 1H), 8.50-8.39 (m, 1H), 8.08 (s, 1H), 7.88 (s, 1H), 7.83 (s, 2H), 7.71 (d, J=7.7 Hz, 1H), 7.53-7.50 (m, 2H), 7.39-7.35 (m, 1H), 6.32 (t, J=6.0 Hz, 1H), 6.15 (d, J=2.1 Hz, 1H), 4.70 (t, J=8.8 Hz, 2H), 4.66 (d, J=6.2 Hz, 2H), 4.44 (d, J=5.8 Hz, 2H), 3.08 (t, J=8.9 Hz, 2H), 3.04 (s, 3H). | Rt = 3.32 min, m/z 430.0 [M+H]⁺ (Method 4) |
| 3 | 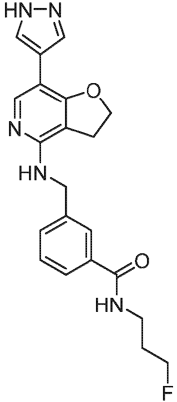 3-(((7-(1H-pyrazol-4-yl)-2,3-dihydrofuro[3,2-c]pyridin-4-yl)amino)methyl)-N-(3-fluoropropyl)benzamide | 3-Fluoropropan -1-amine | (400 MHz, DMSO-d6) δ 12.58 (s, 1H), 8.24-8.16 (m, 1H), 8.08 (s, 1H), 7.91-7.77 (m, 3H), 7.68 (d, J=7.7 Hz, 1H), 7.51 (d, J=7.3 Hz, 1H), 7.40-7.35 (m, 1H), 6.32 (t, J=6.0 Hz, 1H), 4.70 (t, J=8.9 Hz, 2H), 4.66 (d, J=6.4 Hz, 2H), 4.59 (t, J=5.9 Hz, 1H), 4.47 (t, J=6.0 Hz, 1H), 3.42-3.37 (m, 2H), 3.08 (t, J=8.7 Hz, 2H), 1.96 (d, J=51.3 Hz, 2H). | Rt = 2.35 min, m/z 396.0 [M+H]⁺ (Method 3) |
| 4 | 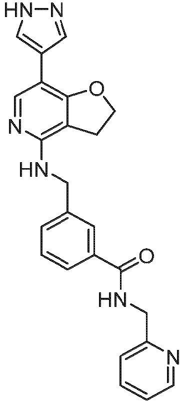 3-(((7-(1H-pyrazol-4-yl)-2,3-dihydrofuro[3,2-c]pyridin-4-yl)amino)methyl)-N-(pyridin-2-ylmethyl)benzamide | pyridin-2-ylmethanami ne | (400 MHz, DMSO-d6) δ 12.74 (br s, 1H), 9.02 (br t, J=5.8 Hz, 1H), 8.47 (br d, J=4.6 Hz, 1H), 8.04 (s, 1H), 7.89 - 7.87 (m, 1H), 7.86 (s, 1H), 7.77 -7.74 (m, 1H), 7.71 (br s, 2H), 7.47 (br d, J=7.7 Hz, 1H), 7.39 - 7.34 (m, 1H), 7.27 (d, J=7.7 Hz, 1H), 7.24 - 7.20 (m, 1H), 6.66 (br t, J=6.0 Hz, 1H), 4.65 (t,J=8.9 Hz, 2H), 4.59 (br d, J=6.1 Hz, 2H), 4.52 (d, J=5.9 Hz, 2H), 4.46 - 4.43 (m, 1H), 3.02 (br t, J=8.9 Hz, 2H). | Rt = 0.44 min, m/z 427.2 [M+H]⁺ (Method 6) |
| 5 | 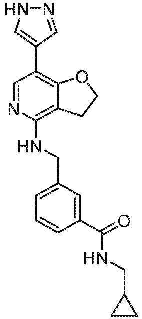 3-(((7-(1H-pyrazol-4-yl)-2,3-dihydrofuro[3,2-c]pyridin-4-yl)amino)methyl)-N-(cyclopropylmethyl)benza mide | Cyclopropyl methanamine | (400 MHz, DMSO-d6) δ 12.58 (s, 1H), 8.30 (t, J=5.6 Hz, 1H), 7.87 (s, 1H), 7.65 (s, 2H), 7.63-7.60 (m, 1H), 7.49-7.44 (m, 1H), 7.27-7.24 (m, 1H), 7.18-7.13 (m, 1H), 6.48 (t, J=6.1 Hz, 1H), 4.48 (t, J=9.0 Hz, 2H), 4.40 (d, J=5.9 Hz, 2H), 3.16-3.10 (m, 2H), 2.91 (t, J=6.2 Hz, 2H), 2.84 (t, J=8.9 Hz, 2H), 0.85-0.77 (m, 1H), 0.23-0.18 (m, 2H). | Rt = 3.75 min, m/z 390.0 [M+H]⁺ (Method 4) |
| 6 | 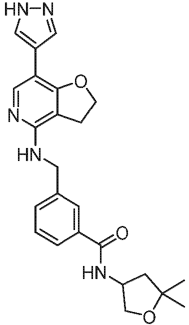 3-(((7-(1H-pyrazol-4-yl)-2,3-dihydrofuro[3,2-c]pyridin-4-yl)amino)methyl)-N-(5,5-dimethyltetrahydrofuran-3-yl)benzamide | 5,5-Dimethyltetra hydrofuran-3-amine | (400 MHz, DMSO-d6) δ 12.81 - 12.80 (m, 1H), 8.47 (d, J=6.5 Hz, 1H), 8.08 (s, 1H), 7.84 - 7.81 (m, 3H), 7.71 - 7.67 (m, 1H), 7.50 - 7.47 (m, 1H), 7.38 (t, J=7.7 Hz, 1H), 6.71 (t, J=6.1 Hz, 1H), 4.70 (t, J=8.9 Hz, 2H), 4.62 (d, J=6.0 Hz, 2H), 4.54 (d, J=8.0 Hz, 1H), 4.00 (dd, J=7.0, 8.8 Hz, 1H), 3.61 (dd, J=6.4, 8.8 Hz, 1H), 3.10 - 3.03 (m, 2H), 2.09 (dd, J=8.3, 12.5 Hz, 1H), 1.82 (dd, J=7.3, 12.5 Hz, 1H), 1.29 (s, 3H), 1.19 (s, 3H). | Rt = 2.46 min, m/z 434.2 [M+H]⁺ (Method 3) |
| 7 | 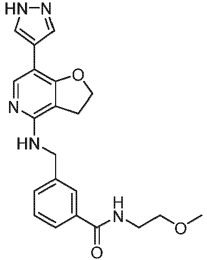 3-(((7-(1H-pyrazol-4-yl)-2,3-dihydrofuro[3,2-c]pyridin-4-yl)amino)methyl)-N-(2-methoxyethyl)benzamide | 2-Methoxyetha n-1-amine | (400 MHz, DMSO-d6) δ 12.6 ( bs , 1H), 8.48 (m, 1H), 8.17 (s, 1H), 8.09 (s, 1H), 7.87 (s, 2H), 7.84 (s, 1H), 7.70-7.67 (m, 1H), 7.50-7.47 (m, 1H), 7.38 (t, J= 7.7 Hz, 1H), 6.73-6.68 (m, 1H), 4.70 (t, J=8.9 Hz, 2H), 4.62 (d, J=6.0 Hz, 2H), 3.47 - 3.41 (m, 4H), 3.28 (s, 3H), 3.07 (t, J=7.6 Hz, 2H). | Rt = 2.17 min, m/z 394.0 [M+H]⁺ (Method 3) |
| 8 | 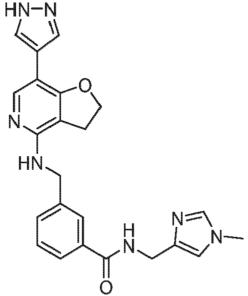 3-(((7-(1H-pyrazol-4-yl)-2,3-dihydrofuro[3,2-c]pyridin-4-yl)amino)methyl)-N-((1-methyl-1H-imidazol-4-yl)methyl)benzamide | (1-Methyl-1H-imidazol-4-yl)methanami ne | (400 MHz, DMSO-d6) δ12.80 (d, J=0.9 Hz, 1H), 8.74 (t, J=5.6 Hz, 1H), 8.09 (s, 1H), 7.86 (s, 3H), 7.71 (d, J=7.8 Hz, 1H), 7.47 (d, J=5.9 Hz, 2H), 7.37 (t, J=7.7 Hz, 1H), 6.93 (s, 1H), 6.70 (dd, J= 6.1 Hz, 1H), 4.70 (t, J=8.9 Hz, 2H), 4.62 (d, J=6.0 Hz, 2H), 4.32 (d, J=5.5 Hz, 2H), 3.60 (s, 3H), 3.06 (t, J=8.9 Hz, 2H). | Rt = 1.64 min, m/z 430.0 [M+H]⁺ (Method 3) |
| 9 | 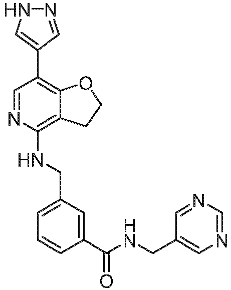 3-(((7-(1H-pyrazol-4-yl)-2,3-dihydrofuro[3,2-c]pyridin-4-yl)amino)methyl)-N-(pyrimidin-5-ylmethyl)benzamide | Pyrimidin-5-ylmethanami ne | (400 MHz, DMSO-d6) δ 12.80 (s, 1H), 9.12 - 9.09 (m, 2H), 8.78 (s, 2H), 8.09 (s, 1H), 7.88 - 7.85 (m, 3H), 7.72 (ddd, J=1.4, 1.4, 7.7 Hz, 1H), 7.53 - 7.49 (m, 1H), 7.40 (t, J=7.7 Hz, 1H), 6.71 (t, J=6.1 Hz, 1H), 4.70 (t, J=8.9 Hz, 2H), 4.62 (d, J=6.0 Hz, 2H), 4.50 (d, J=5.8 Hz, 2H), 3.06 (t, J=8.8 Hz, 2H). | Rt = 2.06 min, m/z 428.0 [M+H]⁺ (Method 3) |
| 10 | 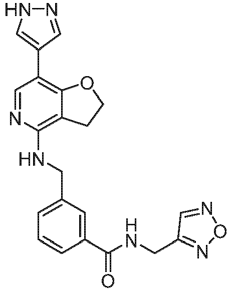 N-((1,2,5-oxadiazol-3-yl)methyl)-3-(((7-(1H-pyrazol-4-yl)-2,3-dihydrofuro[3,2-c]pyridin-4-yl)amino)methyl)benzamid e | (1,2,5-Oxadiazol-3-yl)methanami ne | (400 MHz, DMSO-d6) δ 13.28 (s, 1H), 12.80-12.75 (m, 1H), 9.06 (t, J=5.8 Hz, 1H), 8.09 (s, 1H), 7.87 (s, 3H), 7.74-7.70 (m, 1H), 7.55-7.51 (m, 1H), 7.42 (t, J=7.7 Hz, 1H), 6.73 (t, J=6.0 Hz, 1H), 4.74-4.61 (m, 4H), 4.28 (d, J=5.8 Hz, 2H), 3.10-3.03 (m, 2H). | Rt = 2.3 min, m/z 418.2 [M+H]⁺ (Method 3) |
| 11 | 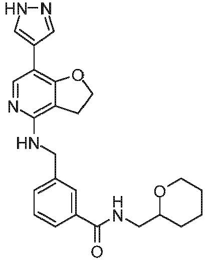 3-(((7-(1H-pyrazol-4-yl)-2,3-dihydrofuro[3,2-c]pyridin-4-yl)amino)methyl)-N-((tetrahydro-2H-pyran-2-yl)methyl)benzamide | (Tetrahydro-2H-pyran-2-yl)methanami ne | (400 MHz, DMSO-d6) δ 12.80 (s, 1H), 8.44 (t, J=5.8 Hz, 1H), 8.09 (s, 1H), 7.85-7.83 (m, 2H), 7.82-7.67 (m, 2H), 7.50-7.46 (m, 1H), 7.37 (t, J=7.7 Hz, 1H), 6.71 (t, J=6.1 Hz, 1H), 4.70 (t, J=8.9 Hz, 2H), 4.62 (d, J=6.0 Hz, 2H), 3.87 (dd, J=2.3, 10.8 Hz, 1H), 3.43 (m, 2H), 3.26 (m, 2H), 3.06 (t, J=8.8 Hz, 2H), 1.80-1.75 (m, 1H), 1.61 (d, J=12.5 Hz, 1H), 1.49-1.41 (m, 3H), 1.22-1.12 (m, 1H). | Rt = 2.61 min, m/z 434.2 [M+H]⁺ (Method 3) |
| 12 | 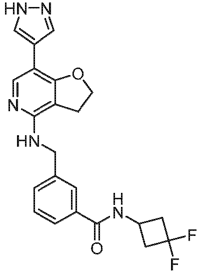 3-(((7-(1H-pyrazol-4-yl)-2,3-dihydrofuro[3,2-c]pyridin-4-yl)amino)methyl)-N-(3,3-difluorocyclobutyl)benzami de | 3,3-Difluorocyclo butan-1-amine | (400 MHz, DMSO-d6) δ 12.80-12.78 (m, 1H), 8.81 (d, J=6.5 Hz, 1H), 8.09-8.08 (m, 2H), 7.86-7.82 (m, 2H), 7.70 (d, J=7.7 Hz, 1H), 7.53-7.49 (m, 1H), 7.41 (t, J=7.7 Hz, 1H), 6.72 (t, J=6.1 Hz, 1H), 4.74-4.61 (m, 4H), 4.32-4.24 (m, 1H), 3.10 (m, 2H), 2.83-2.68 (m, 2H), 2.68 (m, 2H). | Rt = 2.68 min, m/z 426.2 [M+H]⁺ (Method 3) |
| 13 | 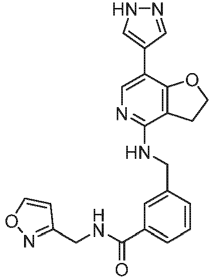 3-(((7-(1H-pyrazol-4-yl)-2,3-dihydrofuro[3,2-c]pyridin-4-yl)amino)methyl)-N-(isoxazol-3-ylmethyl)benzamide | 1,2-Oxazol-3-ylmethanami ne | (400 MHz, DMSO-d6) δ 13.1 (bs, 1H), 9.07 (t, J = 5.6Hz, 1H), 8.34 (d, J = 1.6Hz, 1H), 8.16 (s, 1H), 8.09 (s, 1H), 7.87 (br s, 3H), 7.74-7.70 (m, 1H), 7.53-7.49 (m, 1H), 7.40 (t, J = 7.7Hz, 1H), 6.72 (t, J = 6.2Hz, 1H), 6.50 (d, J = 8.2Hz, 1H), 4.70 (t, J = 8.6Hz, 2H), 4.63 (d, J = 6.1Hz, 2H), 4.55 (d, J = 6.1Hz, 2H), 3.07 (t, J = 8.6Hz, 2H). | Rt = 2.28 min, m/z 417.0 [M+H]⁺ (Method 3) |
| 14 | 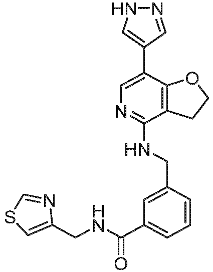 3-(((7-(1H-pyrazol-4-yl)-2,3-dihydrofuro[3,2-c]pyridin-4-yl)amino)methyl)-N-(thiazol-4-ylmethyl)benzamide | Thiazol-4-ylmethanami ne | (400 MHz, DMSO-d6) δ 12.8 (s, 1H), 9.07 (d, J = 2.0Hz, 1H), 9.04 (t, J = 5.5Hz, 1H), 8.09 (s, 1H), 7.90-7.83 (m, 3H), 7.77-7.73 (m, 1H), 7.52-7.48 (m, 1H), 7.45-7.43 (m, 1H), 7.40 (t, J = 7.6Hz, 1H), 6.71 (t, J = 5.9Hz, 1H), 4.70 (t, J = 8.9Hz, 2H), 4.62 (t, J = 5.7Hz, 4H), 3.06 (t, J = 8.9Hz, 2H). | Rt = 2.29 min, m/z 433.0 [M+H]⁺ (Method 3) |
| 15 | 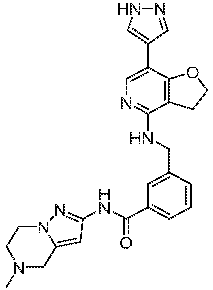 3-(((7-(1H-pyrazol-4-yl)-2,3-dihydrofuro[3,2-c]pyridin-4-yl)amino)methyl)-N-(5-methyl-4,5,6,7-tetrahydropyrazolo[1,5-a]pyrazin-2-yl)benzamide | 5-Methyl-4,5,6,7-tetrahydropyr azolo[1,5-a]pyrazin-2-amine | (400 MHz, DMSO-d6) δ 12.81 (s, 1H), 10.73 (s, 1H), 8.10 (s, 1H), 7.96 (s, 1H), 7.87 (s, 2H), 7.84 (d, J=8.1 Hz, 1H), 7.52 (d, J=7.8 Hz, 1H), 7.43 - 7.38 (m, 1H), 6.71 (t, J=6.1 Hz, 1H), 6.42 (s, 1H), 4.71 (t, J=8.8 Hz, 2H), 4.65 (d, J=5.9 Hz, 2H), 4.01 (t, J=5.5 Hz, 2H), 3.57 (s, 2H), 3.07 (t, J=8.8 Hz, 2H), 2.85 (t, J=5.6 Hz, 2H), 2.40 (s, 3H). | Rt = 2.17 min, m/z 471.6 [M+H]⁺ (Method 13) |
| 16 | 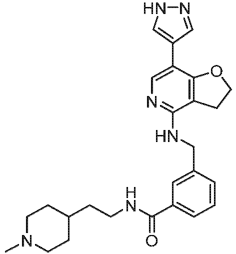 3-(((7-(1H-pyrazol-4-yl)-2,3-dihydrofuro[3,2-c]pyridin-4-yl)amino)methyl)-N-(2-(1-methylpiperidin-4-yl)ethyl)benzamide | 1-methylpiperid in-4-yl)ethanamin e | (400MHz, DMSO-d6) δ 12.75 (br s, 1H), 8.34 (t, J=5.7 Hz, 1H), 8.05 (s, 1H), 7.89 (br s, 1H), 7.78 (s, 2H), 7.63 (dt, J=7.7, 1.5 Hz, 1H), 7.44 (dt, J=7.7, 1.5 Hz, 1H), 7.34 (t, J=7.7 Hz, 1H), 6.65 (t, J=6.0 Hz, 1H), 4.67 (t, J=8.9 Hz, 2H), 4.59 (d, J=5.7 Hz, 2H), 3.27 - 3.24 (m, 2H), 3.03 (t, J=8.9 Hz, 2H), 2.71 - 2.63 (m, 2H), 2.09 (s, 3H), 1.76 (td, J=11.5, 2.0 Hz, , 2H), 1.62 (dd, J=11.8, 2.0 Hz, 2H), 1.42 (q, J=8.0 Hz, 2H), 1.18 - 1.04 (m, 3H). | Rt = 0.33 min, m/z 461.3 [M+H]⁺ (Method 12) |
| | | | | *Note: reaction solvent DCM-DMF mix, coupling agent HATU* |
| 17 | 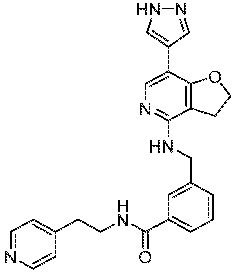 3-(((7-(1H-pyrazol-4-yl)-2,3-dihydrofuro[3,2-c]pyridin-4-yl)amino)methyl)-N-(2-(pyridin-4-yl)ethyl)benzamide | 2-(pyridin-4-yl)ethanamin e | (400MHz, DMSO-d6) δ 12.74 (br s, 1H), 8.48 (t, J=5.5 Hz, 1H), 8.42 (d, J=5.9 Hz, 2H), 8.04 (s, 1H), 7.88 (br s, 1H), 7.77 (br s, 1H), 7.75 (s, 1H), 7.58 (dt, J=7.7, 1.0 Hz, 1H), 7.43 (dt, J=7.7, 1.0 Hz, 1H), 7.32 (t, J=7.7 Hz, 1H), 7.22 (d, J=5.9 Hz, 2H), 6.66 (t, J=5.8 Hz, 1H), 4.66 (t, J=8.9 Hz, 2H), 4.57 (d, J=6.1 Hz, 2H), 3.48 (q, J=5.8 Hz, 2H), 3.02 (t, J=8.9 Hz, 2H), 2.83 (t, J=7.1 Hz, 2H). | Rt = 0.72 min, m/z 441.2 [M+H]⁺ (Method 12) |
| | | | | *Note: reaction solvent DCM-DMF mix, coupling agent HATU* |
| 18 | 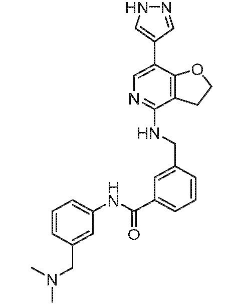 3-(((7-(1H-pyrazol-4-yl)-2,3-dihydrofuro[3,2-c]pyridin-4-yl)amino)methyl)-N-(3-((dimethylamino)methyl)ph enyl)benzamide | 3-((dimethylam ino)methyl)an iline | (400MHz, DMSO-d6) δ12.74 (br s, 1H), 10.16 (s, 1H), 8.05 (s, 1H), 7.89 (s, 2H), 7.78 (s, 1H), 7.76 (s, 1H), 7.71 (br s, 1H), 7.65 (d, J=8.1 Hz, 1H), 7.50 (d, J=7.7 Hz, 1H), 7.40 (t, J=7.7 Hz, 1H), 7.25 (t, J=7.8 Hz, 1H), 6.98 (d, J=7.2 Hz, 1H), 6.68 (t, J=6.0 Hz, 1H), 4.66 (t, J=8.9 Hz, 2H), 4.62 (d, J=5.9 Hz, 2H), 3.34 - 3.33 (m, 2H), 3.03 (t, J=8.9 Hz, 2H), 2.15 (s, 6H). | Rt = 0.39 min, m/z 469.3 [M+H]⁺ (Method 12) |
| | | | | *Note: reaction solvent DCM-DMF mix, coupling agent HATU* |
| 19 | 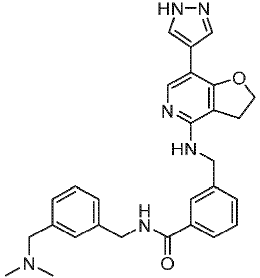 3-(((7-(1H-pyrazol-4-yl)-2,3-dihydrofuro[3,2-c]pyridin-4-yl)amino)methyl)-N-(3-((dimethylamino)methyl)be nzyl)benzamide | 1-(3-(aminomethyl ) phenyl)-N,N-dimethylmeth anamine | (400MHz, DMSO-d6) δ12.74 (br s, 1H), 8.95 (t, J=5.9 Hz, 1H), 8.04 (s, 1H), 7.84 (s, 1H), 7.69 (dt, J=7.7, 1.0 Hz, 1H), 7.45 (dt, J=7.7, 1.0 Hz, 1H), 7.35 (t, J=7.7 Hz, 1H), 7.27 - 7.18 (m, 3H), 7.17 - 7.12 (m, 1H), 7.12 - 7.09 (m, 1H), 6.65 (t, J=5.9 Hz, 1H), 4.65 (t, J=8.9 Hz, 2H), 4.58 (d, J=5.9 Hz, 2H), 4.43 (d, J=5.9 Hz, 2H), 3.31 (s, 2H), 3.01 (t, J=8.9 Hz, 2H), 2.08 (s, 6H). | Rt = 0.38 min, m/z 483.3 [M+H]⁺ (Method 12) |
| | | | | *Note: reaction solvent DCM-DMF mix, coupling agent HATU* |
| 20 | 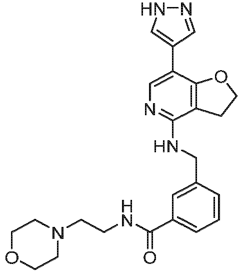 3-(((7-(1H-pyrazol-4-yl)-2,3-dihydrofuro[3,2-c]pyridin-4-yl)amino)methyl)-N-(2-morpholinoethyl)benzamid e | 2-morpholinoet ha namine | (400MHz, DMSO-d6) δ12.74 (br s, 1H), 8.29 (t, J=5.6 Hz, 1H), 8.04 (s, 1H), 7.88 (s, 1H), 7.77 (br s, 2H), 7.61 (dt, J=7.7, 1.0 Hz, 1H), 7.43 (dt, J=7.9 Hz, 1H), 7.33 (t, J=7.7 Hz, 1H), 6.65 (t, J=6.0 Hz, 1H), 4.65 (t, J=8.9 Hz, 2H), 4.57 (d, J=6.0 Hz, 2H), 3.56 - 3.49 (m, 4H), 3.33 (q, J=6.0 Hz, 2H), 3.01 (t, J=8.9 Hz, 2H), 2.42 (t, J=6.0 Hz, 2H), 2.39 - 2.34 (m, 4H). | Rt = 0.29 min, m/z 449.3 [M+H]⁺ (Method 12) |
| | | | | *Note: reaction solvent DCM-DMF mix, coupling agent HATU* |
| 21 | 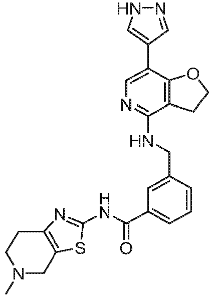 3-(((7-(1H-pyrazol-4-yl)-2,3-dihydrofuro[3,2-c]pyridin-4-yl)amino)methyl)-N-(5-methyl-4,5,6,7-tetrahydrothiazolo[5,4-c]pyridin-2-yl)benzamide | 5-methyl-4,5,6,7-tetrahydrothia zolo[5,4-c]pyridin-2-amine | (400MHz, DMSO-d6) δ 12.74 (s, 1H), 12.41 (s, 1H), 8.04 (s, 1H), 8.00 (s, 1H), 7.93 - 7.85 (m, 2H), 7.76 (s, 1H), 7.53 (d, J=7.7 Hz, 1H), 7.41 (t, J=7.7 Hz, 1H), 6.67 (t, J=6.1 Hz, 1H), 4.66 (t, J=8.8 Hz, 2H), 4.61 (d, J=5.9 Hz, 2H), 3.48 (s, 2H), 3.03 (t, J=8.8 Hz, 2H), 2.72 - 2.60 (m, 4H), 2.35 (s, 3H). | Rt = 0.41 min, m/z 488.1 [M+H]⁺ (Method 12) |
| | | | | *Note: reaction solvent DCM-DMF mix, coupling agent HATU* |
| 22 | 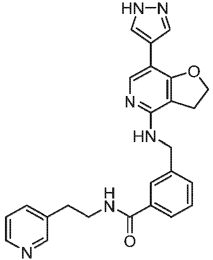 3-(((7-(1H-pyrazol-4-yl)-2,3-dihydrofuro[3,2-c]pyridin-4-yl)amino)methyl)-N-(2-(pyridin-3-yl)ethyl)benzamide | 2-(pyridin-3-yl)ethan amine | (400MHz, DMSO-d6) δ12.74 (br s, 1H), 8.48 (t, J=5.5 Hz, 1H), 8.41 (d, J=1.8 Hz, 1H), 8.37 (dd, J=4.8, 1.5 Hz, 1H), 8.04 (s, 1H), 7.88 (br s, 1H), 7.76 (br s, 1H), 7.75 (s, 1H), 7.64 - 7.56 (m, 2H), 7.43 (d, J=7.7 Hz, 1H), 7.32 (t, J=7.7 Hz, 1H), 7.26 (dd, J=7.6, 4.5 Hz, 1H), 6.64 (t, J=6.1 Hz, 1H), 4.66 (t, J=8.9 Hz, 2H), 4.57 (d, J=5.9 Hz, 2H), 3.47 (q, J=6.0 Hz, 2H), 3.01 (t, J=8.9 Hz, 2H), 2.83 (t, J=7.0 Hz, 2H). | Rt = 0.39 min, m/z 441.2 [M+H]⁺ (Method 12) |
| | | | | *Note: reaction solvent DCM-DMF mix, coupling agent HATU* |
| 23 | 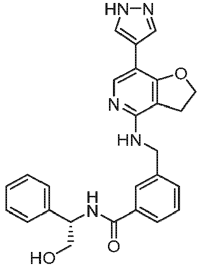 (S)-3-(((7-(1H-pyrazol-4-yl)-2,3-dihydrofuro[3,2-c]pyridin-4-yl)amino)methyl)-N-(2-hydroxy-1-phenylethyl)benzamide | (S)-2-amino-2-phenylethanol | (400MHz, DMSO-d6) δ 12.72 (br s, 1H), 8.59 (d, J=8.1 Hz, 1H), 8.04 (s, 1H), 7.90 - 7.74 (m, 3H), 7.71 (d, J=7.7 Hz, 1H), 7.45 (d, J=7.7 Hz, 1H), 7.39 - 7.31 (m, 3H), 7.30 - 7.24 (m, 2H), 7.22 - 7.16 (m, 1H), 6.65 (t, J=6.1 Hz, 1H), 5.06 - 4.99 (m, 1H), 4.87 (t, J=5.9 Hz, 1H), 4.65 (t, J=8.9 Hz, 2H), 4.59 (d, J=6.1 Hz, 2H), 3.71 - 3.58 (m, 2H), 3.01 (t, J=8.8 Hz, 2H). | Rt = 0.58 min, m/z 456.3 [M+H]⁺ (Method 12) |
| | | | | *Note: reaction solvent DCM-DMF mix, coupling agent HATU* |
| 24 | 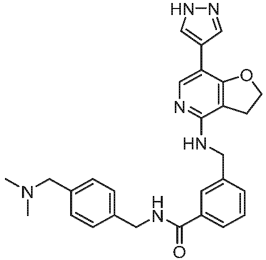 3-(((7-(1H-pyrazol-4-yl)-2,3-dihydrofuro[3,2-c]pyridin-4-yl)amino)methyl)-N-(4-((dimethylamino)methyl)be nzyl)benzamide | 1-(4-(aminomethyl )phenyl)-N,N-dimethylmeth anamine hydrochloride | (400MHz, DMSO-d6) δ 12.74 (br s, 1H), 8.93 (t, J=5.8 Hz, 1H), 8.04 (s, 1H), 7.87 (s, 1H), 7.84 (s, 1H), 7.75 (s, 1H), 7.69 (d, J=7.7 Hz, 1H), 7.45 (d, J=7.5 Hz, 1H), 7.34 (t, J=7.7 Hz, 1H), 7.26 - 7.12 (m, 4H), 6.64 (t, J=5.9 Hz, 1H), 4.65 (t, J=8.9 Hz, 2H), 4.58 (d, J=5.9 Hz, 2H), 4.42 (d, J=5.9 Hz, 2H), 3.30 (s, 2H), 3.01 (t, J=8.9 Hz, 2H), 2.08 (s, 6H). | Rt = 0.31 min, m/z 483.2 [M+H]⁺ (Method 12) |
| | | | | *Note: reaction solvent DCM-DMF mix, coupling agent HATU* |
| 25 | 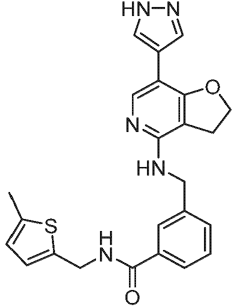 3-(((7-(1H-pyrazol-4-yl)-2,3-dihydrofuro[3,2-c]pyridin-4-yl)amino)methyl)-N-((5-methylthiophen-2-yl)methyl)benzamide | (5-methylthioph en-2-yl)methanami ne | (400MHz, DMSO-d6) δ 12.74 (s, 1H), 8.98 (t, J=5.9 Hz, 1H), 8.04 (s, 1H), 7.88 (s, 1H), 7.81 (s, 1H), 7.76 (s, 1H), 7.65 (d, J=7.7 Hz, 1H), 7.44 (d, J=7.7 Hz, 1H), 7.33 (t, J=7.7 Hz, 1H), 6.73 (d, J=3.5 Hz, 1H), 6.65 (t, J=6.0 Hz, 1H), 6.57 (dd, J=3.5, 1.1 Hz, 1H), 4.65 (t, J=8.9 Hz, 2H), 4.57 (d, J=6.0 Hz, 2H), 4.48 (d, J=5.9 Hz, 2H), 3.01 (t, J=8.9 Hz, 2H), 2.33 (s, 3H). | Rt = 0.58 min, m/z 446.3 [M+H]⁺ (Method 12) |
| | | | | *Note: reaction solvent DCM-DMF mix, coupling agent HATU* |
| 26 | 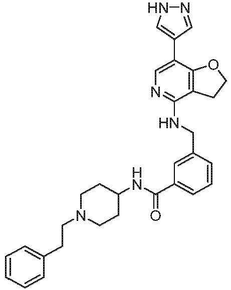 3-(((7-(1H-pyrazol-4-yl)-2,3-dihydrofuro[3,2-c]pyridin-4-yl)amino)methyl)-N-(1-phenethylpiperidin-4-yl)benzamide | 1-phenethylpipe ridin-4-amine | (400MHz, DMSO-d6) δ 12.74 (s, 1H), 8.15 (d, J=7.9 Hz, 1H), 8.04 (s, 1H), 7.88 (br s , 1H), 7.77 (s, 1H), 7.76 (br s, 1H), 7.63 (d, J=7.5 Hz, 1H), 7.43 (d, J=7.7 Hz, 1H), 7.32 (t, J=7.5 Hz, 1H), 7.26 - 7.11 (m, 5H), 6.64 (t, J=6.0 Hz, 1H), 4.65 (t, J=8.9 Hz, 2H), 4.57 (d, J=6.0 Hz, 2H), 3.77 - 3.65 (m, 1H), 3.01 (t, J=8.9 Hz, 2H), 2.91 (d, J=11.8 Hz, 2H), 2.72 - 2.66 (m, 2H), 2.03 - 1.95 (m, 2H), 1.77 - 1.70 (m, 2H), 1.53 (dq, J=11.8, 3.3 Hz, 2H). | Rt = 0.46 min, m/z 523.4 [M+H]⁺ (Method 12) |
| | | | | *Note: reaction solvent DCM-DMF mix, coupling agent HATU* |
| 27 | 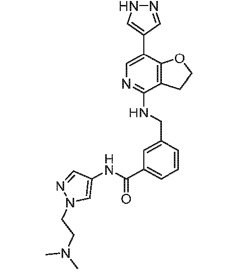 3-(((7-(1H-pyrazol-4-yl)-2,3-dihydrofuro[3,2-c]pyridin-4-yl)amino)methyl)-N-(1-(2-(dimethylamino)ethyl)-1H-pyrazol-4-yl)benzamide | 1-(2-(dimethylami no)ethyl)-1H-pyrazol-4-amine hydrochloride | (400MHz, DMSO-d6) δ 12.74 (br s, 1H), 10.33 (s, 1H), 8.05 (s, 1H), 8.02 (s, 1H), 7.89 (br s, 1H), 7.87 (s, 1H), 7.76 (br s, 1H), 7.74 (d, J=7.7 Hz, 1H), 7.54 (s, 1H), 7.48 (d, J=7.7 Hz, 1H), 7.39 (t, J=7.7 Hz, 1H), 6.67 (t, J=6.0 Hz, 1H), 4.66 (t, J=8.9 Hz, 2H), 4.61 (d, J=6.0 Hz, 2H), 4.13 (t, J=6.5 Hz, 2H), 3.02 (t, J=8.9 Hz, 2H), 2.58 (t, J=6.5 Hz, 2H), 2.13 (s, 6H). | Rt = 0.31 min, m/z 473.4 [M+H]⁺ (Method 12) |
| | | | | *Note: reaction solvent DCM-DMF mix, coupling agent HATU* |
| 28 | 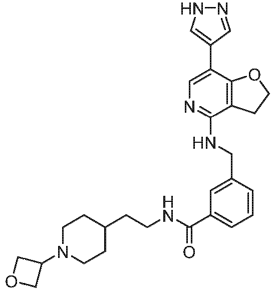 3-(((7-(1H-pyrazol-4-yl)-2,3-dihydrofuro[3,2-c]pyridin-4-yl)amino)methyl)-N-(2-(1-(oxetan-3-yl)piperidin-4-yl)ethyl)benzamide | Intermediate A1 | (400MHz, DMSO-d6) δ 12.74 (br s, 1H), 8.32 (t, J=5.5 Hz, 1H), 8.03 (s, 1H), 7.87 (br s, 1H), 7.76 (br s, 2H), 7.61 (d, J=7.8 Hz, 1H), 7.42 (d, J=7.8 Hz, 1H), 7.32 (t, J=7.8 Hz, 1H), 6.63 (t, J=6.1 Hz, 1H), 4.65 (t, J=8.9 Hz, 2H), 4.57 (d, J=5.9 Hz, 2H), 4.46 (t, J=6.1 Hz, 2H), 4.35 (t, J=6.1 Hz, 2H), 3.26 - 3.20 (m, 3H), 3.01 (t, J=8.9 Hz, 2H), 2.63 - 2.57 (m, 2H), 1.69 - 1.59 (m, 4H), 1.42 (q, J=6.9 Hz, 2H), 1.29 - 1.21 (m, 1H), 1.16 - 1.07 (m, 2H). | Rt = 0.29 min, m/z 503.4 [M+H]⁺ (Method 12) |
| | | | | *Note: reaction solvent DCM-DMF mix, coupling agent HATU* |
| 29 | 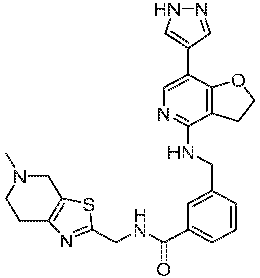 3-(((7-(1H-pyrazol-4-yl)-2,3-dihydrofuro[3,2-c]pyridin-4-yl)amino)methyl)-N-((5-methyl-4,5,6,7-tetrahydrothiazolo[5,4-c]pyridin-2-yl)methyl)benzamide | Intermediate A2 | (400MHz, DMSO-d6) δ 12.73 (br s, 1H), 9.25 (t, J=5.8 Hz, 1H), 8.04 (s, 1H), 7.83 (s, 1H), 7.82 - 7.74 (br s, 2H), 7.68 (d, J=7.7 Hz, 1H), 7.47 (d, J=7.7 Hz, 1H), 7.36 (t, J=7.7 Hz, 1H), 6.65 (t, J=6.1 Hz, 1H), 4.65 (t, J=8.9 Hz, 2H), 4.62 - 4.56 (m, 4H), 3.46 (s, 2H), 3.02 (t, J=8.9 Hz, 2H), 2.71 - 2.61 (m, 4H), 2.31 (s, 3H). | Rt = 0.25 min, m/z 502.0 [M+H]⁺ (Method 12) |
| | | | | *Note: reaction solvent DCM-DMF mix, coupling agent HATU* |
| 30 | 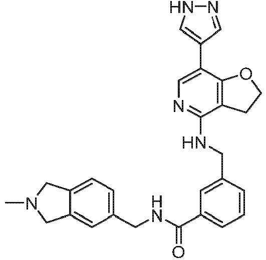 3-(((7-(1H-pyrazol-4-yl)-2,3-dihydrofuro[3,2-c]pyridin-4-yl)amino)methyl)-N-((2-methylisoindolin-5-yl)methyl)benzamide | Intermediate A3 | (400MHz, DMSO-d6) δ 12.74 (br s, 1H), 8.92 (t, J=6.0 Hz, 1H), 8.04 (br s, 1H), 7.88 (br s, 1H), 7.83 (s, 1H), 7.76 (br s, 1H), 7.68 (d, J=7.7 Hz, 1H), 7.44 (d, J=7.7 Hz, 1H), 7.34 (t, J=7.7 Hz, 1H), 7.17 - 7.02 (m, 3H), 6.65 (t, J=6.0 Hz, 1H), 4.65 (t, J=8.9 Hz, 2H), 4.57 (d, J=6.0 Hz, 2H), 4.40 (d, J=6.0 Hz, 2H), 3.72 (s, 4H), 3.01 (t, J=8.9 Hz, 2H), 2.41 (s, 3H). | Rt = 0.29 min, m/z 480.6 [M+H]⁺ (Method 12) |
| | | | | *Note: reaction solvent DCM-DMF mix, coupling* |

### Example 31

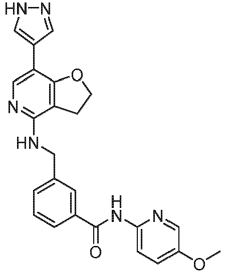

### 3-(((7-(1H-Pyrazol-4-yl)-2,3-dihydrofuro[3,2-c]pyridin-4-yl)amino)methyl)-N-(5-methoxypyridin-2-yl)benzamide (Example 31)

Intermediate G (75 mg, 0.22 mmol), 2-amino-5-methoxypyridine (83 mg, 0.67 mmol), 1-(methylsulfonyl)-1H-benzotriazole (264 mg, 1.34 mmol), triethylamine (0.31 mL, 2.23 mmol) and THF (10 mL) were loaded into a microwave vial and stirred under microwave irradiation at 150°C for 3 h. A further 1 equivalent of 2-amino-5-methoxypyridine and 1-(methylsulfonyl)-1H-benzotriazole and triethylamine were added and the mixture again stirred under microwave irradiation at 150°C for 3 h. The reaction mixture was loaded onto an Isolute^{®} SCX-2 cartridge, washed with MeOH and eluted with 7N NH₃ in MeOH. The samples were concentrated and purified by MDAP (Luna Phenyl-Hexyl 3x50mm, 3µm 5-95% MeOH/H₂O (0.1% FA), 1.7mL/min, RT) to give the title compound (23.1 mg).

LCMS (Method 3): Rt = 2.68 min, m/z 443.2 [M+H]⁺

¹H NMR (400 MHz, DMSO-d6) δ 10.61 (s, 1H), 8.12 (s, 1H), 8.11 - 8.09 (m, 2H), 8.00 (s, 1H), 7.90 - 7.85 (m, 3H), 7.55 (d, J=7.6 Hz, 1H), 7.50 (d, J=3.2 Hz, 1H), 7.48 (d, J=3.2 Hz, 1H), 7.43 (t, J=7.6 Hz, 1H), 6.72 (t, J=6.2 Hz, 1H), 4.71 (t, J=9.0 Hz, 2H), 4.66 (d, J=6.0 Hz, 2H), 3.85 (s, 3H), 3.08 (t, J=9.0 Hz, 2H)

### Example 32

The following example was prepared using a procedure similar to that used for the synthesis of *example 31* by replacing intermediate G with the amine given.

| **Ex** | **Structure** | **Amine** | **¹H NMR** | **LC-MS** |
|---|---|---|---|---|
| 32 | 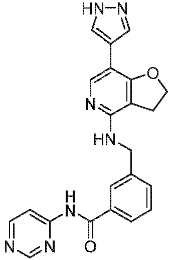 3-(((7-(1H-pyrazol-4-yl)-2,3-dihydrofuro[3,2-c]pyridin-4-yl)amino)methyl)-N-(pyrimidin-4-yl)benzamide | 4-Amino-pyrimidine | ¹H NMR (400 MHz, DMSO-d6) δ 12.78 (s, 1H), 11.19 (s, 1H), 8.95 (d, J=1.0 Hz, 1H), 8.71 (d, J=5.8 Hz, 1H), 8.20 (dd, J=1.1, 5.8 Hz, 1H), 8.08 (s, 1H), 8.01-7.97 (m, 1H), 7.90-7.84 (m, 3H), 7.60-7.55 (m, 1H), 7.47-7.42 (m, 1H), 6.72 (t, J=6.1 Hz, 1H), 4.69 (t, J=8.8 Hz, 2H), 4.65 (d, J=6.1 Hz, 2H), 3.06 (t, J=8.9 Hz, 2H). | Rt = 2.28 min, m/z 414.3 [M+H]⁺ (Method 5) |

### Example 33

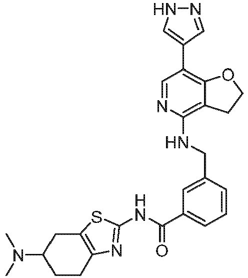

### 3-(((7-(1H-Pyrazol-4-yl)-2,3-dihydrofuro[3,2-c]pyridin-4-yl)amino)methyl)-N-(6-(dimethylamino)-4,5,6,7-tetrahydrobenzo[d]thiazol-2-yl)benzamide (Example 35)

Paraformaldehyde (13 mg, 0.43 mmol) and sodium cyanoborohydride (27 mg, 0.43 mmol) were added to a stirred suspension of intermediate H1 (70 mg, 0.14 mmol) in MeOH (2.0 mL) and DMF (0.5 mL). The resulting mixture was stirred for 20 h then treated with aqueous 1M NaOH (5.0 mL). The mixture was extracted with DCM (2 x 10 mL) then with 10% MeOH in DCM (4 x 5 mL). The combined organic phase was passed through an Isolute^{®} SCX-2 cartridge which was then washed with 1:1 DCM:MeOH and eluted with 20% 2M methanolic ammonia in DCM. Concentration *in vacuo* gave the crude product which was purified by MDAP (Luna Phenylhexyl 21.2x150 mm, 10 µm 5-60% MeOH/H₂O (0.1% FA) 20mL/ min) to afford the desired product as formate salt (21.6 mg)

LCMS (Method 3): Rt = 2.05 min, m/z 516.4 [M+H]⁺

¹H NMR (400 MHz, DMSO-*d6*) δ 12.40 (br s, 2H), 8.18 (s, 1H), 8.09 (s, 1H), 8.04 (s, 1H), 7.93 (d J=7.4 Hz, 1H), 7.87 (s, 2H), 7.57 (d J=7.9 Hz, 1H), 7.45 (t J=7.7 Hz, 1H), 6.73 (t J=6.0 Hz, 1H), 4.71 (t J=8.9 Hz, 2H), 4.65, (d J=6.0 Hz, 2H), 3.08 (t J=8.9 Hz, 2H), 2.90-2.60 (m, 6H), 2.32 (s, 6H), 2.09-2.01 (m, 1H), 1.76-1.64 (m, 1H).

### Example 34

The following example was prepared from intermediate H1 and oxetan-3-one using a procedure similar to that used to prepare *example 33.*

| **Ex** | **Structure** | **¹H NMR** | **LC-MS** |
|---|---|---|---|
| 34 | 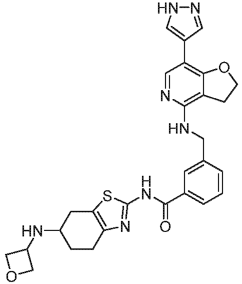 3-(((7-(1H-pyrazol-4-yl)-2,3-dihydrofuro[3,2-c]pyridin-4-yl)amino)methyl)-N-(6-(oxetan-3-ylamino)-4,5,6,7-tetrahydrobenzo[d]thiazol-2-yl)benzamide | ¹H NMR (400 MHz, DMSO-d6) δ 12.82 (s, 1H), 8.10 (s, 1H), 8.05 (s, 1H), 7.93 (d, J=7.7 Hz, 1H), 7.87 (s, 1H), 7.53 (d, J=7.4 Hz, 1H), 7.44 - 7.39 (m, 1H), 6.72 (dd, J=6.0, 6.0 Hz, 1H), 4.74 - 4.62 (m, 5H), 4.41 (s, 2H), 4.35 (m, 2H), 4.06 - 3.98 (m, 3H), 3.07 (t, J=8.8 Hz, 2H), 2.92 - 2.79 (m, 2H), 2.70 - 2.57 (m, 2H), 2.39 - 2.33 (m, 1H), 1.95 - 1.83 (m, 1H), 1.61 - 1.56 (m, 1H). | Rt = 2.02 min, m/z 544.0 [M+H]⁺ (Method 3) |

### Example 35

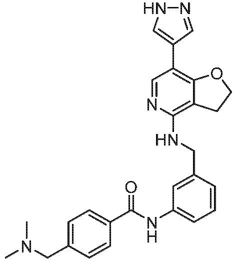

**N-(3-(((7-(1H-Pyrazol-4-yl)-2,3-dihydrofuro[3,2-c]pyridin-4-yl)amino)methyl)phenyl)-4-((dimethylamino)methyl)benzamide (Example 35)**

Intermediate K1 was dissolved in acetonitrile (2 mL) and then aqueous 12 M HCl (2 mL) was added. The solution was stirred for 1 h at RT until complete conversion. The reaction mixture was dried under reduced pressure and the resulting crude purified by flash chromatography on C18-silica by eluting 0-20% B with A (A: water/acetonitrile 95/5 + 0.1% HCOOH, B: acetonitrile/water 95/5 + 0.1% HCOOH) to give the desired product (3.8 mg).

LCMS (Method 13): Rt = 3.24 min, m/z 469.2 [M+H]⁺

¹H NMR (400 MHz, DMSO-d6) δ 10.25 (s, 1H), 8.13 (s, 1H), 8.08 (s, 1H), 7.95 - 8.00 (d, J=7.9Hz, 2H), 7.85 (s, 2H), 7.73 (s, 1H), 7.51 - 7.68 (m, 3H), 7.27 (t, J=7.9 Hz, 1H), 7.09 (d, J=7.9 Hz, 1H), 6.67 (br s, 1H), 6.50 (s, 1H), 4.71 (t, J=4.7 Hz, 2H), 4.60 (d, J=4.6 Hz, 2H), 3.06 (br t, J=9.0 Hz, 2H), 2.60 (s, 6H).

### Example 36 to 52

The following examples were prepared in a similar manner of *example* 35 by replacing intermediate K1 with the given intermediates in the table below.

| **Ex** | **Structure** | **Intermediate** | **¹H NMR** | **LC-MS** |
|---|---|---|---|---|
| 36 | 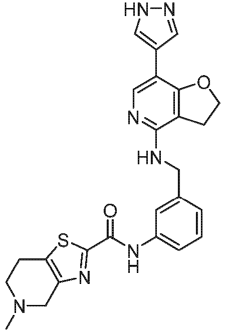 N-(3-(((7-(1H-pyrazol-4-yl)-2,3-dihydrofuro[3,2-c]pyridin-4-yl)amino)methyl)phenyl)-5-methyl-4,5,6,7-tetrahydrothiazolo[4,5-c]pyridine-2-carboxamide | Intermediate K2 | (400 MHz, DMSO-d6) δ 10.54 - 10.59 (m, 1H), 8.16 (s, 1H), 8.06 - 8.10 (m, 1H), 7.80 - 7.89 (m, 3H), 7.61 - 7.68 (m, 1H), 7.26 (t, J=7.8 Hz, 1H), 7.09 (d, J=7.7 Hz, 1H), 6.63 (t, J=6.0 Hz, 1H), 4.64 - 4.73 (m, 2 H), 4.57 (d, J=5.9 Hz, 2H), 3.61 (s, 2H), 3.05 (br t, J=8.9 Hz, 2 H), 2.95 (br t, J=5.59 Hz, 2H), 2.68 - 2.73 (m, 2H), 2.42 (s, 3H). | Rt = 3.24 min, m/z 488.2 [M+H]⁺ (Method 13) |
| 37 | 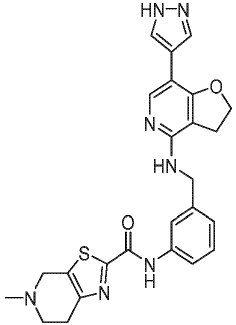 N-(3-(((7-(1H-pyrazol-4-yl)-2,3-dihydrofuro[3,2-c]pyridin-4-yl)amino)methyl)phenyl)-5-methyl-4,5,6,7-tetrahydrothiazolo[5,4-c]pyridine-2-carboxamide | Intermediate K3 | (400 MHz, DMSO-d6) δ 10.58 (s, 1H), 8.15 (s, 1H), 8.07 (s, 1H), 7.78 - 7.92 (m, 3H), 7.64 (d, J=7.9 Hz, 1H), 7.25 (t, J=7.7 Hz, 1H), 7.09 (d, J=7.9 Hz, 1H), 6.58 - 6.73 (m, 1H), 4.68 (t, J=8.8 Hz, 2H), 4.56 (d, J=5.7 Hz, 2H), 3.69 (s, 2H), 3.05 (t, J=9.0 Hz, 2H), 2.83 - 2.96 (m, 2H), 2.68 - 2.83 (m, 2H), 2.39 (s, 3H). | Rt = 3.19 min, m/z 488.1 [M+H]⁺ (Method 13) |
| 38 | 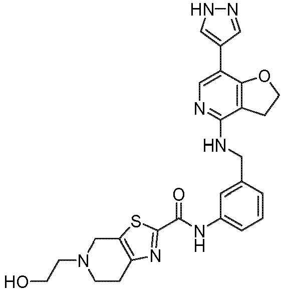 N-(3-(((7-(1H-pyrazol-4-yl)-2,3-dihydrofuro[3,2-c]pyridin-4-yl)amino)methyl)phenyl)-5-(2-hydroxyethyl)-4,5,6,7-tetrahydrothiazolo[5,4-c]pyridine-2-carboxamide | Intermediate K4 | (400 MHz, DMSO-d6) δ 13.31 (br s, 1H), 11.17 (br s, 1H), 10.83 (s, 1H), 8.63 (br t, J=6.1 Hz, 1H), 8.07 (s, 2 H), 7.99 (s, 1H), 7.89 (s, 1H), 7.77 (br d, J=8.3 Hz, 1H), 7.37 (t, J=7.9 Hz, 1H), 7.17 (br d, J=7.89 Hz, 1H), 4.97 (br t, J=9.2 Hz, 2H), 3.15 - 4.89 (m, 14 H). | Rt = 3.12 min, m/z 518.2 [M+H]⁺ (Method 13) |
| 39 | 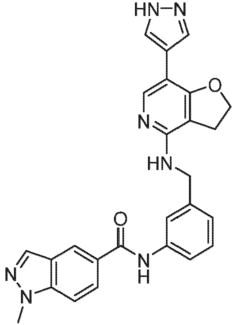 N-(3-(((7-(1H-pyrazol-4-yl)-2,3-dihydrofuro[3,2-c]pyridin-4-yl)amino)methyl)phenyl)-1-methyl-1H-indazole-5-carboxamide | Intermediate K5 | (400 MHz, DMSO-d6) δ 12.77 (s, 1H), 10.22 (s, 1 H), 8.45 (s, 1H), 8.23 (d, J=0.9 Hz, 1H), 8.09 (s, 1H), 7.58 - 8.02 (m, 6H), 7.27 (t, J=7.7 Hz, 1H), 7.07 (d, J=7.5 Hz, 1H), 6.64 (t, J=5.9, 1H), 4.69 (t, J=9.0 Hz, 2H), 4.59 (d, J=4.6 Hz, 2H), 4.09 (s, 3 H), 3.06 (t, J=9.0 Hz, 2H). | Rt = 4.03 min, m/z 466.2 [M+H]⁺ (Method 13) |
| 40 | 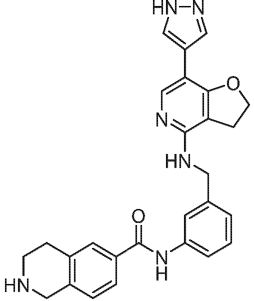 N-(3-(((7-(1H-pyrazol-4-yl)-2,3-dihydrofuro[3,2-c]pyridin-4-yl)amino)methyl)phenyl)-1,2,3,4-tetrahydroisoquinoline-6-carboxamide | Intermediate K6 | (400 MHz, DMSO-d6) d ppm 13.24 (br s, 1H), 10.33 (s, 1H), 9.46 (br s, 2H), 8.62 (br s, 1H), 7.99 - 8.07 (m, 3H), 7.80 - 7.90 (m, 3H), 7.68 (d, J=9.2 Hz, 1H), 7.23 - 7.49 (m, 2H), 6.87 - 7.23 (m, 1H), 4.97 (t, J=9.0 Hz, 2H), 4.74 (d, J=6.1 Hz, 2H), 4.27 - 4.47 (m, 2H), 3.40 (d, J=6.1 Hz, 2H), 3.25 (t, J=9.2 Hz, 2H), 3.09 (t, J=6.1 Hz, 2H). | Rt = 3.45 min, m/z 467.1 [M+H]⁺ (Method 13) |
| 41 | 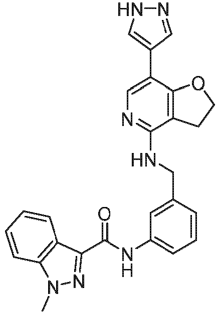 N-(3-(((7-(1H-pyrazol-4-yl)-2,3-dihydrofuro[3,2-c]pyridin-4-yl)amino)methyl)phenyl)-1-methyl-1H-indazole-3-carboxamide | Intermediate K7 | (400 MHz, DMSO-d6) δ 10.20 (s, 1H), 8.22 (d, J=7.9 Hz, 2H), 8.09 (s, 1H), 7.80 - 7.85 (m, 3H), 7.78 (d, J=8.8 Hz, 1H), 7.70 (d, J=8.3 Hz, 1H), 7.50 (t, J=7.5 Hz, 1H), 7.32 (t, J=7.5 Hz, 1H), 7.26 (t, J=7.9 Hz, 1H), 7.07 (d, J=7.5 Hz, 1H), 6.63 (t, J=6.1 Hz, 1H), 4.69 (t, J=8.8 Hz, 2H), 4.59 (d, J=6.14 Hz, 2H), 4.19 (s, 3 H), 3.07 (t, J=8.8 Hz, 2H). | Rt = 4.74 min, m/z 466.4 [M+H]⁺ (Method 13) |
| 42 | 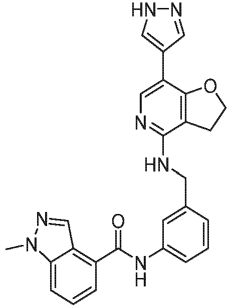 N-(3-(((7-(1H-pyrazol-4-yl)-2,3-dihydrofuro[3,2-c]pyridin-4-yl)amino)methyl)phenyl)-1-methyl-1H-indazole-4-carboxamide | Intermediate K8 | (400 MHz, DMSO-d6) δ 12.16 - 12.95 (br s, 1H), 10.34 (s, 1H), 8.32 (s, 1H), 8.09 (s, 1H), 7.82 - 7.90 (m, 3H), 7.79 (s, 1H), 7.75 (d, J=7.0 Hz, 1H), 7.66 - 7.76 (m, 1H), 7.52 (dd, J=8.3, 7.5 Hz, 1H), 7.28 (t, J=7.9 Hz, 1H), 7.09 (d, J=7.5 Hz, 1H), 6.66 (m, 1H), 4.69 (t, J=8.8 Hz, 2H), 4.60 (d, J=6.1 Hz, 2H), 4.10 (s, 3 H), 3.06 (t, J=9.0 Hz, 2H). | Rt = 4.07 min, m/z 466.1 [M+H]⁺ (Method 13) |
| 43 | 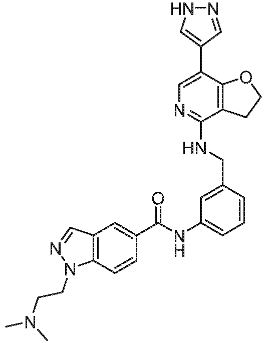 N-(3-(((7-(1H-pyrazol-4-yl)-2,3-dihydrofuro[3,2-c]pyridin-4-yl)amino)methyl)phenyl)-1-(2-(dimethylamino)ethyl)-1H-indazole-5-carboxamide | Intermediate K9 | (400 MHz, DMSO-d6) δ 12.77 (br s, 1H), 10.21 (s, 1H), 8.39 - 8.49 (m, 1H), 8.24 (s, 1H), 8.09 (s, 1H), 7.86 - 8.01 (m, 2H), 7.71 - 7.86 (m, 3H), 7.67 (m, J=7.89 Hz, 1H), 7.27 (t, J=7.89 Hz, 1H), 7.07 (m, 1H), 6.65 (t, J=6.14 Hz, 1H), 4.69 (t, J=8.99 Hz, 2H), 4.48 - 4.63 (m, 4H), 3.06 (t, J=8.77 Hz, 2H), 2.77 (br s 2H), 2.20 (br s, 6H). | Rt = 3.36 min, m/z 523.0 [M+H]⁺ (Method 13) |
| 44 | 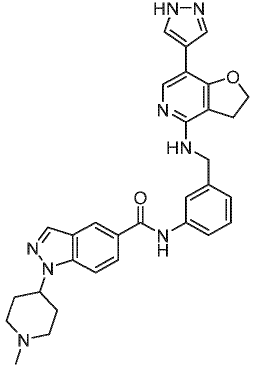 N-(3-(((7-(1H-pyrazol-4-yl)-2,3-dihydrofuro[3,2-c]pyridin-4-yl)amino)methyl)phenyl)-1-(1-methylpiperidin-4-yl)-1H-indazole-5-carboxamide | Intermediate K10 | (400 MHz, DMSO-d6) δ 10.21 (s, 1H), 8.46 (s, 1H), 8.27 (s, 1H), 8.14 (s, 1H), 8.09 (s, 1H), 7.98 (dd, J=9.0, 1.5 Hz, 1H), 7.78 - 7.89 (m, 3H), 7.75 (s, 1H), 7.60 - 7.70 (m, 1H), 7.27 (t, J=7.9 Hz, 1H), 7.07 (d, J=7.5 Hz, 1H), 6.65 (t, J=6.1 Hz, 1H), 4.60 - 4.70 (m, 3H), 4.59 (d, J=5.7 Hz, 2H), 2.98 - 3.16 (m, 5H), 2.37 - 2.47 (m, 4H), 2.23 (dq, J=12.1, 3.5 Hz, 2H), 2.00 (br s, 2H). | Rt = 3.43 min, m/z 549.2 [M+H]⁺ (Method 13) |
| 45 | 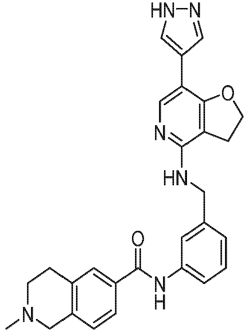 N-(3-(((7-(1H-pyrazol-4-yl)-2,3-dihydrofuro[3,2-c]pyridin-4-yl)amino)methyl)phenyl)-2-methyl-1,2,3,4-tetrahydroisoquinoline-6-carboxamide | Intermediate K11 | (400 MHz, DMSO-d6) δ 12.16 - 13.09 (br s, 1H), 10.23 (s, 1H), 8.07 (s, 1H), 7.88 (br s, 2H), 7.83 (s, 2H), 7.73 - 7.77 (m, 1H), 7.64 (d, J=8.3 Hz, 1H), 7.23 - 7.37 (m, 2H), 7.09 (d, J=7.5 Hz, 1H), 4.73 (t, J=8.1 Hz, 2H), 4.61 (d, J=5.7 Hz, 2H), 4.38 (br s, 2H), 3.35 - 3.61 (m, 2H), 3.01 - 3.24 (m, 4H), 2.89 (s, 3H). | Rt = 3.19 min, m/z 481.2 [M+H]⁺ (Method 13) |
| 46 | 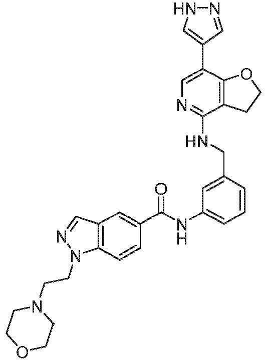 N-(3-(((7-(1H-pyrazol-4-yl)-2,3-dihydrofuro[3,2-c]pyridin-4-yl)amino)methyl)phenyl)-1-(2-morpholinoethyl)-1H-indazole-5-carboxamide | Intermediate K12 | (400 MHz, DMSO-d6) δ 12.76 (br s, 1H) 10.23 (s, 1H) 8.44 (s, 1H) 8.22 - 8.30 (m, 1H) , 8.13 (s, 1H), 8.08 (s, 1H), 7.97 (d, J=8.8 Hz, 1H), 7.85 - 7.90 (m, 2H), 7.70 - 7.80 (m, 2H), 7.67 (d, J=7.9 Hz, 1H), 7.28 (t, J=7.7 Hz, 1H), 7.08 (d, J=7.5 Hz, 1H), 4.73 (t, J=8.6 Hz, 2H), 4.58 - 4.68 (d, J=6.1 Hz, 4H), 3.40 - 3.50 (br s, 4H), 3.09 (t, J=8.8 Hz, 2H), 2.80 (br s, 2H), 2.40 - 2.50 (br s, 2H), 1.30 - 1.50 (m, 2H). | Rt = 3.40 min, m/z 565.2 [M+H]⁺ (Method 13) |
| 47 | 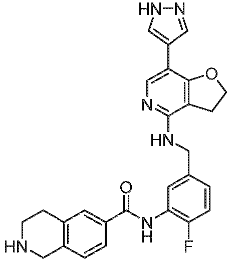 N-(5-(((7-(1H-pyrazol-4-yl)-2,3-dihydrofuro[3,2-c]pyridin-4-yl)amino)methyl)-2-fluorophenyl)-1,2,3,4-tetrahydroisoquinoline-6-carboxamide | Intermediate K13 | (400 MHz, DMSO-d6) δ 9.97 (s, 1H), 8.21 (s, 2H), 8.08 (s, 1H), 7.85 (s, 2H), 7.68 - 7.76 (m, 2H), 7.45 - 7.53 (m, 1H), 7.10 - 7.26 (m, 3H), 6.68 (t, J=6.1 Hz, 1H), 4.68 (t, J=8.8 Hz, 2H), 4.56 (d, J=6.1 Hz, 2H), 4.03 (s, 2H), 2.96 - 3.18 (m, 4H), 2.85 (t, J=5.9 Hz, 2H). | Rt = 3.21 min, m/z 485.1 [M+H]⁺ (Method 13) |
| 48 | 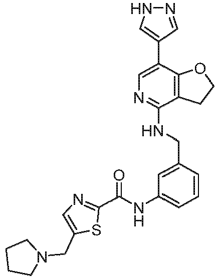 N-(3-(((7-(1H-pyrazol-4-yl)-2,3-dihydrofuro[3,2-c]pyridin-4-yl)amino)methyl)phenyl)-5-(pyrrolidin-1-ylmethyl)thiazole-2-carboxamide | Intermediate K14 | (400 MHz, DMSO-d6) δ 13.30 (br s, 1H), 11.53 - 11.63 (m, 1H), 10.85 (s, 1H), 8.65 (br t, J=6.0 Hz, 1H), 8.26 (s, 1H), 8.07 (s, 2H), 8.02 (s, 1H), 7.88 (s, 1H), 7.78 (br d, J=8.3 Hz, 1H), 7.38 (t, J=7.9 Hz, 1H), 7.18 (d, J=7.7 Hz, 1H), 4.98 (br t, J=9.1 Hz, 2H), 4.74 (br dd, J=9.2, 6.1 Hz, 4H), 3.34 - 3.39 (m, 2H), 3.25 (br t, J=9.1 Hz, 2H), 3.04 - 3.16 (m, 2H), 1.85 - 2.08 (m, 4H). | Rt = 3.24 min, m/z 502.3 [M+H]⁺ (Method 13) |
| 49 | 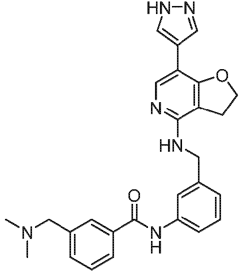 N-(3-(((7-(1H-pyrazol-4-yl)-2,3-dihydrofuro[3,2-c]pyridin-4-yl)amino)methyl)phenyl)-3-((dimethylamino)methyl)benz amide | Intermediate K15 | (400 MHz, DMSO-d₆) δ 10.26 (s, 1H), 8.13 (s, 1H), 8.08 (s, 1H), 8.03 (s, 1H), 7.98 (d, J=7.5 Hz, 1H), 7.85 (s, 2H), 7.72 (s, 1H), 7.65 (s, 2H), 7.53-7.61 (m, 1H), 7.28 (t, J=7.9 Hz, 1H), 7.09 (d, J=7.5 Hz, 1H), 6.61-6.76 (br s, 1H), 4.69 (m, 2H), 4.59 (d, J=6.1 Hz, 2H), 4.12 (br s, 2H), 3.06 (m, 2H), 2.58 (br s, 6H). | Rt = 3.06 min, m/z 469.2 [M+H]⁺ (Method 13) |
| 50 | 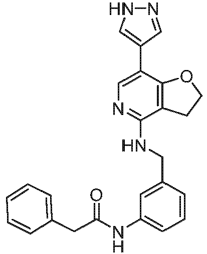 N-(3-(((7-(1H-pyrazol-4-yl)-2,3-dihydrofuro[3,2-c]pyridin-4-yl)amino)methyl)phenyl)-2-phenylacetamide | Intermediate K16 | (400 MHz, DMSO-d6) δ 12.27 - 13.05 (br s, 1H), 10.33 (s, 1H), 8.05 (s, 1H), 7.76 - 7.97 (m, 2H), 7.43 - 7.59 (m, 2H), 7.20 - 7.34 (m, 7H), 6.93 - 7.07 (m, 1H), 4.65 - 4.87 (m, 2H), 4.56 (d, J=6.1 Hz, 2H), 3.61 (s, 2H), 2.90 - 3.19 (m, 2H). | Rt = 4.08 min, m/z 426.1 [M+H]⁺ (Method 13) |
| 51 | 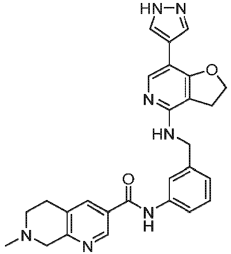 N-(3-(((7-(1H-pyrazol-4-yl)-2,3-dihydrofuro[3,2-c]pyridin-4-yl)amino)methyl)phenyl)-7-methyl-5,6,7,8-tetrahydro-1,7-naphthyridine-3-carboxamide | Intermediate K17 | (400 MHz, DMSO-d6) δ 13.31 (br s, 1H), 11.47 (br s, 1H), 10.60 (s, 1H), 8.97 (d, J=2.2 Hz, 1H), 8.64 (t, J=6.1 Hz, 1H), 8.24 (d, J=1.8 Hz, 1H), 8.07 (s, 2H), 8.02 (s, 1H), 7.85 (s, 1H), 7.69 (d, J=8.3 Hz, 1H), 7.37 (t, J=7.9 Hz, 1H), 7.15 (d, J=7.9 Hz, 1H), 4.97 (t, J=9.2 Hz, 2H), 4.76 (d, J=6.1 Hz, 2H), 4.32 - 4.58 (m, 4H), 3.30 - 3.45 (m, 2H), 3.25 (t, J=9.2 Hz, 2H), 2.95 (d, J=4.4 Hz, 3 H). | Rt = 3.03 min, m/z 482.1 [M+H]⁺ (Method 13) |
| 52 | 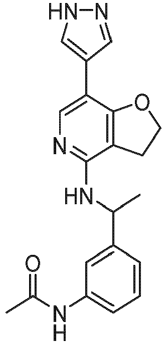 N-(3-(1-((7-(1H-pyrazol-4-yl)-2,3-dihydrofuro[3,2-c]pyridin-4-yl)amino)ethyl)phenyl)acetam ide | Intermediate I3 | (400 MHz, DMSO-d6) δ 9.83 (s, 1H), 8.02 (s, 1H), 7.83 (s, 2 H), 7.51 (s, 1H), 7.43 (br d, J=8.1 Hz, 1H), 7.18 (t, J=7.8 Hz, 1H), 7.06 (d, J=7.7 Hz, 1H), 6.33 (d, J=7.9 Hz, 1H), 5.17 - 5.26 (m, 1H), 4.68 (t, J=9.0 Hz, 2H), 3.08 (t, J=8.9 Hz, 2H), 2.01 (s, 3H), 1.44 (d, J=7.0 Hz, 3H). | Rt = 0.41 min, m/z 364.3 [M+H]⁺ (Method 6) |

### Example 53

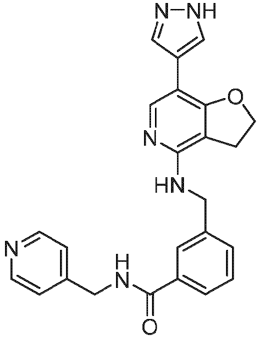

### 3-(((7-(1H-Pyrazol-4-yl)-2,3-dihydrofuro[3,2-c]pyridin-4-yl)amino)methyl)-N-(pyridin-4-ylmethyl)benzamide (Example 53)

Intermediate E3 (90 mg, 0.205 mmol), 1-(tetrahydro-2H-pyran-2-yl)-4-(4,4,5,5-tetramethyl-1,3,2-dioxaborolan-2-yl)-1H-pyrazole (114 mg, 0.410 mmol), tripotassium phosphate (130 mg, 0.615 mmol) were dissolved in THF (1 mL) and water (1 mL). The reaction was purged with argon for 10 min, then chloro(2-dicyclohexylphosphino-2',4',6'-triisopropyl-1,1'-biphenyl)[2-(2'-amino-1,1'-biphenyl)]palladium(II) (24.18 mg, 0.031 mmol) was added and the resulting mixture heated at 95°C for 1 h. Reaction was quenched with water and the resulting mixture extracted with DCM. The organic phase was washed with saturated aqueous NaCl, passed down a phase separator, and the solvent evaporated *in vacuo.* The resulting crude was purified by flash chromatography on C18-silica by gradient elution with 0-30% B in A (A: water/acetonitrile 95/5+ 0.1% HCOOH, B:acetonitrile/water 95/5 + 0.1% HCOOH). The appropriate fractions were pooled, treated with aqueous 1M HCl and dried under reduced pressure to afford the title compound (32 mg).

LCMS (Method 6): Rt = 0.27 min, m/z 427.2 [M+H]⁺

¹H NMR (400 MHz, DMSO-d6) δ 13.02 - 13.85 (br s, 1H), 9.53 (br s, 1H), 8.83 (d, J=6.6 Hz, 2H), 8.53 - 8.73 (m, 1H), 7.99 - 8.16 (m, 4H), 7.85 - 7.98 (m, 3H), 7.62 - 7.64 (d, 1H), 7.44 - 7.56 (m, 1H), 4.96 (t, J=9.2 Hz, 2H), 4.83 (d, J=6.1 Hz, 2H), 4.73 (d, J=5.7 Hz, 2H), 3.25 (t, J=9.2 Hz, 2H).

### Example 54

The following example was prepared from intermediate E4 using a procedure similar to that used to prepare *example 53.*

| **Ex** | **Structure** | **¹H NMR** | **LC-MS** |
|---|---|---|---|
| 54 | 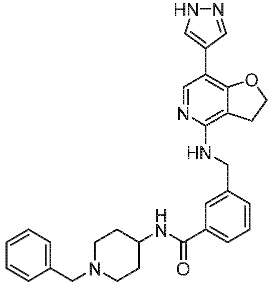 3-(((7-(1H-pyrazol-4-yl)-2,3-dihydrofuro[3,2-c]pyridin-4-yl)amino)methyl)-N-(1-benzylpiperidin-4-yl)benzamide | (400 MHz, DMSO-d6) δ 12.28 - 12.93 (br s, 1H), 8.20 (d, J=7.9 Hz, 1H), 8.14 (s, 1H), 8.07 (s, 1H), 7.85 (s, 2H), 7.80 (s, 1H), 7.65 (d, J=7.5 Hz, 1H), 7.46 (d, J=7.5 Hz, 1H), 7.17 - 7.39 (m, 6H), 6.67 (t, J=5.9 Hz, 1H), 4.68 (t, J=8.8 Hz, 2H), 4.60 (d, J=5.7 Hz, 2H), 3.68 - 3.85 (m, 1H), 3.52 (br s, 2H), 3.04 (t, 2H), 2.85 (d, J=11.0 Hz, 2H), 2.09 (br s, 2H), 1.78 (d, J=10.5 Hz, 2H), 1.50 - 1.68 (m, 2H) | Rt = 3.24 min, m/z 509.1 [M+H]⁺ (Method 13) |

### Example 55 and 56

Example 55 and example 56 were enantiomerically resolved starting from racemic *example 11,* using the following chiral chromatographic conditions: YMC Cellulose-C 5µm, eluents: 30/70 IPA (0.5% DEA)/CO₂, flow 15 mL/min at 120 bar, column temperature 40°C. Two fractions were isolated and characterized as example 55 (enantiomer 1) and example 56 (enantiomer 2).

| **Ex** | **Structure** | **¹H NMR** | **LC-MS** | **Chiral LC** |
|---|---|---|---|---|
| 55 | 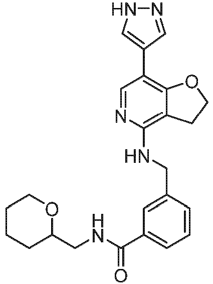 3-(((7-(1H-pyrazol-4-yl)-2,3-dihydrofuro[3,2-c]pyridin-4-yl)amino)methyl)-N-((tetrahydro-2H-pyran-2-yl)methyl)benzamide (*Enantiomer 1*) | (400 MHz, d6-DMSO) δ 12.80 (s, 1H), 8.44 (t, J=5.8 Hz, 1H), 8.09 (s, 1H), 7.85-7.83 (m, 2H), 7.82-7.67 (m, 2H), 7.50-7.46 (m, 1H), 7.37 (t, J=7.7 Hz, 1H), 6.71 (t, J=6.1 Hz, 1H), 4.70 (t, J=8.9 Hz, 2H), 4.62 (d, J=6.0 Hz, 2H), 3.87 (dd, J=2.3, 10.8 Hz, 1H), 3.46 - 3.39 (m, 1H), 3.29 - 3.23 (m, 2H), 3.06 (t, J=8.8 Hz, 2H), 1.80-1.75 (m, 1H), 1.61 (d, J=12.5 Hz, 1H), 1.49-1.41 (m, 3H), 1.22-1.12 (m, 2H). | Rt = 2.68 min, m/z 434.0 [M+H]⁺ (Method 3) | Rt = 8.62 min |
| | | | | Conditions: YMC Cellulose-C 30/70 IPA (0.1% DEA)/CO₂ |
| 56 | 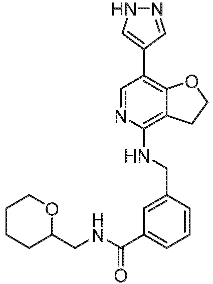 3-(((7-(1H-pyrazol-4-yl)-2,3-dihydrofuro[3,2-c]pyridin-4-yl)amino)methyl)-N-((tetrahydro-2H-pyran-2-yl)methyl)benzamide *(Enantiomer 2)* | (400 MHz, d6-DMSO) δ 12.80 (s, 1H), 8.44 (t, J=5.8 Hz, 1H), 8.09 (s, 1H), 7.85-7.83 (m, 2H), 7.82-7.67 (m, 2H), 7.50-7.46 (m, 1H), 7.37 (t, J=7.7 Hz, 1H), 6.71 (t, J=6.1 Hz, 1H), 4.70 (t, J=8.9 Hz, 2H), 4.62 (d, J=6.0 Hz, 2H), 3.87 (dd, J=2.3, 10.8 Hz, 1H), 3.45 - 3.40 (m, 1H), 3.30 - 3.23 (m, 2H), 3.06 (t, J=8.8 Hz, 2H), 1.80-1.75 (m, 1H), 1.61 (d, J=12.5 Hz, 1H), 1.49-1.41 (m, 3H), 1.22-1.12 (m, 2H). | Rt = 2.68 min, m/z 434.0 [M+H]⁺ (Method 3) | Rt = 10.10 min |
| | | | | Conditions: YMC Cellulose-C 30/70 IPA (0.1% DEA)/CO₂ |

### Example 57

### Step A

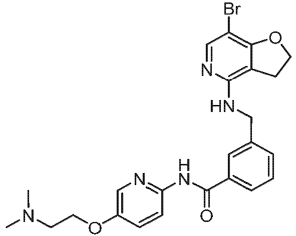

### 3-(((7-Bromo-2,3-dihydrofuro[3,2-c]pyridin-4-yl)amino)methyl)-N-(5-(2-(dimethylamino)ethoxy)pyridin-2-yl)benzamide (Intermediate 57A)

To a solution of Intermediate D1 (400 mg, 1.15 mmol), 5-(2-dimethyl-aminoethoxy)pyridine-2-ylamine (230 mg, 1.26 mmol) and TBTU (440 mg, 1.37 mmol) in DCM (12 mL) was added DIPEA (0.60 mL, 3.44 mmol) . The reaction mixture was stirred at room temperature for 18 h. A further amount of TBTU (369 mg, 1.15 mmol) was added and the mixture was stirred for 48 h. The reaction mixture was diluted with DCM and extracted with water. The organic phase was dried over magnesium sulfate, filtered and concentrated *in vacuo* to obtain the title compound (380 mg).

LCMS (Method 2): Rt = 1.46 min, m/z 512.3/514.3 [M+H]⁺

### Step B

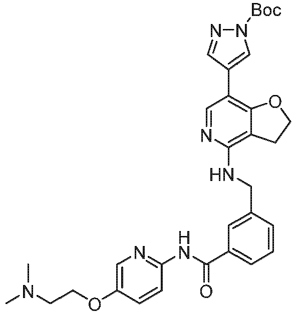

### tert-Butyl 4-(4-((3-((5-(2-(dimethylamino)ethoxy)pyridin-2-yl)carbamoyl)-benzyl)amino)-2,3-dihydrofuro[3,2-c]pyridin-7-yl)-1H-pyrazole-1-carboxylate

### (Intermediate 57B)

To a degassed mixture Intermediate 57A (100 mg, 0.195 mmol), tert-butyl 4-(4,4,5,5-tetramethyl-1,3,2-dioxaborolan-2-yl)pyrazole-1-carboxylate (63 mg, 0.215 mmol) and cesium carbonate (95 mg, 0.293 mmol) in 1,4-dioxane (3 mL) and water (0.3 mL) added [1,1'bis(diphenylphosphino)ferrocene]dichloropalladium(II) complex with dichloro-methane (16 mg, 0.0195 mmol) and the reaction mixture was heated and stirred at 80°C for 1 h. The reaction mixture was cooled, filtered through a pad of Celite^{®} and concentrated. The crude compound was loaded on an Isolute^{®} SCX-2 cartridge and eluted with 2N ammonia in MeOH. The basic fractions were concentrated to afford the title compound (81 mg).

LCMS (Method 2): Rt = 1.50 min, m/z 600.5 [M+H]⁺

### Step C

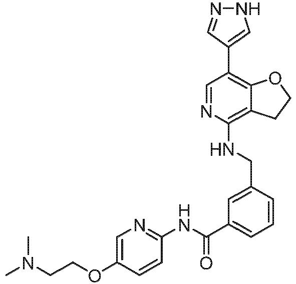

### 3-(((7-(1H-Pyrazol-4-yl)-2,3-dihydrofuro[3,2-c]pyridin-4-yl)amino)methyl)-N-(5-(2-(dimethylamino)ethoxy)pyridin-2-yl)benzamide (Example 57)

To a solution Intermediate 57B (60%, 80 mg, 0.080 mmol) in dichloromethane (2 mL) added trifluoroacetic acid (0.25 mL, 3.20 mmol) and the reaction mixture was stirred at room temperature for 2h. The reaction mixture was concentrated to dryness and submitted for MDAP (Sunfire C18 3x50mm, 3 µm 5-95% ACN / H₂O (10mM NH₄CO₃), 1.7mL/min, RT then Luna Phenyl-Hexyl 21.2x150mm, 10µm 5-60% MeOH / H₂O +0.1% FA, 20mL/min, RT). The desired product was obtained as an off-white solid (15 mg).

LCMS (Method 3): Rt = 1.98 min, m/z 500 [M+H]⁺

¹H NMR (400 MHz, DMSO-d6) δ 10.60 (s, 1H), 8.18 (s, 1H), 8.12 - 8.08 (m, 3H), 7.99 (s, 1H), 7.90 - 7.85 (m, 3H), 7.54 (d, J=7.7 Hz, 1H), 7.50 (dd, J=3.1, 9.2 Hz, 1H), 7.42 (t, J=7.7 Hz, 1H), 6.75 - 6.70 (m, 1H), 4.71 (t, J=8.9 Hz, 2H), 4.66 (d, J=5.9 Hz, 2H), 4.14 (t, J=5.7 Hz, 2H), 3.07 (t, J=8.8 Hz, 2H), 2.66 (t, J=5.8 Hz, 2H), 2.25 (s, 6H).

### Example A

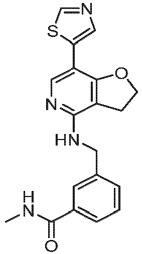

### N-Methyl-3-(((7-(thiazol-5-yl)-2,3-dihydrofuro[3,2-c]pyridin-4-yl)amino)methyl)benzamide (Example A)

A degassed mixture of intermediate E1 (29 mg, 0.08 mmol), 5-(tributylstannyl)thiazole (33 mg, 0.09 mmol), tetrakis(triphenylphosphine)palladium(0) (4.6 mg, 0.004 mmol) and copper(I) thiophene-2-carboxylate (1.5 mg, 0.008 mmol) in dioxane (5 mL) was heated at 150°C under microwave irradiation for 1 h. The reaction mixture, diluted with MeOH, was passed down an Isolute^{®} SCX-2 cartridge eluting with MeOH and then 2M methanolic ammonia. The solution was concentrated in *vacuo* and the residue was purified by MDAP (Sunfire acetonitrile Basic 5-60, Sunfire C18 19x150mm, 10µm, 5-60% acetonitrile/H₂O (10mM NH₄CO₃), 20mL/min, RT) to give the desired product (7.8 mg).

LCMS (Method 3): Rt = 2.29 min, m/z 367.0 [M+H]⁺

¹H NMR (400 MHz, DMSO-d6) δ 8.96 (s, 1H), 8.45-8.35 (m, 1H), 8.16 (s, 1H), 8.15 (d, J=0.7 Hz, 1H), 7.83-7.80 (m, 1H), 7.68-7.66 (m, 1H), 7.49-7.46 (m, 1H), 7.41-7.36 (m, 1H), 7.11-7.06 (m, 1H), 4.77 (t, J=8.9 Hz, 2H), 4.65 (d, J=6.0 Hz, 2H), 3.10 (t, J=8.9 Hz, 2H), 2.78 (d, J=4.5 Hz, 3H).

### Example B

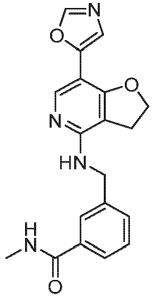

### N-Methyl-3-(((7-(oxazol-5-yl)-2,3-dihydrofuro[3,2-c]pyridin-4-yl)amino)methyl)benzamide (Example B)

A mixture of intermediate E1 (75 mg, 0.21 mmol), 5-(4,4,5,5-tetramethyl-1,3,2-dioxaborolan-2-yl)oxazole (48 mg, 0.25 mmol), tetrakis(triphenylphosphine)palladium(0) (24 mg, 0.021 mmol) and cesium carbonate (0.20 g, 0.62 mmol) in DMF (4 mL) and water (0.4 mL) was degassed with argon and heated at 100°C for 18 h. The reaction mixture was filtered through Celite^{®} and concentrated *in vacuo* and the residue was purified by MDAP (Luna Phenyl-Hexyl 3x50mm, 3µm 5-95% MeOH/H₂O (0.1% FA), 1.7 mL/min, RT) to give the product (13.4 mg).

LCMS (Method 3): Rt = 2.16 min, m/z 351.0 [M+H]⁺

¹H NMR (400 MHz, DMSO-d6) δ 8.43-8.38 (m, 1H), 8.33 (s, 1H), 8.13 (s, 1H), 7.82 (s, 1H), 7.68 (ddd, J=1.5, 1.5, 8.0 Hz, 1H), 7.47 (d, J=7.9 Hz, 1H), 7.39 (t, J=7.7 Hz, 1H), 7.18 (s, 1H,), 7.12 (t, J=6.1 Hz, 1H), 4.78 (t, J=9.0 Hz, 2H), 4.67 (d, J=6.0 Hz, 2H), 3.09 (t, J=8.9 Hz, 2H), 2.78 (d, J=4.5 Hz, 3H).

### Example C

### Step A

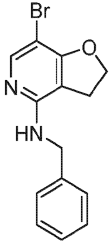

### N-Benzyl-7-bromo-2,3-dihydrofuro[3,2-c]pyridin-4-amine (Intermediate CA)

Intermediate CA was prepared using a procedure similar to that used for the synthesis of Intermediate C1 by replacing methyl 3-formylbenzoate with benzaldehyde.

LCMS (Method 16): Rt = 1.57 min, m/z 305.1/307.1 [M+H]⁺

### Step B

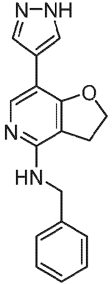

### N-Benzyl-7-(1H-pyrazol-4-yl)-2,3-dihydrofuro[3,2-c]pyridin-4-amine (Example C)

A degassed mixture of Intermediate CA (50 mg, 0.164 mmol), tert-butyl 4-(4,4,5,5-tetramethyl-1,3,2-dioxaborolan-2-yl)pyrazole-1-carboxylate (58 mg, 0.197 mmol), [1,1'-bis(diphenylphosphino)-ferrocene]dichloropalladium(II) complex with DCM, (14 mg, 0.0164 mmol), cesium carbonate (53 mg, 0.164 mmol) in 1,4-dioxane (1.5 mL) and water (0.15 mL) was heated at 90°C for 20 h. A further portion of tert-butyl 4-(4,4,5,5-tetramethyl-1,3,2-dioxaborolan-2-yl)pyrazole-1-carboxylate (58 mg, 0.197 mmol), [1,1'-bis(diphenylphosphino)-ferrocene]dichloropalladium(II) complex with dichloromethane, (14 mg, 0.0164 mmol), and cesium carbonate (53 mg, 0.164 mmol) were added and the resulting mixture was allowed to stir for a further 2 h.. The reaction mixture was loaded onto an Isolute^{®} SCX-2 cartridge and washed with DCM, then MeOH, then the product eluted with MeOH/NH₃ 2N and concentrated *in vacuo.* The residue was purified by MDAP (Sunfire C18 19x150mm, 10µm 20-80% ACN / H₂O (10mM NH₄CO₃), 20mL/min, RT) to give the product (10.74 mg).

LCMS (Method 3) RT = 2.59 min, m/z 293.3 [MH]⁺

¹H NMR (400 MHz, DMSO-d6) δ 12.79 (s, 1H), 8.09 (s, 1H), 7.91 (s, 1H), 7.83 (s, 1H), 7.36 - 7.27 (m, 4H), 7.20 (tt, J=1.7, 7.1 Hz, 1H), 6.67 - 6.62 (m, 1H), 4.70 (t, J=8.9 Hz, 2H), 4.59 (d, J=6.0 Hz, 2H), 3.05 (t, J=8.9 Hz, 2H).

### PHARMACOLOGICAL ACTIVITY OF THE COMPOUNDS OF THE INVENTION.

### In vitro inhibitory activity assay description ROCK1 and ROCK2

### (Method A)

The effectiveness of compounds of the present invention to inhibit Rho kinase activity can be determined in a 10µl assay containing 40mM Tris pH7.5, 20mM MgCl₂ 0.1mg/mL BSA, 50µM DTT and 2.5µM peptide substrate (Myelin Basic Protein) using an ADP-Glo kit (Promega). Compounds were dissolved in DMSO such that the final concentration of DMSO was 1% in the assay. All reactions/incubations are performed at 25°C. Compound (2uL) and either Rho kinase 1 or 2 (4µl) were mixed and incubated for 30 min. Reactions were initiated by addition of ATP (4µL) such that the final concentration of ATP in the assay was 10µM. After a 1hour incubation 10µl of ADP-Glo Reagent was added and after a further 45 minutes incubation 20uL of Kinase Detection Buffer was added and the mixture incubated for a further 30 minutes. The luminescent signal was measured on a luminometer. Controls consisted of assay wells that did not contain compound with background determined using assay wells with no enzyme added. Compounds were tested in dose-response format and the inhibition of kinase activity was calculated at each concentration of compound. To determine the IC₅₀ (concentration of compound required to inhibit 50% of the enzyme activity) data were fit to a plot of % inhibition vs Log₁₀ compound concentration using a sigmoidal fit with a variable slope and fixing the maximum to 100% and the minimum to 0%. To determine the Ki values the Cheng-Prusoff equation was utilized (Ki=IC₅₀/(1+[S]/Km).

### In vitro inhibitory activity assay description ROCK1 and ROCK2

### (Method B)

The effectiveness of compounds of the present invention to inhibit Rho kinase activity can be determined in a 10µl assay containing 40mM Tris pH7.5, 20mM MgCl₂ 0.1mg/mL BSA, 50µM DTT and 2.5µM peptide substrate (Myelin Basic Protein) using an ADP-Glo kit (Promega). Compounds were dissolved in DMSO such that the final concentration of DMSO was 1% in the assay. All reactions/incubations are performed at 25oC. Compound (2µL) and either Rho kinase 1 or 2 (4µl) were mixed and incubated for 30 min. Reactions were initiated by addition of ATP (4µL) such that the final concentration of ATP in the assay was 200µM. After a 1hour incubation 10µl of ADP-Glo Reagent was added and after a further 45 minute incubation 20µL of Kinase Detection Buffer was added and the mixture incubated for a further 30 minutes. The luminescent signal was measured on a luminometer. Controls consisted of assay wells that did not contain compound with background determined using assay wells with no enzyme added. Compounds were tested in dose-response format and the inhibition of kinase activity was calculated at each concentration of compound. To determine the IC₅₀ (concentration of compound required to inhibit 50% of the enzyme activity) data were fit to a plot of % inhibition vs Log10 compound concentration using a sigmoidal fit with a variable slope and fixing the maximum to 100% and the minimum to 0%. To determine the Ki values the Cheng-Prusoff equation was utilized (Ki=IC₅₀/(1+[S]/Km).

The Ki values obtained with Method A and with Method B were consistent.

Compounds according to the invention showed Ki values lower that 500 nM on both isoforms.

The results for individual compounds of the examples are provided below in Table 1 and are expressed as range of activity.

### In vitro inhibitory activity assay description for PKA

The effectiveness of compounds of the present invention to inhibit PKA activity can be determined in a 10µL assay containing 40mM Tris pH7.5, 20mM MgCl₂ 0.1mg/mL BSA, 50µM DTT and 260µM peptide substrate (kemptide) using an ADP-Glo kit (Promega). Compounds were dissolved in DMSO such that the final concentration of DMSO was 1% in the assay. All reactions/incubations are performed at 25°C. Compound and PKA enzyme (6µl) were mixed and incubated for 30 min. Reactions were initiated by addition of ATP (4µL) such that the final concentration of ATP in the assay was 10µM. After a 30 minute incubation 10µL of ADP-Glo Reagent was added and after a further 1 hour incubation 20µL of Kinase Detection Buffer was added and the mixture incubated for a further 45 minutes. The luminescent signal was measured on a luminometer. Controls consisted of assay wells that did not contain compound with background determined using assay wells with no enzyme added. Compounds were tested in dose-response format and the inhibition of kinase activity was calculated at each concentration of compound. To determine the IC₅₀ (concentration of compound required to inhibit 50% of the enzyme activity) data were fit to a plot of % inhibition vs Log₁₀ compound concentration using a sigmoidal fit with a variable slope and fixing the maximum to 100% and the minimum to 0%. To determine the Ki values the Cheng-Prusoff equation was utilized (Ki=IC₅₀/(1+[S]/Km).

In vitro inhibitory activities for PKA were reported as selectivity ratio vs. ROCK2. Selectivity ratio PKA/ROCK2 was calculated by dividing the Ki value for PKA by Ki value of ROCK2 (method B) and reported into table 1.

**Table 1.**

| **Ex No.** | **Method A** | | **Method B** | | **Ratio Ki PKA/ROCK2** |
|---|---|---|---|---|---|
| | ROCK 1 | ROCK 2 | ROCK 1 | ROCK 2 | |
| 1 | | | +++ | +++ | *** |
| 2 | | | ++ | +++ | *** |
| 3 | | | ++ | ++ | *** |
| 4 | | | ++ | ++ | |
| 5 | | | ++ | ++ | *** |
| 6 | | | ++ | ++ | *** |
| 7 | | | ++ | +++ | |
| 8 | | | ++ | +++ | *** |
| 9 | | | ++ | ++ | |
| 10 | | | +++ | +++ | *** |
| 11 | | | +++ | +++ | *** |
| 12 | | | ++ | ++ | *** |
| 13 | | | ++ | +++ | |
| 14 | | | +++ | +++ | |
| 15 | +++ | +++ | +++ | +++ | *** |
| 16 | +++ | +++ | +++ | +++ | *** |
| 17 | +++ | +++ | | | |
| 18 | +++ | +++ | | | |
| 19 | +++ | +++ | | | |
| 20 | +++ | +++ | | | |
| 21 | +++ | +++ | | | |
| 22 | +++ | +++ | | | |
| 23 | +++ | +++ | | | |
| 24 | +++ | +++ | | | |
| 25 | +++ | +++ | | | |
| 26 | +++ | +++ | | | |
| 27 | +++ | +++ | | | |
| 28 | +++ | +++ | +++ | +++ | *** |
| 29 | ++ | +++ | | | |
| 30 | +++ | +++ | | | |
| 31 | | | +++ | +++ | *** |
| 32 | | | +++ | +++ | *** |
| 33 | +++ | +++ | +++ | +++ | *** |
| 34 | +++ | +++ | +++ | +++ | *** |
| 35 | +++ | +++ | | | |
| 36 | +++ | +++ | | | |
| 37 | +++ | +++ | | | |
| 38 | +++ | +++ | | | |
| 39 | +++ | +++ | | | |
| 40 | +++ | +++ | | | |
| 41 | + | ++ | | | |
| 42 | ++ | +++ | | | |
| 43 | +++ | +++ | +++ | +++ | |
| 44 | +++ | +++ | +++ | +++ | |
| 45 | +++ | +++ | +++ | +++ | |
| 46 | +++ | +++ | +++ | +++ | |
| 47 | +++ | +++ | | | |
| 48 | +++ | +++ | +++ | +++ | |
| 49 | +++ | +++ | | | |
| 50 | ++ | ++ | | | |
| 51 | +++ | +++ | | | |
| 52 | ++ | ++ | | | |
| 53 | +++ | +++ | | | |
| 54 | +++ | +++ | | | |
| 55 | | | +++ | +++ | *** |
| 56 | | | +++ | +++ | *** |
| 57 | | | +++ | +++ | *** |
| A | | | + | + | |
| B | | | + | ++ | *** |
| C | | | + | + | * |

wherein the compounds are classified in term of potency with respect to their inhibitory activity on ROCK1 and ROCK2 isoforms according to the following classification criterion:
+ + + : Ki ≤ 3 nM
+ + : 3 < Ki ≤ 30 nM
+ : Ki > 30 nM

The Compounds according to the invention showed advantageously Ki values equal to or lower than 30 nM , preferably even equal to or lower that 3 nM, at least on ROCK2; further preferably lower than 30 nM, preferably even equal to or lower that 3 nM, on both isoforms. . The compounds according to the invention are more potent than the *comparative example A and B.*

Moreover, preferred compounds according to the invention exhibit marked selectivity versus PKA. The compounds according to the invention are at least 5 fold, preferably equal to or more than 10 fold, selective in terms of ROCK2 selectivity vs PKA. Overall the compounds of the invention are more selective than the *comparative example C*.

The compounds are classified in term of selectivity with respect to their ratio of inhibitory activity (Ki) of PKA on ROCK2 isoform according to the following classification criterion:
*** : ratio ≥ 10
** : 5 < ratio < 10
* : ratio ≤ 5

## Claims

1. A compound of formula (I) 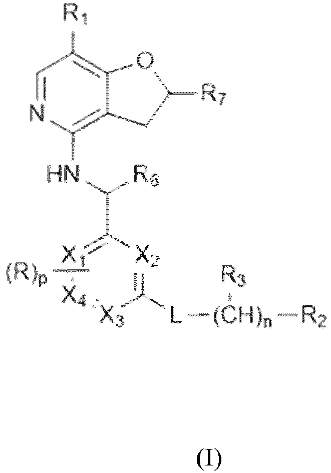 wherein
**X₁**, **X₂**, **X₃** and **X₄** are all CH or one of X₁, X₂, X₃ and X₄ is N and the others are CH;
p is zero or an integer from 1 to 4;
each R, when present, is halogen in each occurrence independently selected from (C₁-C₆)alkyl and halogen selected from F, Cl, Br and I;
R₁ is pyrazolyl;
L is -C(O)NH- or -NHC(O)- ;
n is in each occurrence independently 0 or an integer selected from 1, 2 or 3;
R₂ and R₃ are in each occurrence independently selected from the group consisting of
-H,
halogen,
-OH,
-(CH₂)ₘNR₄R₅,
(C₁-C₆)alkyl,
(C₁-C₆)hydroxyalkyl,
(C₁-C₆) alkoxy,
(C₁-C₆) alkoxy (C₁-C₆)alkyl,
(C₁-C₆)haloalkyl,
(C₁-C₆)haloalkoxy,
(C₁-C₆)haloalkoxy (C₁-C₆)alkyl,
(C₃-C₁₀)cycloalkyl,
aryl, heteroaryl and (C₃-C₆)heterocycloalkyl,
each of which cycloalkyl, aryl, heteroaryl and heterocycloalkyl
is in its turn optionally and independently substituted with one or more groups selected from
halogen,
-OH,
(C₁-C₆)alkyl,
(C₁-C₆)hydroxyalkyl,
(C₁-C₆) alkoxy,
(C₁-C₆) alkoxy (C₁-C₆)alkyl,
(C₁-C₆)haloalkyl,
(C₁-C₆)haloalkoxy,
-(CH₂)ₘNR₄R₅,
-O-(CH₂)ₘNR₄R₅,
alkanoyl,
aryl, heteroaryl, cycloalkyl,
aryl-(C₁-C₆)alkyl,
(C₃-C₆)heterocycloalkyl,
(C₃-C₈)heterocycloalkyl-(C₁-C₆)alkyl,
each of said aryl, heteroaryl, cycloalkyl, heterocycloalkyl is still further optionally substituted by one or more group selected independently from halogen, -OH, (C₁-C₈)alkyl, (C₁-C₆)haloalkyl, (C₁-C₆)hydroxyalkyl;
m is in each occurrence independently 0 or an integer selected from 1, 2 or 3;
R₄ and R₅, the same or different, are selected from the group consisting of -H,
(C₁-C₆)alkyl,
(C₁-C₆)haloalkyl,
(C₁-C₆)hydroxyalkyl,
(C₃-C₆)heterocycloalkyl;
R₆ and R7 are independently selected from the group consisting of -H, (C₁-C₆)alkyl;
wherein, where not already specified, aryl refers to mono, bi- or tri-cyclic ring systems which have 6 to 20 ring atoms, in which at least one ring is aromatic; heteroaryl refers to mono-, bi- or tri-cyclic ring systems with 5 to 20 ring atoms, in which at least one ring is aromatic and in which at least one ring atom is a heteroatom selected from N, S or O; (C₃-C₆)heterocycloalkyl refers to saturated or partially unsaturated monocyclic (C₃-C₆)cycloalkyl groups in which at least one ring carbon atom is replaced by at least one heteroatom selected from N, NH, S or O and/or may bear an oxo substituent group;
single enantiomers, diastereoisomers and mixtures thereof in any proportion and/or pharmaceutically acceptable salts and solvates thereof.

2. A compound according to Claim 1, wherein X₃ and X₄ are all CH groups and X₁ or X₂ are in the alternative independently a CH group or a nitrogen atom ;
R₁ is pyrazol-4-yl;
all the other variables being as defined in claim 1;
single enantiomers, diastereoisomers and mixtures thereof in any proportion and/or pharmaceutically acceptable salts and solvates thereof.

3. A compound according to Claim 2, wherein X₁ , X₂, X₃ , X₄ are all CH group;
each R, when present, is halogen in each occurrence independently selected from F, Cl, Br and I;
R₁ is pyrazolyl;
L is -C(O)NH- or -NHC(O)- ;
n is 0;
R₂ is in each occurrence independently selected from the group consisting of (C₁-C₆)alkyl,
(C₁-C₆)hydroxyalkyl,
(C₁-C₆) alkoxy (C₁-C₆)alkyl,
(C₁-C₆)haloalkyl,
(C₁-C₆)haloalkoxy (C₁-C₆)alkyl;
all the other variables being as defined in claim 1,
single enantiomers, diastereoisomers and mixtures thereof in any proportion and/or pharmaceutically acceptable salts and solvates thereof.

4. A compound according to Claim 1, wherein **X₁**, **X₂**, **X₃** and **X₄** are all CH;
p is zero or an integer from 1 to 4;
each R, when present, is halogen in each occurrence independently selected from F, Cl, Br and I;
R₁ is pyrazolyl;
L is -C(O)NH or-NHC(O)- ;
n is in each occurrence independently 0 or an integer selected from 1, 2 or 3;
R₃ when present is H, or (C₁-C₆)hydroxyalkyl, and
R₂ is selected from the group consisting of
aryl, heteroaryl and (C₃-C₆)heterocycloalkyl,
each of which aryl, heteroaryl and heterocycloalkyl
is in its turn optionally and independently substituted with one or more groups selected from
(C₁-C₆)alkyl,
(C₁-C₆)hydroxyalkyl,
(C₁-C₆) alkoxy,
(C₁-C₆) alkoxy (C₁-C₆)alkyl,
-(CH₂)ₘNR₄R₅,
-O-(CH₂)ₘNR₄R₅,
aryl, heteroaryl, cycloalkyl,
aryl-(C₁-C₆)alkyl,
(C₃-C₆)heterocycloalkyl,
(C₃-C₈)heterocycloalkyl-(C₁-C₆)alkyl,
each of said aryl, heteroaryl, cycloalkyl, heterocycloalkyl is still further optionally substituted by one or more group selected independently from halogen, -OH, (C₁-C₈)alkyl, (C₁-C₆)haloalkyl, (C₁-C₆)hydroxyalkyl;
m is in each occurrence independently 0 or an integer selected from 1, 2 or 3;
R₄ and R₅, the same or different, are selected from the group consisting of
-H,
(C₁-C₆)alkyl,
(C₁-C₆)haloalkyl,
(C₁-C₆)hydroxyalkyl,
(C₃-C₆)heterocycloalkyl;
or pharmaceutically acceptable salts and solvates thereof.

5. A compound according to Claim 4, wherein R₂ is selected from (pyridinyl)methyl, (pyridinyl)ethyl, methoxypyridinyl, ((dimethylamino)ethoxy)pyridinyl, or from 1-(2-(dimethylamino)ethyl)-1H-indazole-5-yl, 1-(2-morpholinoethyl)-1H-indazole-5-yl, 1-(1-methylpiperidin-4-yl)-1H-indazole-5-yl.

6. A compound of formula (I) according to Claim 1, wherein **X₁, X₂, X₃** and **X₄** are all CH;
p is zero or 1;
each R, when present, is F;
R₁ is **pyrazol 4-yl;**
L is -C(O)NH- or -NHC(O)- ;
n is in each occurrence independently 0 or an integer selected from 1, 2;
R₂ and R₃ are in each occurrence independently selected from the group consisting of
-H,
(C₁-C₆)alkyl which is methyl,
(C₁-C₆) alkoxy (C₁-C₆)alkyl which is 2-methoxyethyl,
(C₁-C₆)haloalkyl which is 3-fluoropropyl,
(C₃-C₁₀)cycloalkyl which is cyclopropyl, cyclobutyl,
aryl which is phenyl;
heteroaryl which is pyridinyl, pyrazolyl, thiophenyl, imidazolyl, oxazolyl, isoxazolyl, thiazolyl, pyrimidinyl, 1,2,5-oxadiazol-3-yl, 4,5,6,7-tetrahydropyrazolo[1,5-a]pyrazin-2-yl, 4,5,6,7-tetrahydrothiazolo[5,4-c]pyridin-2-yl, 4,5,6,7-tetrahydrobenzo[d]thiazol-2-yl, 1,2,3,4-tetrahydroisoquinoline-6yl, 5,6,7,8-tetrahydro-1,7-naphthyridinyl, 1H-indole-5yl, isoindolin-yl, 1H-indazole-5yl,
and
(C₃-C₆)heterocycloalkyl which is piperidinyl, morpholinyl, tetrahydrofuranyl, tetrahydro-2H-pyran-yl;
each of which cycloalkyl, aryl, heteroaryl and heterocycloalkyl
is in its turn optionally and independently substituted with one or more groups selected from
halogen which is Fluoro,
(C₁-C₆)alkyl which is methyl,
(C₁-C₆)hydroxyalkyl, which is hydroxyethyl,
(C₁-C₆) alkoxy, which is methoxy,
-(CH₂)ₘNR₄R₅ which is (dimethylamino)methyl, 2-(dimethylamino)ethyl, dimethylamino wherein R₄ and R₅ are methyl and m is 0, 1 or 2, oxetan-3-ylamino wherein R₄ is H, R₅ is oxetanyl and m is 0,
-O-(CH₂)ₘNR₄R₅ which is ((dimethylamino)ethoxy)pyridinyl;
cycloalkyl which is cyclopropyl,
aryl-(C₁-C₆)alkyl, which is benzyl, phenethyl,
(C₃-C₆)heterocycloalkyl, which is oxetan-3-yl, piperidin-4-yl,
(C₃-C₈)heterocycloalkyl-(C₁-C₆)alkyl, which is morpholinoethyl, pyrrolidin-1-ylmethyl,
each of said heterocycloalkyl is still further optionally substituted by (C₁-C₈)alkyl which is methyl;
R₆ is -H, or methyl; and R₇ is -H
single enantiomers, diastereoisomers and mixtures thereof in any proportion and/or pharmaceutically acceptable salts and solvates thereof.

7. A compound according to claim 1 selected from:
3-(((7-(1H-pyrazol-4-yl)-2,3-dihydrofuro[3,2-c]pyridin-4-yl)amino)methyl)-N-(1-cyclopropylpiperidin-4-yl)benzamide;
3-(((7-(1H-pyrazol-4-yl)-2,3-dihydrofuro[3,2-c]pyridin-4-yl)amino)methyl)-N-((1-methyl-1H-pyrazol-3-yl)methyl)benzamide;
3-(((7-(1H-pyrazol-4-yl)-2,3-dihydrofuro[3,2-c]pyridin-4-yl)amino)methyl)-N-(3-fluoropropyl)benzamide;
3-(((7-(1H-pyrazol-4-yl)-2,3-dihydrofuro[3,2-c]pyridin-4-yl)amino)methyl)-N-(pyridin-2-ylmethyl)benzamide;
3-(((7-(1H-pyrazol-4-yl)-2,3-dihydrofuro[3,2-c]pyridin-4-yl)amino)methyl)-N-(cyclopropylmethyl)benzamide;
3-(((7-(1H-pyrazol-4-yl)-2,3-dihydrofuro[3,2-c]pyridin-4-yl)amino)methyl)-N-(5,5-dimethyltetrahydrofuran-3-yl)benzamide;
3-(((7-(1H-pyrazol-4-yl)-2,3-dihydrofuro[3,2-c]pyridin-4-yl)amino)methyl)-N-(2-methoxyethyl)benzamide;
3-(((7-(1H-pyrazol-4-yl)-2,3-dihydrofuro[3,2-c]pyridin-4-yl)amino)methyl)-N-((1-methyl-1H-imidazol-4-yl)methyl)benzamide;
3-(((7-(1H-pyrazol-4-yl)-2,3-dihydrofuro[3,2-c]pyridin-4-yl)amino)methyl)-N-(pyrimidin-5-ylmethyl)benzamide;
N-((1,2,5-oxadiazol-3-yl)methyl)-3-(((7-(1H-pyrazol-4-yl)-2,3-dihydrofuro[3,2-c]pyridin-4-yl)amino)methyl)benzamide;
3-(((7-(1H-pyrazol-4-yl)-2,3-dihydrofuro[3,2-c]pyridin-4-yl)amino)methyl)-N-((tetrahydro-2H-pyran-2-yl)methyl)benzamide;
3-(((7-(1H-pyrazol-4-yl)-2,3-dihydrofuro[3,2-c]pyridin-4-yl)amino)methyl)-N-(3,3-difluorocyclobutyl)benzamide;
3-(((7-(1H-pyrazol-4-yl)-2,3-dihydrofuro[3,2-c]pyridin-4-yl)amino)methyl)-N-(isoxazol-3-ylmethyl)benzamide;
3-(((7-(1H-pyrazol-4-yl)-2,3-dihydrofuro[3,2-c]pyridin-4-yl)amino)methyl)-N-(thiazol-4-ylmethyl)benzamide;
3-(((7-(1H-pyrazol-4-yl)-2,3-dihydrofuro[3,2-c]pyridin-4-yl)amino)methyl)-N-(5-methyl-4,5,6,7-tetrahydropyrazolo[1,5-a]pyrazin-2-yl)benzamide;
3-(((7-(1H-pyrazol-4-yl)-2,3-dihydrofuro[3,2-c]pyridin-4-yl)amino)methyl)-N-(2-(1-methylpiperidin-4-yl)ethyl)benzamide;
3-(((7-(1H-pyrazol-4-yl)-2,3-dihydrofuro[3,2-c]pyridin-4-yl)amino)methyl)-N-(2-(pyridin-4-yl)ethyl)benzamide;
3-(((7-(1H-pyrazol-4-yl)-2,3-dihydrofuro[3,2-c]pyridin-4-yl)amino)methyl)-N-(3-((dimethylamino)methyl)phenyl)benzamide;
3-(((7-(1H-pyrazol-4-yl)-2,3-dihydrofuro[3,2-c]pyridin-4-yl)amino)methyl)-N-(3-((dimethylamino)methyl)benzyl)benzamide;
3-(((7-(1H-pyrazol-4-yl)-2,3-dihydrofuro[3,2-c]pyridin-4-yl)amino)methyl)-N-(2-morpholinoethyl)benzamide;
3-(((7-(1H-pyrazol-4-yl)-2,3-dihydrofuro[3,2-c]pyridin-4-yl)amino)methyl)-N-(5-methyl-4,5,6,7-tetrahydrothiazolo[5,4-c]pyridin-2-yl)benzamide;
3-(((7-(1H-pyrazol-4-yl)-2,3-dihydrofuro[3,2-c]pyridin-4-yl)amino)methyl)-N-(2-(pyridin-3-yl)ethyl)benzamide;
(S)-3-(((7-(1H-pyrazol-4-yl)-2,3-dihydrofuro[3,2-c]pyridin-4-yl)amino)methyl)-N-(2-hydroxy-1-phenylethyl)benzamide;
3-(((7-(1H-pyrazol-4-yl)-2,3-dihydrofuro[3,2-c]pyridin-4-yl)amino)methyl)-N-(4-((dimethylamino)methyl)benzyl)benzamide;
3-(((7-(1H-pyrazol-4-yl)-2,3-dihydrofuro[3,2-c]pyridin-4-yl)amino)methyl)-N-((5-methylthiophen-2-yl)methyl)benzamide;
3-(((7-(1H-pyrazol-4-yl)-2,3-dihydrofuro[3,2-c]pyridin-4-yl)amino)methyl)-N-(1-phenethylpiperidin-4-yl)benzamide;
3-(((7-(1H-pyrazol-4-yl)-2,3-dihydrofuro[3,2-c]pyridin-4-yl)amino)methyl)-N-(1-(2-(dimethylamino)ethyl)-1H-pyrazol-4-yl)benzamide;
3-(((7-(1H-pyrazol-4-yl)-2,3-dihydrofuro[3,2-c]pyridin-4-yl)amino)methyl)-N-(2-(1-(oxetan-3-yl)piperidin-4-yl)ethyl)benzamide;
3-(((7-(1H-pyrazol-4-yl)-2,3-dihydrofuro[3,2-c]pyridin-4-yl)amino)methyl)-N-((5-methyl-4,5,6,7-tetrahydrothiazolo[5,4-c]pyridin-2-yl)methyl)benzamide;
3-(((7-(1H-pyrazol-4-yl)-2,3-dihydrofuro[3,2-c]pyridin-4-yl)amino)methyl)-N-((2-methylisoindolin-5-yl)methyl)benzamide;
3-(((7-(1H-pyrazol-4-yl)-2,3-dihydrofuro[3,2-c]pyridin-4-yl)amino)methyl)-N-(5-methoxypyridin-2-yl)benzamide;
3-(((7-(1H-pyrazol-4-yl)-2,3-dihydrofuro[3,2-c]pyridin-4-yl)amino)methyl)-N-(pyrimidin-4-yl)benzamide;
3-(((7-(1H-pyrazol-4-yl)-2,3-dihydrofuro[3,2-c]pyridin-4-yl)amino)methyl)-N-(6-(dimethylamino)-4,5,6,7-tetrahydrobenzo[d]thiazol-2-yl)benzamide;
3-(((7-(1H-pyrazol-4-yl)-2,3-dihydrofuro[3,2-c]pyridin-4-yl)amino)methyl)-N-(6-(oxetan-3-ylamino)-4,5,6,7-tetrahydrobenzo[d]thiazol-2-yl)benzamide;
N-(3-(((7-(1H-pyrazol-4-yl)-2,3-dihydrofuro[3,2-c]pyridin-4-yl)amino)methyl)phenyl)-4-((dimethylamino)methyl)benzamide;
N-(3-(((7-(1H-pyrazol-4-yl)-2,3-dihydrofuro[3,2-c]pyridin-4-yl)amino)methyl)phenyl)-5-methyl-4,5,6,7-tetrahydrothiazolo[4,5-c]pyridine-2-carboxamide;
N-(3-(((7-(1H-pyrazol-4-yl)-2,3-dihydrofuro[3,2-c]pyridin-4-yl)amino)methyl)phenyl)-5-methyl-4,5,6,7-tetrahydrothiazolo[5,4-c]pyridine-2-carboxamide;
N-(3-(((7-(1H-pyrazol-4-yl)-2,3-dihydrofuro[3,2-c]pyridin-4-yl)amino)methyl)phenyl)-5-(2-hydroxyethyl)-4,5,6,7-tetrahydrothiazolo[5,4-c]pyridine-2-carboxamide;
N-(3-(((7-(1H-pyrazol-4-yl)-2,3-dihydrofuro[3,2-c]pyridin-4-yl)amino)methyl)phenyl)-1-methyl-1H-indazole-5-carboxamide;
N-(3-(((7-(1H-pyrazol-4-yl)-2,3-dihydrofuro[3,2-c]pyridin-4-yl)amino)methyl)phenyl)-1,2,3,4-tetrahydroisoquinoline-6-carboxamide;
N-(3-(((7-(1H-pyrazol-4-yl)-2,3-dihydrofuro[3,2-c]pyridin-4-yl)amino)methyl)phenyl)-1-methyl-1H-indazole-3-carboxamide;
N-(3-(((7-(1H-pyrazol-4-yl)-2,3-dihydrofuro[3,2-c]pyridin-4-yl)amino)methyl)phenyl)-1-methyl-1H-indazole-4-carboxamide;
N-(3-(((7-(1H-pyrazol-4-yl)-2,3-dihydrofuro[3,2-c]pyridin-4-yl)amino)methyl)phenyl)-1-(2-(dimethylamino)ethyl)-1H-indazole-5-carboxamide;
N-(3-(((7-(1H-pyrazol-4-yl)-2,3-dihydrofuro[3,2-c]pyridin-4-yl)amino)methyl)phenyl)-1-(1-methylpiperidin-4-yl)-1H-indazole-5-carboxamide;
N-(3-(((7-(1H-pyrazol-4-yl)-2,3-dihydrofuro[3,2-c]pyridin-4-yl)amino)methyl)phenyl)-2-methyl-1,2,3,4-tetrahydroisoquinoline-6-carboxamide;
N-(3-(((7-(1H-pyrazol-4-yl)-2,3-dihydrofuro[3,2-c]pyridin-4-yl)amino)methyl)phenyl)-1-(2-morpholinoethyl)-1H-indazole-5-carboxamide;
N-(5-(((7-(1H-pyrazol-4-yl)-2,3-dihydrofuro[3,2-c]pyridin-4-yl)amino)methyl)-2-fluorophenyl)-1,2,3,4-tetrahydroisoquinoline-6-carboxamide;
N-(3-(((7-(1H-pyrazol-4-yl)-2,3-dihydrofuro[3,2-c]pyridin-4-yl)amino)methyl)phenyl)-5-(pyrrolidin-1-ylmethyl)thiazole-2-carboxamide;
N-(3-(((7-(1H-pyrazol-4-yl)-2,3-dihydrofuro[3,2-c]pyridin-4-yl)amino)methyl)phenyl)-3-((dimethylamino)methyl)benzamide;
N-(3-(((7-(1H-pyrazol-4-yl)-2,3-dihydrofuro[3,2-c]pyridin-4-yl)amino)methyl)phenyl)-2-phenylacetamide;
N-(3-(((7-(1H-pyrazol-4-yl)-2,3-dihydrofuro[3,2-c]pyridin-4-yl)amino)methyl)phenyl)-7-methyl-5,6,7,8-tetrahydro-1,7-naphthyridine-3-carboxamide;
N-(3-(1-((7-(1H-pyrazol-4-yl)-2,3-dihydrofuro[3,2-c]pyridin-4-yl)amino)ethyl)phenyl)acetamide;
3-(((7-(1H-pyrazol-4-yl)-2,3-dihydrofuro[3,2-c]pyridin-4-yl)amino)methyl)-N-(pyridin-4-ylmethyl)benzamide;
3-(((7-(1H-pyrazol-4-yl)-2,3-dihydrofuro[3,2-c]pyridin-4-yl)amino)methyl)-N-(1-benzylpiperidin-4-yl)benzamide;
3-(((7-(1H-pyrazol-4-yl)-2,3-dihydrofuro[3,2-c]pyridin-4-yl)amino)methyl)-N-((tetrahydro-2H-pyran-2-yl)methyl)benzamide (Enantiomer 1);
3-(((7-(1H-pyrazol-4-yl)-2,3-dihydrofuro[3,2-c]pyridin-4-yl)amino)methyl)-N-((tetrahydro-2H-pyran-2-yl)methyl)benzamide (Enantiomer 2);
3-(((7-(1H-pyrazol-4-yl)-2,3-dihydrofuro[3,2-c]pyridin-4-yl)amino)methyl)-N-(5-(2-(dimethylamino)ethoxy)pyridin-2-yl)benzamide;
single enantiomers, diastereoisomers and mixtures thereof in any proportion and/or pharmaceutically acceptable salts and solvates thereof.

8. A pharmaceutical composition comprising a compound as defined in any one of claims 1 to 7, or a pharmaceutically acceptable salt or solvate thereof, in admixture with one or more pharmaceutically acceptable carrier or excipient.

9. A pharmaceutical composition according to claim 8 suitable to be administered by inhalation, selected from inhalable powders, propellant-containing metering aerosols or propellant-free inhalable formulations.

10. A device comprising the pharmaceutical composition according to claim 9, which may be a single- or multi-dose dry powder inhaler, a metered dose inhaler and a soft mist nebulizer.

11. A pharmaceutical composition according to claim 8 suitable to be administered by oral route, selected from, gelcaps, capsules, caplets, granules, lozenges and bulk powders or aqueous and non-aqueous solutions, emulsions, suspensions, syrups, and elixirs formulations.

12. A compound or a pharmaceutical composition according to any one of claims 1 to 7 or 8 for use as a medicament.

13. A compound or a pharmaceutical composition for use according to claim 12, in the prevention and /or treatment of pulmonary disease selected from the group consisting of asthma, chronic obstructive pulmonary disease COPD, idiopathic pulmonary fibrosis (IPF), pulmonary hypertension (PH) and specifically Pulmonary Arterial Hypertension (PAH).

14. A combination of a compound as defined in any one of the claims 1 to 7 with one or more active ingredients selected from the classes consisting of organic nitrates and NO donors; inhaled NO; stimulator of soluble guanylate cyclase (sGC); prostaciclin analogue PGI2 and agonist of prostacyclin receptors; compounds that inhibit the degradation of cyclic guanosine monophosphate (cGMP) and/or cyclic adenosine monophosphate (cAMP); human neutrophilic elastase inhibitors; compounds inhibiting the signal transduction cascade; active substances for lowering blood pressure; neutral endopeptidase inhibitor; osmotic agents; ENaC blockers; anti-inflammatories including corticosteroids and antagonists of chemokine receptors; antihistamine drugs; anti-tussive drugs; antibiotics and DNase drug substance and selective cleavage agents; agents that inhibit ALK5 and/or ALK4 phosphorylation of Smad2 and Smad3; tryptophan hydroylase 1 (TPH1) inhibitors and multi-kinase inhibitors.

## Patentansprüche

1. Verbindung der Formel (I), 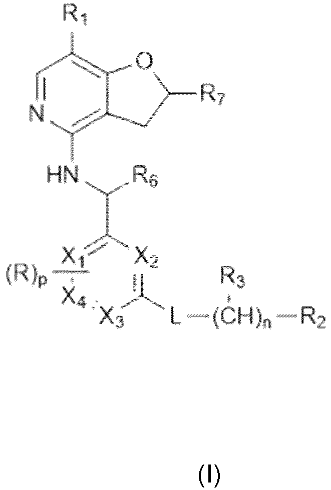 wobei
**X₁, X₂, X₃** und **X₄** alle CH sind oder eines von X₁, X₂, X₃ und X₄ N ist und die anderen CH sind;
p Null oder eine ganze Zahl von 1 bis 4 ist;
jedes R, wenn vorhanden, bei jedem Vorkommen Halogen ist, unabhängig ausgewählt aus (C₁-C₆)-Alkyl und Halogen, ausgewählt aus F, Cl, Br und I;
R₁ Pyrazolyl ist;
L -C(O)NH- oder -NHC(O)- ist;
n bei jedem Vorkommen unabhängig 0 oder eine ganze Zahl, ausgewählt aus 1, 2 oder 3, ist;
R₂ und R₃ bei jedem Vorkommen unabhängig ausgewählt sind aus der Gruppe bestehend aus
Halogen,
-OH,
-(CH₂)ₘNR₄R₅,
(C₁-C₆)-Alkyl,
(C₁-C₆)-Hydroxyalkyl,
(C₁-C₆)-Alkoxy,
(C₁-C₆)-Alkoxy-(C₁-C₆)-alkyl,
(C₁-C₆)-Halogenalkyl,
(C₁-C₆)-Halogenalkoxy,
(C₁-C₆)-Halogenalkoxy-(C₁-C₆)-alkyl,
(C₃-C₁₀)-Cycloalkyl,
Aryl, Heteroaryl und (C₃-C₆)-Heterocycloalkyl,
wobei jedes Cycloalkyl, Aryl, Heteroaryl und Heterocycloalkyl
wiederum optional und unabhängig substituiert ist mit einer oder mehreren Gruppen ausgewählt aus
Halogen,
-OH,
(C₁-C₆)-Alkyl,
(C₁-C₆)-Hydroxyalkyl,
(C₁-C₆)-Alkoxy,
(C₁-C₆)-Alkoxy-(C₁-C₆)-alkyl,
(C₁-C₆)-Halogenalkyl,
(C₁-C₆)-Halogenalkoxy,
-(CH₂)ₘNR₄R₅,
-O-(CH₂)ₘNR₄R₅,
Alkanoyl,
Aryl, Heteroaryl, Cycloalkyl,
Aryl-(C₁-C₆)-alkyl,
(C₃-C₆)-Heterocycloalkyl,
(C₃-C₈)-Heterocycloalkyl-(C₁-C₆)-alkyl,
wobei jedes Aryl, Heteroaryl, Cycloalkyl und Heterocycloalkyl noch weiter optional durch eine oder mehrere Gruppen substituiert ist, unabhängig ausgewählt aus Halogen, -OH, (C₁-C₈)-Alkyl, (C₁-C₆)-Halogenalkyl, (C₁-C₆)-Hydroxyalkyl;
m bei jedem Vorkommen unabhängig 0 oder eine ganze Zahl, ausgewählt aus 1, 2 oder 3 ist;
R₄ und R₅, gleich oder verschieden, ausgewählt sind aus der Gruppe bestehend aus
-H,
(C₁-C₆)-Alkyl,
(C₁-C₆)-Halogenalkyl,
(C₁-C₆)-Hydroxyalkyl,
(C₃-C₆)-Heterocycloalkyl;
R₆ und R₇ unabhängig ausgewählt sind aus der Gruppe bestehend aus -H, (C₁-C₆)-Alkyl;
wobei sich, wenn nicht bereits spezifiziert, Aryl auf mono-, bi- oder tricyclische Ringsysteme bezieht, die 6 bis 20 Ringatome aufweisen, in denen mindestens ein Ring aromatisch ist; sich Heteroaryl auf mono-, bi- oder tricyclische Ringsysteme mit 5 bis 20 Ringatomen bezieht, in denen mindestens ein Ring aromatisch ist und in denen mindestens ein Ringatom ein Heteroatom, ausgewählt aus N, S oder O, ist; sich (C₃-C₆)-Heterocycloalkyl auf gesättigte oder teilweise ungesättigte monocyclische (C₃-C₆)-Cycloalkylgruppen bezieht, in denen mindestens ein Ringkohlenstoffatom durch mindestens ein Heteroatom, ausgewählt aus N, NH, S oder O, ersetzt ist und/oder eine Oxo-Substituentengruppe tragen kann;
einzelne Enantiomere, Diastereoisomere und Mischungen davon in beliebigem Verhältnis und/oder pharmazeutisch verträgliche Salze und Solvate davon.

2. Verbindung nach Anspruch 1, wobei X₃ und X₄ alle CH-Gruppen sind und X₁ oder X₂ alternativ unabhängig eine CH-Gruppe oder ein Stickstoffatom sind;
R₁ Pyrazol-4-yl ist;
alle anderen Variablen wie in Anspruch 1 definiert sind;
einzelne Enantiomere, Diastereoisomere und Mischungen davon in beliebigem Verhältnis und/oder pharmazeutisch verträgliche Salze und Solvate davon.

3. Verbindung nach Anspruch 2, wobei X₁, X₂, X₃, X₄ alle CH-Gruppen sind;
jedes R, wenn vorhanden, bei jedem Vorkommen Halogen ist, unabhängig ausgewählt aus F, Cl, Br und I;
R₁ Pyrazolyl ist;
L -C(O)NH- oder -NHC(O)- ist;
n 0 ist;
R₂ bei jedem Vorkommen unabhängig ausgewählt ist aus der Gruppe bestehend aus
(C₁-C₆)-Alkyl,
(C₁-C₆)-Hydroxyalkyl,
(C₁-C₆)-Alkoxy-(C₁-C₆)-alkyl,
(C₁-C₆)-Halogenalkyl,
(C₁-C₆)-Halogenalkoxy-(C₁-C₆)-alkyl;
alle anderen Variablen wie in Anspruch 1 definiert sind,
einzelne Enantiomere, Diastereoisomere und Mischungen davon in beliebigem Verhältnis und/oder pharmazeutisch verträgliche Salze und Solvate davon.

4. Verbindung nach Anspruch 1, wobei **X₁, X₂, X₃** und **X₄** alle CH sind;
p Null oder eine ganze Zahl von 1 bis 4 ist;
jedes R, wenn vorhanden, bei jedem Vorkommen Halogen ist, unabhängig ausgewählt aus F, Cl, Br und I;
R₁ Pyrazolyl ist;
L -C(O)NH oder -NHC(O)- ist;
n bei jedem Vorkommen unabhängig 0 oder eine ganze Zahl, ausgewählt aus 1, 2 oder 3, ist;
R₃, wenn vorhanden, H oder (C₁-C₆)-Hydroxyalkyl ist und
R₂ ausgewählt ist aus der Gruppe bestehend aus
Aryl, Heteroaryl und (C₃-C₆)-Heterocycloalkyl,
wobei jedes Aryl, Heteroaryl und Heterocycloalkyl
wiederum optional und unabhängig substituiert ist mit einer oder mehreren Gruppen ausgewählt aus
(C₁-C₆)-Alkyl,
(C₁-C₆)-Hydroxyalkyl,
(C₁-C₆)-Alkoxy,
(C₁-C₆)-Alkoxy-(C₁-C₆)-alkyl,
-(CH₂)ₘNR₄R₅,
-O-(CH₂)ₘNR₄R₅,
Aryl, Heteroaryl, Cycloalkyl,
Aryl-(C₁-C₆)-alkyl,
(C₃-C₆)-Heterocycloalkyl,
(C₃-C₈)-Heterocycloalkyl-(C₁-C₆)-alkyl,
wobei jedes Aryl, Heteroaryl, Cycloalkyl und Heterocycloalkyl noch weiter optional durch eine oder mehrere Gruppen substituiert ist, unabhängig ausgewählt aus Halogen, -OH, (C₁-C₈)-Alkyl, (C₁-C₆)-Halogenalkyl, (C₁-C₆)-Hydroxyalkyl;
m bei jedem Vorkommen unabhängig 0 oder eine ganze Zahl, ausgewählt aus 1, 2 oder 3 ist;
R₄ und R₅, gleich oder verschieden, ausgewählt sind aus der Gruppe bestehend aus
-H,
(C₁-C₆)-Alkyl,
(C₁-C₆)-Halogenalkyl,
(C₁-C₆)-Hydroxyalkyl,
(C₃-C₆)-Heterocycloalkyl;
oder pharmazeutisch verträglichen Salzen und Solvaten davon.

5. Verbindung nach Anspruch 4, wobei R₂ ausgewählt ist aus (Pyridinyl)methyl, (Pyridinyl)ethyl, Methoxypyridinyl, ((Dimethylamino)ethoxy)pyridinyl oder aus 1-(2-(Dimethylamino)ethyl)-1H-indazol-5-yl, 1-(2-Morpholinoethyl)-1H-indazol-5-yl, 1-(1-Methylpiperidin-4-yl)-1H-indazol-5-yl.

6. Verbindung der Formel (I) nach Anspruch 1, wobei **X₁, X₂, X₃** und **X₄** alle CH sind;
p Null oder 1 ist;
jedes R, wenn vorhanden, F ist;
R₁ Pyrazol-4-yl ist;
L -C(O)NH- oder -NHC(O)- ist;
n bei jedem Vorkommen unabhängig 0 oder eine ganze Zahl, ausgewählt aus 1, 2 ist;
R₂ und R₃ bei jedem Vorkommen unabhängig ausgewählt sind aus der Gruppe bestehend aus
-H,
(C₁-C₆)-Alkyl, das Methyl ist,
(C₁-C₆)-Alkoxy-(C₁-C₆)-alkyl, das 2-Methoxyethyl ist,
(C₁-C₆)-Halogenalkyl, das 3-Fluorpropyl ist,
(C₃-C₁₀)-Cycloalkyl, das Cyclopropyl, Cyclobutyl ist,
Aryl, das Phenyl ist;
Heteroaryl, das Pyridinyl, Pyrazolyl, Thiophenyl, Imidazolyl, Oxazolyl, Isoxazolyl, Thiazolyl, Pyrimidinyl, 1,2,5-Oxadiazol-3-yl, 4,5,6,7-Tetrahydropyrazolo[1,5-a]pyrazin-2-yl, 4,5,6,7-Tetrahydrothiazolo[5,4-c]pyridin-2-yl, 4,5,6,7-Tetrahydrobenzo[d]thiazol-2-yl, 1,2,3,4-Tetrahydroisochinolin-6-yl, 5,6,7,8-Tetrahydro-1,7-naphthyridinyl, 1H-Indol-5-yl, Isoindolinyl, 1H-Indazol-5-yl ist,
und
(C₃-C₆)-Heterocycloalkyl, das Piperidinyl, Morpholinyl, Tetrahydrofuranyl, Tetrahydro-2H-pyran-yl ist;
wobei jedes Cycloalkyl, Aryl, Heteroaryl und Heterocycloalkyl
wiederum optional und unabhängig substituiert ist mit einer oder mehreren Gruppen ausgewählt aus
Halogen, das Fluor ist,
(C₁-C₆)-Alkyl, das Methyl ist,
(C₁-C₆)-Hydroxyalkyl, das Hydroxyethyl ist,
(C₁-C₆)-Alkoxy, das Methoxy ist,
-(CH₂)ₘNR₄R₅, das (Dimethylamino)methyl, 2-(Dimethylamino)ethyl, Dimethylamino ist, wobei R₄ und R₅ Methyl sind und m 0, 1 oder 2 ist, Oxetan-3-ylamino, wobei R₄ H ist, R₅ Oxetanyl ist und m 0 ist,
-O-(CH₂)ₘNR₄R₅, das ((Dimethylamino)ethoxy)pyridinyl ist;
Cycloalkyl, das Cyclopropyl ist,
Aryl-(C₁-C₆)-alkyl, das Benzyl, Phenethyl ist,
(C₃-C₆)-Heterocycloalkyl, das Oxetan-3-yl, Piperidin-4-yl ist,
(C₃-C₈)-Heterocycloalkyl-(C₁-C₆)-alkyl, das Morpholinoethyl, Pyrrolidin-1-ylmethyl ist,
wobei jedes des Heterocycloalkyls optional noch weiter substituiert ist durch (C₁-C₈)-Alkyl, das Methyl ist;
R₆ -H oder Methyl ist; und R₇ -H ist
einzelne Enantiomere, Diastereoisomere und Mischungen davon in beliebigem Verhältnis und/oder pharmazeutisch verträgliche Salze und Solvate davon.

7. Verbindung nach Anspruch 1, ausgewählt aus:
3-(((7-(1H-Pyrazol-4-yl)-2,3-dihydrofuro[3,2-c]pyridin-4-yl)amino)methyl)-N-(1-cyclopropylpiperidin-4-yl)benzamid;
3-(((7-(1H-Pyrazol-4-yl)-2,3-dihydrofuro[3,2-c]pyridin-4-yl)amino)methyl)-N-((1-methyl-1H-pyrazol-3-yl)methyl)benzamid;
3-(((7-(1H-Pyrazol-4-yl)-2,3-dihydrofuro[3,2-c]pyridin-4-yl)amino)methyl)-N-(3-fluorpropyl)benzamid;
3-(((7-(1H-Pyrazol-4-yl)-2,3-dihydrofuro[3,2-c]pyridin-4-yl)amino)methyl)-N-(pyridin-2-ylmethyl)benzamid;
3-(((7-(1H-Pyrazol-4-yl)-2,3-dihydrofuro[3,2-c]pyridin-4-yl)amino)methyl)-N-(cyclopropylmethyl)benzamid;
3-(((7-(1H-Pyrazol-4-yl)-2,3-dihydrofuro[3,2-c]pyridin-4-yl)amino)methyl)-N-(5,5-dimethyltetrahydrofuran-3-yl)benzamid;
3-(((7-(1H-Pyrazol-4-yl)-2,3-dihydrofuro[3,2-c]pyridin-4-yl)amino)methyl)-N-(2-methoxyethyl)benzamid;
3-(((7-(1H-Pyrazol-4-yl)-2,3-dihydrofuro[3,2-c]pyridin-4-yl)amino)methyl)-N-((1-methyl-1H-imidazol-4-yl)methyl)benzamid;
3-(((7-(1H-Pyrazol-4-yl)-2,3-dihydrofuro[3,2-c]pyridin-4-yl)amino)methyl)-N-(pyrimidin-5-ylmethyl)benzamid;
N-((1,2,5-Oxadiazol-3-yl)methyl)-3-(((7-(1H-pyrazol-4-yl)-2,3-dihydrofuro[3,2-c]pyridin-4-yl)amino)methyl)benzamid;
3-(((7-(1H-Pyrazol-4-yl)-2,3-dihydrofuro[3,2-c]pyridin-4-yl)amino)methyl)-N-((tetrahydro-2H-pyran-2-yl)methyl)benzamid;
3-(((7-(1H-Pyrazol-4-yl)-2,3-dihydrofuro[3,2-c]pyridin-4-yl)amino)methyl)-N-(3,3-difluorcyclobutyl)benzamid;
3-(((7-(1H-Pyrazol-4-yl)-2,3-dihydrofuro[3,2-c]pyridin-4-yl)amino)methyl)-N-(isoxazol-3-ylmethyl)benzamid;
3-(((7-(1H-Pyrazol-4-yl)-2,3-dihydrofuro[3,2-c]pyridin-4-yl)amino)methyl)-N-(thiazol-4-ylmethyl)benzamid;
3-(((7-(1H-Pyrazol-4-yl)-2,3-dihydrofuro[3,2-c]pyridin-4-yl)amino)methyl)-N-(5-methyl-4,5,6,7-tetrahydropyrazolo[1,5-a]pyrazin-2-yl)benzamid;
3-(((7-(1H-Pyrazol-4-yl)-2,3-dihydrofuro[3,2-c]pyridin-4-yl)amino)methyl)-N-(2-(1-methylpiperidin-4-yl)ethyl)benzamid;
3-(((7-(1H-Pyrazol-4-yl)-2,3-dihydrofuro[3,2-c]pyridin-4-yl)amino)methyl)-N-(2-(pyridin-4-yl)ethyl)benzamid;
3-(((7-(1H-Pyrazol-4-yl)-2,3-dihydrofuro[3,2-c]pyridin-4-yl)amino)methyl)-N-(3-((dimethylamino)methyl)phenyl)benzamid;
3-(((7-(1H-Pyrazol-4-yl)-2,3-dihydrofuro[3,2-c]pyridin-4-yl)amino)methyl)-N-(3-((dimethylamino)methyl)benzyl)benzamid;
3-(((7-(1H-Pyrazol-4-yl)-2,3-dihydrofuro[3,2-c]pyridin-4-yl)amino)methyl)-N-(2-morpholinoethyl)benzamid;
3-(((7-(1H-Pyrazol-4-yl)-2,3-dihydrofuro[3,2-c]pyridin-4-yl)amino)methyl)-N-(5-methyl-4,5,6,7-tetrahydrothiazolo[5,4-c]pyridin-2-yl)benzamid;
3-(((7-(1H-Pyrazol-4-yl)-2,3-dihydrofuro[3,2-c]pyridin-4-yl)amino)methyl)-N-(2-(pyridin-3-yl)ethyl)benzamid;
(S)-3-(((7-(1H-Pyrazol-4-yl)-2,3-dihydrofuro[3,2-c]pyridin-4-yl)amino)methyl)-N-(2-hydroxy-1-phenylethyl)benzamid;
3-(((7-(1H-Pyrazol-4-yl)-2,3-dihydrofuro[3,2-c]pyridin-4-yl)amino)methyl)-N-(4-((dimethylamino)methyl)benzyl)benzamid;
3-(((7-(1H-Pyrazol-4-yl)-2,3-dihydrofuro[3,2-c]pyridin-4-yl)amino)methyl)-N-((5-methylthiophen-2-yl)methyl)benzamid;
3-(((7-(1H-Pyrazol-4-yl)-2,3-dihydrofuro[3,2-c]pyridin-4-yl)amino)methyl)-N-(1-phenethylpiperidin-4-yl)benzamid;
3-(((7-(1H-Pyrazol-4-yl)-2,3-dihydrofuro[3,2-c]pyridin-4-yl)amino)methyl)-N-(1-(2-(dimethylamino)ethyl)-1H-pyrazol-4-yl)benzamid;
3-(((7-(1H-Pyrazol-4-yl)-2,3-dihydrofuro[3,2-c]pyridin-4-yl)amino)methyl)-N-(2-(1-(oxetan-3-yl)piperidin-4-yl)ethyl)benzamid;
3-(((7-(1H-Pyrazol-4-yl)-2,3-dihydrofuro[3,2-c]pyridin-4-yl)amino)methyl)-N-((5-methyl-4,5,6,7-tetrahydrothiazolo[5,4-c]pyridin-2-yl)methyl)benzamid;
3-(((7-(1H-Pyrazol-4-yl)-2,3-dihydrofuro[3,2-c]pyridin-4-yl)amino)methyl)-N-((2-methylisoindolin-5-yl)methyl)benzamid;
3-(((7-(1H-Pyrazol-4-yl)-2,3-dihydrofuro[3,2-c]pyridin-4-yl)amino)methyl)-N-(5-methoxypyridin-2-yl)benzamid;
3-(((7-(1H-Pyrazol-4-yl)-2,3-dihydrofuro[3,2-c]pyridin-4-yl)amino)methyl)-N-(pyrimidin-4-yl)benzamid;
3-(((7-(1H-Pyrazol-4-yl)-2,3-dihydrofuro[3,2-c]pyridin-4-yl)amino)methyl)-N-(6-(dimethylamino)-4,5,6,7-tetrahydrobenzo[d]thiazol-2-yl)benzamid;
3-(((7-(1H-Pyrazol-4-yl)-2,3-dihydrofuro[3,2-c]pyridin-4-yl)amino)methyl)-N-(6-(oxetan-3-ylamino)-4,5,6,7-tetrahydrobenzo[d]thiazol-2-yl)benzamid;
N-(3-(((7-(1H-Pyrazol-4-yl)-2,3-dihydrofuro[3,2-c]pyridin-4-yl)amino)methyl)phenyl)-4-((dimethylamino)methyl)benzamid;
N-(3-(((7-(1H-Pyrazol-4-yl)-2,3-dihydrofuro[3,2-c]pyridin-4-yl)amino)methyl)phenyl)-5-methyl-4,5,6,7-tetrahydrothiazolo[4,5-c]pyridin-2-carboxamid;
N-(3-(((7-(1H-Pyrazol-4-yl)-2,3-dihydrofuro[3,2-c]pyridin-4-yl)amino)methyl)phenyl)-5-methyl-4,5,6,7-tetrahydrothiazolo[5,4-c]pyridin-2-carboxamid;
N-(3-(((7-(1H-Pyrazol-4-yl)-2,3-dihydrofuro[3,2-c]pyridin-4-yl)amino)methyl)phenyl)-5-(2-hydroxyethyl)-4,5,6,7-tetrahydrothiazolo[5,4-c]pyridin-2-carboxamid;
N-(3-(((7-(1H-Pyrazol-4-yl)-2,3-dihydrofuro[3,2-c]pyridin-4-yl)amino)methyl)phenyl)-1-methyl-1H-indazol-5-carboxamid;
N-(3-(((7-(1H-Pyrazol-4-yl)-2,3-dihydrofuro[3,2-c]pyridin-4-yl)amino)methyl)phenyl)-1,2,3,4-tetrahydroisochinolin-6-carboxamid;
N-(3-(((7-(1H-Pyrazol-4-yl)-2,3-dihydrofuro[3,2-c]pyridin-4-yl)amino)methyl)phenyl)-1-methyl-1H-indazol-3-carboxamid;
N-(3-(((7-(1H-Pyrazol-4-yl)-2,3-dihydrofuro[3,2-c]pyridin-4-yl)amino)methyl)phenyl)-1-methyl-1H-indazol-4-carboxamid;
N-(3-(((7-(1H-Pyrazol-4-yl)-2,3-dihydrofuro[3,2-c]pyridin-4-yl)amino)methyl)phenyl)-1-(2-(dimethylamino)ethyl)-1H-indazol-5-carboxamid;
N-(3-(((7-(1H-Pyrazol-4-yl)-2,3-dihydrofuro[3,2-c]pyridin-4-yl)amino)methyl)phenyl)-1-(1-methylpiperidin-4-yl)-1H-indazol-5-carboxamid;
N-(3-(((7-(1H-Pyrazol-4-yl)-2,3-dihydrofuro[3,2-c]pyridin-4-yl)amino)methyl)phenyl)-2-methyl-1,2,3,4-tetrahydroisochinolin-6-carboxamid;
N-(3-(((7-(1H-Pyrazol-4-yl)-2,3-dihydrofuro[3,2-c]pyridin-4-yl)amino)methyl)phenyl)-1-(2-morpholinoethyl)-1H-indazol-5-carboxamid;
N-(5-(((7-(1H-Pyrazol-4-yl)-2,3-dihydrofuro[3,2-c]pyridin-4-yl)amino)methyl)-2-fluorphenyl)-1,2,3,4-tetrahydroisochinolin-6-carboxamid;
N-(3-(((7-(1H-Pyrazol-4-yl)-2,3-dihydrofuro[3,2-c]pyridin-4-yl)amino)methyl)phenyl)-5-(pyrrolidin-1-ylmethyl)thiazol-2-carboxamid;
N-(3-(((7-(1H-Pyrazol-4-yl)-2,3-dihydrofuro[3,2-c]pyridin-4-yl)amino)methyl)phenyl)-3-((dimethylamino)methyl)benzamid;
N-(3-(((7-(1H-Pyrazol-4-yl)-2,3-dihydrofuro[3,2-c]pyridin-4-yl)amino)methyl)phenyl)-2-phenylacetamid;
N-(3-(((7-(1H-Pyrazol-4-yl)-2,3-dihydrofuro[3,2-c]pyridin-4-yl)amino)methyl)phenyl)-7-methyl-5,6,7,8-tetrahydro-1,7-naphthyridin-3-carboxamid;
N-(3-(1-((7-(1H-Pyrazol-4-yl)-2,3-dihydrofuro[3,2-c]pyridin-4-yl)amino)ethyl)phenyl)acetamid;
3-(((7-(1H-Pyrazol-4-yl)-2,3-dihydrofuro[3,2-c]pyridin-4-yl)amino)methyl)-N-(pyridin-4-ylmethyl)benzamid;
3-(((7-(1H-Pyrazol-4-yl)-2,3-dihydrofuro[3,2-c]pyridin-4-yl)amino)methyl)-N-(1-benzylpiperidin-4-yl)benzamid;
3-(((7-(1H-Pyrazol-4-yl)-2,3-dihydrofuro[3,2-c]pyridin-4-yl)amino)methyl)-N-((tetrahydro-2H-pyran-2-yl)methyl)benzamid (Enantiomer 1);
3-(((7-(1H-Pyrazol-4-yl)-2,3-dihydrofuro[3,2-c]pyridin-4-yl)amino)methyl)-N-((tetrahydro-2H-pyran-2-yl)methyl)benzamid (Enantiomer 2);
3-(((7-(1H-Pyrazol-4-yl)-2,3-dihydrofuro[3,2-c]pyridin-4-yl)amino)methyl)-N-(5-(2-(dimethylamino)ethoxy)pyridin-2-yl)benzamid;
einzelne Enantiomere, Diastereoisomere und Mischungen davon in beliebigem Verhältnis und/oder pharmazeutisch verträgliche Salze und Solvate davon.

8. Pharmazeutische Zusammensetzung, umfassend eine Verbindung nach einem der Ansprüche 1 bis 7 oder ein pharmazeutisch verträgliches Salz oder Solvat davon, in Beimischung mit einem oder mehreren pharmazeutisch verträglichen Trägern oder Hilfsstoffen.

9. Zur Verabreichung durch Inhalation geeignete pharmazeutische Zusammensetzung nach Anspruch 8, ausgewählt aus inhalierbaren Pulvern, treibgashaltigen Dosieraerosolen oder treibgasfreien inhalierbaren Formulierungen.

10. Vorrichtung, umfassend die pharmazeutische Zusammensetzung nach Anspruch 9, die ein Trockenpulverinhalator für Einzel- oder Mehrfachdosen, ein Dosierinhalator und ein Zerstäuber für sanften Nebel sein kann.

11. Pharmazeutische Zusammensetzung nach Anspruch 8, die geeignet ist, um durch oralen Weg verabreicht zu werden und aus Gelkapseln, Kapseln, Dragees, Granulat, Lutschtabletten und losen Pulvern oder wässrigen und nichtwässrigen Lösungen, Emulsionen, Suspensionen, Sirupen und Elixirformulierungen ausgewählt ist.

12. Verbindung oder pharmazeutische Zusammensetzung nach einem der Ansprüche 1 bis 7 oder 8 zur Verwendung als ein Medikament.

13. Verbindung oder pharmazeutische Zusammensetzung zur Verwendung nach Anspruch 12 bei der Vorbeugung und/oder Behandlung von Lungenkrankheit, ausgewählt aus der Gruppe bestehend aus Asthma, chronisch obstruktiver Lungenkrankheit COPD, idiopathischer Lungenfibrose (IPF), Lungenhochdruck (PH) und spezifisch arteriellem Lungenhochdruck (PAH).

14. Kombination einer Verbindung nach einem der Ansprüche 1 bis 7 mit einem oder mehreren Wirkstoffen, ausgewählt aus den Klassen bestehend aus organischen Nitraten und NO-Donatoren; inhaliertem NO; Stimulator der löslichen Guanylatcyclase (sGC); Prostacyclin-Analogon PGI2 und Agonist von Prostacyclin-Rezeptoren; Verbindungen, die den Abbau von cyclischem Guanosinmonophosphat (cGMP) und/oder cyclischem Adenosinmonophosphat (cAMP) hemmen; Inhibitoren der humanen neutrophilen Elastase; Verbindungen, die die Signaltransduktionskaskade hemmen; aktiven Stoffen zum Senken des Blutdrucks; neutralem Endopeptidase-Inhibitor; osmotischen Mitteln; ENaC-Blockern; Entzündungshemmern einschließlich Kortikosteroiden und Antagonisten von Chemokinrezeptoren; Antihistaminika; hustenstillenden Arzneimitteln; Antibiotika und DNase-Arzneimittelstoff und selektiven Spaltmitteln; Mitteln, die die ALK5- und/oder ALK4-Phosphorylierung von Smad2 und Smad3 hemmen; Tryptophanhydroylase-1-Inhibitoren (TPH1-Inhibitoren) und Multikinase-Inhibitoren.

## Revendications

1. Composé de formule (I) 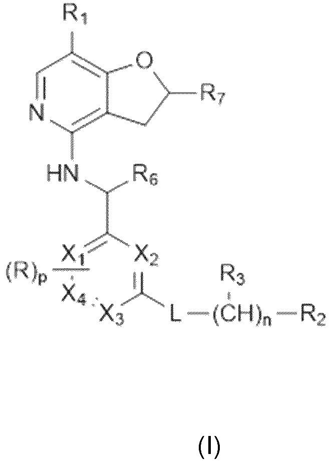 dans lequel
**X₁, X₂, X₃** et **X₄** sont tous CH ou l'un de X₁, X₂, X₃ et X₄ est N et les autres sont CH ;
p vaut zéro ou est un nombre entier allant de 1 à 4 ;
chaque R, lorsqu'il est présent, est halogène dans chaque cas, choisi indépendamment parmi alkyle en C₁-C₆ et halogène choisi parmi F, Cl, Br et I ;
R₁ est pyrazolyle ;
L est -C(O)NH- ou -NHC(O)- ;
n, dans chaque cas, vaut indépendamment 0 ou est un nombre entier choisi parmi 1, 2 ou 3 ;
R₂ et R₃, dans chaque cas, sont choisis indépendamment dans le groupe constitué de
-H,
halogène,
-OH,
-(CH₂)ₘNR₄R₅,
alkyle en C₁-C₆,
hydroxyalkyle en C₁-C₆,
alcoxy en C₁-C₆,
alcoxy en C₁-C₆-alkyle en C₁-C₆,
haloalkyle en C₁-C₆,
haloalcoxy en C₁-C₆,
haloalcoxy en C₁-C₆-alkyle en C₁-C₆,
cycloalkyle en C₃-C₁₀,
aryle, hétéroaryle et hétérocycloalkyle en C₃-C₆,
dont chaque cycloalkyle, aryle, hétéroaryle ou hétérocycloalkyle
est à son tour facultativement et indépendamment substitué par un ou plusieurs groupes choisis parmi
halogène,
-OH,
alkyle en C₁-C₆,
hydroxyalkyle en C₁-C₆,
alcoxy en C₁-C₆,
alcoxy en C₁-C₆-alkyle en C₁-C₆,
haloalkyle en C₁-C₆,
haloalcoxy en C₁-C₆,
-(CH₂)ₘNR₄R₅,
-O-(CH₂)ₘNR₄R₅,
alcanoyle,
aryle, hétéroaryle, cycloalkyle,
aryl-alkyle en C₁-C₆,
hétérocycloalkyle en C₃-C₆,
hétérocycloalkyle en C₃-C₈-alkyle en C₁-C₆,
chacun desdits aryle, hétéroaryle, cycloalkyle, hétérocycloalkyle est en outre facultativement substitué par un ou plusieurs groupes choisis indépendamment parmi halogène, -OH, alkyle en C₁-C₈, haloalkyle en C₁-C₆, hydroxyalkyle en C₁-C₆ ;
m, dans chaque cas, vaut indépendamment 0 ou est un nombre entier choisi parmi 1, 2 ou 3 ;
R₄ et R₅, identiques ou différents, sont choisis dans le groupe constitué de
-H,
alkyle en C₁-C₆,
haloalkyle en C₁-C₆,
hydroxyalkyle en C₁-C₆,
hétérocycloalkyle en C₃-C₆ ;
R₆ et R7 sont choisis indépendamment dans le groupe constitué de -H, alkyle en C₁-C₆ ;
dans lequel, lorsque ce n'est pas déjà spécifié, aryle se réfère à des systèmes cycliques mono-, bi- ou tri-cycliques qui ont 6 à 20 atomes de cycle, dans lesquels au moins un cycle est aromatique ; hétéroaryle se réfère à des systèmes cycliques mono-, bi- ou tri-cycliques avec 5 à 20 atomes de cycle, dans lesquels au moins un cycle est aromatique et dans lesquels au moins un atome de cycle est un hétéroatome choisi parmi N, S ou O ; hétérocycloalkyle en C₃-C₆ se réfère à des groupes cycloalkyle en C₃-C₆ monocycliques, saturés ou partiellement insaturés, dans lesquels au moins un atome de carbone de cycle est remplacé par au moins un hétéroatome choisi parmi N, NH, S ou O et/ou peut porter un groupe substituant oxo ;
énantiomères seuls, diastéréo-isomères et mélanges de ceux-ci dans n'importe quelle proportion et/ou sels et solvates pharmaceutiquement acceptables de ceux-ci.

2. Composé selon la revendication 1, dans lequel X₃ et X₄ sont tous des groupes CH et X₁ ou X₂ sont, à titre subsidiaire, indépendamment un groupe CH ou un atome d'azote ;
R₁ est pyrazol-4-yle ;
toutes les autres variables étant telles que définies dans la revendication 1 ;
énantiomères seuls, diastéréo-isomères et mélanges de ceux-ci dans n'importe quelle proportion et/ou sels et solvates pharmaceutiquement acceptables de ceux-ci.

3. Composé selon la revendication 2, dans lequel X₁, X₂, X₃, X₄ sont tous des groupes CH ;
chaque R, lorsqu'il est présent, est halogène dans chaque cas choisi indépendamment parmi F, Cl, Br et I ;
R₁ est pyrazolyle ;
L est -C(O)NH- ou -NHC(O)- ;
n vaut 0 ;
R₂, dans chaque cas, est choisi indépendamment dans le groupe constitué de
alkyle en C₁-C₆,
hydroxyalkyle en C₁-C₆,
alcoxy en C₁-C₆-alkyle en C₁-C₆,
haloalkyle en C₁-C₆,
haloalcoxy en C₁-C₆-alkyle en C₁-C₆ ;
toutes les autres variables étant telles que définies dans la revendication 1,
énantiomères seuls, diastéréo-isomères et mélanges de ceux-ci dans n'importe quelle proportion et/ou sels et solvates pharmaceutiquement acceptables de ceux-ci.

4. Composé selon la revendication 1, dans lequel **X₁, X₂, X₃** et **X₄** sont tous CH;
p vaut zéro ou est un nombre entier allant de 1 à 4 ;
chaque R, lorsqu'il est présent, est halogène dans chaque cas choisi indépendamment parmi F, Cl, Br et I ;
R₁ est pyrazolyle ;
L est -C(O)NH ou-NHC(O)- ;
n, dans chaque cas, vaut indépendamment 0 ou est un nombre entier choisi parmi 1, 2 ou 3 ;
R₃, lorsqu'il est présent, est H, ou hydroxyalkyle en C₁-C₆, et
R₂ est choisi dans le groupe constitué de
aryle, hétéroaryle et hétérocycloalkyle en C₃-C₆,
dont chaque aryle, hétéroaryle ou hétérocycloalkyle
est à son tour facultativement et indépendamment substitué par un ou plusieurs groupes choisis parmi
alkyle en C₁-C₆,
hydroxyalkyle en C₁-C₆,
alcoxy en C₁-C₆,
alcoxy en C₁-C₆-alkyle en C₁-C₆,
-(CH₂)ₘNR₄R₅,
-O-(CH₂)ₘNR₄R₅,
aryle, hétéroaryle, cycloalkyle,
aryl-alkyle en C₁-C₆,
hétérocycloalkyle en C₃-C₆,
hétérocycloalkyle en C₃-C₈-alkyle en C₁-C₆,
chacun desdits aryle, hétéroaryle, cycloalkyle, hétérocycloalkyle est en outre facultativement substitué par un ou plusieurs groupes choisis indépendamment parmi halogène, -OH, alkyle en C₁-C₈, haloalkyle en C₁-C₆, hydroxyalkyle en C₁-C₆ ;
m, dans chaque cas, vaut indépendamment 0 ou est un nombre entier choisi parmi 1, 2 ou 3 ;
R₄ et R₅, identiques ou différents, sont choisis dans le groupe constitué de
-H,
alkyle en C₁-C₆,
haloalkyle en C₁-C₆,
hydroxyalkyle en C₁-C₆,
hétérocycloalkyle en C₃-C₆ ;
ou sels et solvates pharmaceutiquement acceptables de ceux-ci.

5. Composé selon la revendication 4, dans lequel R₂ est choisi parmi (pyridinyl)méthyle, (pyridinyl)éthyle, méthoxypyridinyle, ((diméthylamino)éthoxy)pyridinyle, ou parmi 1-(2-(diméthylamino)éthyl)-1H-indazol-5-yle, 1-(2-morpholinoéthyl)-1H-indazol-5-yle, 1-(1-méthylpipéridin-4-yl)-1H-indazol-5-yle.

6. Composé de formule (I) selon la revendication 1, dans lequel **X₁, X₂, X₃** et **X₄** sont tous CH ;
p vaut zéro ou 1 ;
chaque R, lorsqu'il est présent, est F ;
R₁ est **pyrazol-4-yle** ;
L est -C(O)NH- ou -NHC(O)- ;
n, dans chaque cas, vaut indépendamment 0 ou est un nombre entier choisi parmi 1, 2 ;
R₂ et R₃, dans chaque cas, sont choisis indépendamment dans le groupe constitué de
-H,
alkyle en C₁-C₆ qui est méthyle,
alcoxy en C₁-C₆-alkyle en C₁-C₆ qui est 2-méthoxyéthyle,
haloalkyle en C₁-C₆ qui est 3-fluoropropyle,
cycloalkyle en C₃-C₁₀ qui est cyclopropyle, cyclobutyle,
aryle qui est phényle ;
hétéroaryle qui est pyridinyle, pyrazolyle, thiophényle, imidazolyle, oxazolyle, isoxazolyle, thiazolyle, pyrimidinyle, 1,2,5-oxadiazol-3-yle, 4,5,6,7-tétrahydropyrazolo[1,5-a]pyrazin-2-yle, 4,5,6,7-tétrahydrothiazolo[5,4-c]pyridin-2-yle, 4,5,6,7-tétrahydrobenzo[d]thiazol-2-yle, 1,2,3,4-tétrahydroisoquinolin-6-yle, 5,6,7,8-tétrahydro-1,7-naphtyridinyle, 1H-indol-5-yle, isoindolinyle, 1H-indazol-5-yle,
et
hétérocycloalkyle en C₃-C₆ qui est pipéridinyle, morpholinyle, tétrahydrofuranyle, tétrahydro-2H-pyranyle ;
dont chaque cycloalkyle, aryle, hétéroaryle ou hétérocycloalkyle
est à son tour facultativement et indépendamment substitué par un ou plusieurs groupes choisis parmi
halogène qui est fluoro,
alkyle en C₁-C₆ qui est méthyle,
hydroxyalkyle en C₁-C₆, qui est hydroxyéthyle,
alcoxy en C₁-C₆, qui est méthoxy,
-(CH₂)ₘNR₄R₅ qui est (diméthylamino)méthyle, 2-(diméthylamino)éthyle, diméthylamino dans lequel R₄ et R₅ sont méthyle et m vaut 0, 1 ou 2, oxétan-3-ylamino dans lequel R₄ est H, R₅ est oxétanyle et m vaut 0,
-O-(CH₂)ₘNR₄R₅ qui est ((diméthylamino)éthoxy)pyridinyle ;
cycloalkyle qui est cyclopropyle,
aryl-alkyle en C₁-C₆, qui est benzyle, phénéthyle,
hétérocycloalkyle en C₃-C₆, qui est oxétan-3-yle, pipéridin-4-yle,
hétérocycloalkyl en C₃-C₈-alkyle en C₁-C₆, qui est morpholinoéthyle, pyrrolidin-1-ylméthyle,
chacun desdits hétérocycloalkyles est en outre facultativement substitué par alkyle en C₁-C₈ qui est méthyle ;
R₆ est -H, ou méthyle ; et R₇ est -H
énantiomères seuls, diastéréo-isomères et mélanges de ceux-ci dans n'importe quelle proportion et/ou sels et solvates pharmaceutiquement acceptables de ceux-ci.

7. Composé selon la revendication 1 choisi parmi :
3-(((7-(1H-pyrazol-4-yl)-2,3-dihydrofuro[3,2-c]pyridin-4-yl)amino)méthyl)-N-(1-cyclopropylpipéridin-4-yl)benzamide ;
3-(((7-(1H-pyrazol-4-yl)-2,3-dihydrofuro[3,2-c]pyridin-4-yl)amino)méthyl)-N-((1-méthyl-1H-pyrazol-3-yl)méthyl)benzamide ;
3-(((7-(1H-pyrazol-4-yl)-2,3-dihydrofuro[3,2-c]pyridin-4-yl)amino)méthyl)-N-(3-fluoropropyl)benzamide ;
3-(((7-(1H-pyrazol-4-yl)-2,3-dihydrofuro[3,2-c]pyridin-4-yl)amino)méthyl)-N-(pyridin-2-ylméthyl)benzamide ;
3-(((7-(1H-pyrazol-4-yl)-2,3-dihydrofuro[3,2-c]pyridin-4-yl)amino)méthyl)-N-(cyclopropylméthyl)benzamide ;
3-(((7-(1H-pyrazol-4-yl)-2,3-dihydrofuro[3,2-c]pyridin-4-yl)amino)méthyl)-N-(5,5-diméthyltétrahydrofuran-3-yl)benzamide ;
3-(((7-(1H-pyrazol-4-yl)-2,3-dihydrofuro[3,2-c]pyridin-4-yl)amino)méthyl)-N-(2-méthoxyéthyl)benzamide ;
3-(((7-(1H-pyrazol-4-yl)-2,3-dihydrofuro[3,2-c]pyridin-4-yl)amino)méthyl)-N-((1-méthyl-1H-imidazol-4-yl)méthyl)benzamide ;
3-(((7-(1H-pyrazol-4-yl)-2,3-dihydrofuro[3,2-c]pyridin-4-yl)amino)méthyl)-N-(pyrimidin-5-ylméthyl)benzamide ;
N-((1,2,5-oxadiazol-3-yl)méthyl)-3-(((7-(1H-pyrazol-4-yl)-2,3-dihydrofuro[3,2-c]pyridin-4-yl)amino)méthyl)benzamide ;
3-(((7-(1H-pyrazol-4-yl)-2,3-dihydrofuro[3,2-c]pyridin-4-yl)amino)méthyl)-N-((tétrahydro-2H-pyran-2-yl)méthyl)benzamide ;
3-(((7-(1H-pyrazol-4-yl)-2,3-dihydrofuro[3,2-c]pyridin-4-yl)amino)méthyl)-N-(3,3-difluorocyclobutyl)benzamide ;
3-(((7-(1H-pyrazol-4-yl)-2,3-dihydrofuro[3,2-c]pyridin-4-yl)amino)méthyl)-N-(isoxazol-3-ylméthyl)benzamide ;
3-(((7-(1H-pyrazol-4-yl)-2,3-dihydrofuro[3,2-c]pyridin-4-yl)amino)méthyl)-N-(thiazol-4-ylméthyl)benzamide ;
3-(((7-(1H-pyrazol-4-yl)-2,3-dihydrofuro[3,2-c]pyridin-4-yl)amino)méthyl)-N-(5-méthyl-4,5,6,7-tétrahydropyrazolo[1,5-a]pyrazin-2-yl)benzamide ;
3-(((7-(1H-pyrazol-4-yl)-2,3-dihydrofuro[3,2-c]pyridin-4-yl)amino)méthyl)-N-(2-(1-méthylpipéridin-4-yl)éthyl)benzamide ;
3-(((7-(1H-pyrazol-4-yl)-2,3-dihydrofuro[3,2-c]pyridin-4-yl)amino)méthyl)-N-(2-(pyridin-4-yl)éthyl)benzamide ;
3-(((7-(1H-pyrazol-4-yl)-2,3-dihydrofuro[3,2-c]pyridin-4-yl)amino)méthyl)-N-(3-((diméthylamino)méthyl)phényl)benzamide ;
3-(((7-(1H-pyrazol-4-yl)-2,3-dihydrofuro[3,2-c]pyridin-4-yl)amino)méthyl)-N-(3-((diméthylamino)méthyl)benzyl)benzamide ;
3-(((7-(1H-pyrazol-4-yl)-2,3-dihydrofuro[3,2-c]pyridin-4-yl)amino)méthyl)-N-(2-morpholinoéthyl)benzamide ;
3-(((7-(1H-pyrazol-4-yl)-2,3-dihydrofuro[3,2-c]pyridin-4-yl)amino)méthyl)-N-(5-méthyl-4,5,6,7-tétrahydrothiazolo[5,4-c]pyridin-2-yl)benzamide ;
3-(((7-(1H-pyrazol-4-yl)-2,3-dihydrofuro[3,2-c]pyridin-4-yl)amino)méthyl)-N-(2-(pyridin-3-yl)éthyl)benzamide ;
(S)-3-(((7-(1H-pyrazol-4-yl)-2,3-dihydrofuro[3,2-c]pyridin-4-yl)amino)méthyl)-N-(2-hydroxy-1-phényléthyl)benzamide ;
3-(((7-(1H-pyrazol-4-yl)-2,3-dihydrofuro[3,2-c]pyridin-4-yl)amino)méthyl)-N-(4-((diméthylamino)méthyl)benzyl)benzamide ;
3-(((7-(1H-pyrazol-4-yl)-2,3-dihydrofuro[3,2-c]pyridin-4-yl)amino)méthyl)-N-((5-méthylthiophén-2-yl)méthyl)benzamide ;
3-(((7-(1H-pyrazol-4-yl)-2,3-dihydrofuro[3,2-c]pyridin-4-yl)amino)méthyl)-N-(1-phénéthylpipéridin-4-yl)benzamide ;
3-(((7-(1H-pyrazol-4-yl)-2,3-dihydrofuro[3,2-c]pyridin-4-yl)amino)méthyl)-N-(1-(2-(diméthylamino)éthyl)-1H-pyrazol-4-yl)benzamide ;
3-(((7-(1H-pyrazol-4-yl)-2,3-dihydrofuro[3,2-c]pyridin-4-yl)amino)méthyl)-N-(2-(1-(oxétan-3-yl)pipéridin-4-yl)éthyl)benzamide ;
3-(((7-(1H-pyrazol-4-yl)-2,3-dihydrofuro[3,2-c]pyridin-4-yl)amino)méthyl)-N-((5-méthyl-4,5,6,7-tétrahydrothiazolo[5,4-c]pyridin-2-yl)méthyl)benzamide ;
3-(((7-(1H-pyrazol-4-yl)-2,3-dihydrofuro[3,2-c]pyridin-4-yl)amino)méthyl)-N-((2-méthylisoindolin-5-yl)méthyl)benzamide ;
3-(((7-(1H-pyrazol-4-yl)-2,3-dihydrofuro[3,2-c]pyridin-4-yl)amino)méthyl)-N-(5-méthoxypyridin-2-yl)benzamide ;
3-(((7-(1H-pyrazol-4-yl)-2,3-dihydrofuro[3,2-c]pyridin-4-yl)amino)méthyl)-N-(pyrimidin-4-yl)benzamide ;
3-(((7-(1H-pyrazol-4-yl)-2,3-dihydrofuro[3,2-c]pyridin-4-yl)amino)méthyl)-N-(6-(diméthylamino)-4,5,6,7-tétrahydrobenzo[d]thiazol-2-yl)benzamide ;
3-(((7-(1H-pyrazol-4-yl)-2,3-dihydrofuro[3,2-c]pyridin-4-yl)amino)méthyl)-N-(6-(oxétan-3-ylamino)-4,5,6,7-tétrahydrobenzo[d]thiazol-2-yl)benzamide ;
N-(3-(((7-(1H-pyrazol-4-yl)-2,3-dihydrofuro[3,2-c]pyridin-4-yl)amino)méthyl)phényl)-4-((diméthylamino)méthyl)benzamide ;
N-(3-(((7-(1H-pyrazol-4-yl)-2,3-dihydrofuro[3,2-c]pyridin-4-yl)amino)méthyl)phényl)-5-méthyl-4,5,6,7-tétrahydrothiazolo[4,5-c]pyridine-2-carboxamide ;
N-(3-(((7-(1H-pyrazol-4-yl)-2,3-dihydrofuro[3,2-c]pyridin-4-yl)amino)méthyl)phényl)-5-méthyl-4,5,6,7-tétrahydrothiazolo[5,4-c]pyridine-2-carboxamide ;
N-(3-(((7-(1H-pyrazol-4-yl)-2,3-dihydrofuro[3,2-c]pyridin-4-yl)amino)méthyl)phényl)-5-(2-hydroxyéthyl)-4,5,6,7-tétrahydrothiazolo[5,4-c]pyridine-2-carboxamide ;
N-(3-(((7-(1H-pyrazol-4-yl)-2,3-dihydrofuro[3,2-c]pyridin-4-yl)amino)méthyl)phényl)-1-méthyl-1H-indazol-5-carboxamide ;
N-(3-(((7-(1H-pyrazol-4-yl)-2,3-dihydrofuro[3,2-c]pyridin-4-yl)amino)méthyl)phényl)-1,2,3,4-tétrahydroisoquinoline-6-carboxamide ;
N-(3-(((7-(1H-pyrazol-4-yl)-2,3-dihydrofuro[3,2-c]pyridin-4-yl)amino)méthyl)phényl)-1-méthyl-1H-indazol-3-carboxamide ;
N-(3-(((7-(1H-pyrazol-4-yl)-2,3-dihydrofuro[3,2-c]pyridin-4-yl)amino)méthyl)phényl)-1-méthyl-1H-indazol-4-carboxamide ;
N-(3-(((7-(1H-pyrazol-4-yl)-2,3-dihydrofuro[3,2-c]pyridin-4-yl)amino)méthyl)phényl)-1-(2-(diméthylamino)éthyl)-1H-indazol-5-carboxamide ;
N-(3-(((7-(1H-pyrazol-4-yl)-2,3-dihydrofuro[3,2-c]pyridin-4-yl)amino)méthyl)phényl)-1-(1-méthylpipéridin-4-yl)-1H-indazol-5-carboxamide ;
N-(3-(((7-(1H-pyrazol-4-yl)-2,3-dihydrofuro[3,2-c]pyridin-4-yl)amino)méthyl)phényl)-2-méthyl-1,2,3,4-tétrahydroisoquinoline-6-carboxamide ;
N-(3-(((7-(1H-pyrazol-4-yl)-2,3-dihydrofuro[3,2-c]pyridin-4-yl)amino)méthyl)phényl)-1-(2-morpholinoéthyl)-1H-indazol-5-carboxamide ;
N-(5-(((7-(1H-pyrazol-4-yl)-2,3-dihydrofuro[3,2-c]pyridin-4-yl)amino)méthyl)-2-fluorophényl)-1,2,3,4-tétrahydroisoquinoline-6-carboxamide ;
N-(3-(((7-(1H-pyrazol-4-yl)-2,3-dihydrofuro[3,2-c]pyridin-4-yl)amino)méthyl)phényl)-5-(pyrrolidin-1-ylméthyl)thiazol-2-carboxamide ;
N-(3-(((7-(1H-pyrazol-4-yl)-2,3-dihydrofuro[3,2-c]pyridin-4-yl)amino)méthyl)phényl)-3-((diméthylamino)méthyl)benzamide ;
N-(3-(((7-(1H-pyrazol-4-yl)-2,3-dihydrofuro[3,2-c]pyridin-4-yl)amino)méthyl)phényl)-2-phénylacétamide ;
N-(3-(((7-(1H-pyrazol-4-yl)-2,3-dihydrofuro[3,2-c]pyridin-4-yl)amino)méthyl)phényl)-7-méthyl-5,6,7,8-tétrahydro-1,7-naphtyridine-3-carboxamide ;
N-(3-(1-((7-(1H-pyrazol-4-yl)-2,3-dihydrofuro[3,2-c]pyridin-4-yl)amino)éthyl)phényl)acétamide ;
3-(((7-(1H-pyrazol-4-yl)-2,3-dihydrofuro[3,2-c]pyridin-4-yl)amino)méthyl)-N-(pyridin-4-ylméthyl)benzamide ;
3-(((7-(1H-pyrazol-4-yl)-2,3-dihydrofuro[3,2-c]pyridin-4-yl)amino)méthyl)-N-(1-benzylpipéridin-4-yl)benzamide ;
3-(((7-(1H-pyrazol-4-yl)-2,3-dihydrofuro[3,2-c]pyridin-4-yl)amino)méthyl)-N-((tétrahydro-2H-pyran-2-yl)méthyl)benzamide (énantiomère 1) ;
3-(((7-(1H-pyrazol-4-yl)-2,3-dihydrofuro[3,2-c]pyridin-4-yl)amino)méthyl)-N-((tétrahydro-2H-pyran-2-yl)méthyl)benzamide (énantiomère 2) ;
3-(((7-(1H-pyrazol-4-yl)-2,3-dihydrofuro[3,2-c]pyridin-4-yl)amino)méthyl)-N-(5-(2-(diméthylamino)éthoxy)pyridin-2-yl)benzamide ;
énantiomères seuls, diastéréo-isomères et mélanges de ceux-ci dans n'importe quelle proportion et/ou sels et solvates pharmaceutiquement acceptables de ceux-ci.

8. Composition pharmaceutique comprenant un composé selon l'une quelconque des revendications 1 à 7, ou un sel ou solvate pharmaceutiquement acceptable de celui-ci, en mélange avec un ou plusieurs supports ou excipients pharmaceutiquement acceptables.

9. Composition pharmaceutique selon la revendication 8, apte à être administrée par inhalation, choisie parmi des poudres inhalables, aérosols-doseurs contenant un agent propulseur ou formulations inhalables sans agent propulseur.

10. Dispositif comprenant la composition pharmaceutique selon la revendication 9, qui peut être un inhalateur de poudre sèche à dose unique ou à doses multiples, un aérosol-doseur et un nébuliseur à brouillard doux.

11. Composition pharmaceutique selon la revendication 8, apte à être administrée par voie orale, choisie parmi des gélules, capsules, comprimés, granules, pastilles et poudres en vrac ou solutions aqueuses et non aqueuses, émulsions, suspensions, sirops, et formulations d'élixirs.

12. Composé ou composition pharmaceutique selon l'une quelconque des revendications 1 à 7 ou 8, pour utilisation comme médicament.

13. Composé ou composition pharmaceutique pour utilisation selon la revendication 12, dans la prévention et/ou le traitement de maladie pulmonaire choisie dans le groupe constitué d'asthme, maladie pulmonaire obstructive chronique MPOC, fibrose pulmonaire idiopathique (FPI), hypertension pulmonaire (HP) et plus particulièrement hypertension artérielle pulmonaire (HTAP).

14. Combinaison d'un composé selon l'une quelconque des revendications 1 à 7 avec un ou plusieurs ingrédients actifs choisis parmi les classes constituées de nitrates organiques et donneurs de NO ; NO inhalé ; stimulateur de la guanylate cyclase soluble (sGC) ; analogue de la prostacycline PGI2 et agoniste des récepteurs de la prostacycline ; composés qui inhibent la dégradation de la guanosine monophosphate cyclique (GMPc) et/ou de l'adénosine monophosphate cyclique (AMPc) ; inhibiteurs de l'élastase des neutrophiles humains ; composés inhibant la cascade de transduction du signal ; substances actives permettant d'abaisser la pression artérielle ; inhibiteur de l'endopeptidase neutre ; agents osmotiques ; bloqueurs ENaC ; anti-inflammatoires, y compris corticostéroïdes et antagonistes des récepteurs de la chimiokine ; médicaments antihistaminiques ; médicaments antitussifs ; antibiotiques et substance médicamenteuse DNase et agents de clivage sélectif ; agents qui inhibent la phosphorylation ALK5 et/ou ALK4 de Smad2 et Smad3 ; inhibiteurs de la tryptophane hydroxylase 1 (TPH1) et inhibiteurs multikinases.
